# EUROPEAN PATENT APPLICATION

(11) **EP 3 348 576 A1**
(43) Date of publication of application: **18.07.2018**
(21) Application number: 17206437.0
(22) Date of filing: 30.10.2013
(51) Int. Cl.: C07K 16/40, A61K 39/00, G01N 33/573

(54) **THERAPEUTIC AND DIAGNOSTIC METHODS RELATED TO LYSYL OXIDASE-LIKE 2 (LOXL2)**

(30) Priority: 30.10.2012 US 201261720350 P; 09.11.2012 US 201261724858 P
(62) Divisional of application: 13792134.2
(71) Applicant: Gilead Sciences, Inc., Foster City, CA 94404 (US)
(72) Inventor: BORNSTEIN, Jeffrey D., Foster City, CA 94404 (US); ADAMKEWICZ, Joanne I., Foster City, CA 94404 (US); SMITH, Victoria, Foster City, CA 94404 (US); LYMAN, Susan K., Foster City, CA 94404 (US); CHIEN, Jason, Foster City, CA 94404 (US); LI, Xiaoming, Foster City, CA 94404 (US); SHAO, Lixin, Foster City, CA 94404 (US)
(74) Representative: Schnappauf, Georg

(57) **Abstract**

Provided are therapeutic, diagnostic, and prognostic methods for disease, including diseases associated with fibrosis and cancer using agents that bind to, inhibit, and/or detect lysyl oxidase-like 2 (LOXL2), and agents, compositions, kits, assay systems, and devices for use with such methods.

## Description

### Cross Reference to Related Applications

This application claims priority to U.S. Provisional Application No. 61/724,858 filed on November 9, 2012 and U.S. Provisional Application No. 61/720,350 filed on October 30, 2012, which applications are incorporated by reference herein in their entirety.

### Statement Regarding Sequence Listing

The Sequence Listing associated with this application is provided in text format in lieu of a paper copy, and is hereby incorporated by reference into the specification. The name of the text file containing the Sequence Listing is GILE-077 _03WO_ST25.txt. The text file is 66 KB, was created on October 30, 2013 and is being submitted electronically via EFS-Web.

### Technical Field

The present disclosure relates in some aspects to treatment and amelioration of symptoms of, and diagnostic and prognostic methods for, disease, including diseases associated with fibrosis, such as liver disease, using agents that are inhibitors of and/or bind to lysyl oxidase-like 2 (LOXL2).

### Background

Lysyl oxidase-like 2 (LOXL2) is a protein of the extracellular matrix induced in a variety of fibrotic diseases and conditions and tumors. It is secreted by activated fibroblasts, disease-associated smooth muscle cells, endothelial cells, and epithelia. There is a need for treatments and diagnostics for fibrotic diseases and conditions and other diseases and conditions associated with LOLX2, including liver diseases and lung diseases associated with fibrosis.

### Summary

The present disclosure relates in some aspects to methods of detection, diagnosis and treatment of diseases and conditions and compositions, agents, devices, kits, and assay systems for use in such methods.

Provided are methods for treating or ameliorating one or more symptoms of a disease or condition, such as a liver disease or condition, fibrotic disease or condition, e.g., fibrotic liver disease. Such methods generally are carried out by administering an agent that binds to and/or inhibits LOXL2 to a subject having a liver disease or condition, thereby treating or ameliorating the disease or condition.

In some embodiments, the disease or condition is a liver disease or condition, such as a liver disease or condition associated with fibrosis. In some aspects, the disease or condition is selected from the group consisting of: hepatitis C virus (HCV), NASH (nonalcoholic steatohepatitis), PSC (primary sclerosing cholangitis), cirrhosis, liver fibrosis, portal hypertension. In some aspects, the disease or condition is selected from the group consisting of PBC (primary biliary cirrhosis), autoimmune hepatitis, alcoholic cirrhosis, alpha 1 antitrypsin deficiency disease, hereditary hemochromatosis, Wilson's disease, hepatitis B virus (HBV), and HIV associated steatohepatitis. In some aspects, the liver disease or condition is a viral hepatitis, such as HCV or HBV. In some aspects, the liver disease is compenstated liver disease. On other aspects, it is decompenstated liver disease, such as liver disease associated with ascites, esophageal varices, encephalopathy, and/or jaundice.

In some embodiments, the agent is an antibody that specifically binds to LOXL2. In some aspects, the antibody is a monoclonal antibody. In some aspects, it is an antibody fragment, e.g., Fv, scFv, Fab, Fab' F(ab')₂ or Fab₂ fragment. In some aspects, it is a monoclonal antibody. In some aspects, the agent is an inhibitor of LOXL2, such as a non-competitive inhibitor. In some aspects, the agent binds to LOXL2 outside the catalytic domain, such as binds to an epitope within the SRCR3-4 domain of LOXL2.

In some embodiments, the antibody competes for binding to LOXL2 with an antibody having a heavy chain variable region sequence of SEQ ID NO: 8 and/or a light chain variable region sequence of SEQ ID NO: 9, and/or having one or more CDRs of such sequences. In some embodiments, the antibody comprises a heavy chain variable region having an amino acid sequence with at least at or about 75, 80, 85, 90, 95, 96, 97, 98, 99, or 100 % identity to a sequence set forth in SEQ ID NO: 6, 8, 10, 11, 12 and/or a light chain variable region having an amino acid sequence with at least 75, 80, 85, 90, 95, 96, 97, 98, 99, or 100 % identity to a sequence of SEQ ID NO: 7, 9, 13, or 14. In some embodiments, the antibody comprises a heavy chain variable region having an amino acid sequence with at least at or about 75, 80, 85, 90, 95, 96, 97, 98, 99, or 100 % identity to a sequence set forth in SEQ ID NO: 8 and a light chain variable region having an amino acid sequence with at least at or about 75, 80, 85, 90, 95, 96, 97, 98, 99, or 100 % identity to a sequence of SEQ ID NO: 9. In some embodiments, the antibody includes a heavy chain CDR of the heavy chain variable region sequence set forth in SEQ ID NO: 8 and/or a light chain CDR of the light chain variable region sequence set forth in SEQ ID NO: 9. In some aspects, such a CDR includes CDR3 or includes CDR1, CDR2, and/or CDR3, such as the CDR1, CDR2, and/or CDR3, of such heavy and light chain sequences.

In some embodiments, the agent is administered at a dose of at or about or at least at or about 10 mg/kg or 20 mg/kg or between about 10-20 mg/kg, is administered at a dose of at least at or about or at or about 200 mg or 700 mg or between about 200-700 mg, is administered at a dose of at least at or about or at or about 75 mg or 125 mg or between at or about 75-125 mg. In other embodiments, the agent is administered at a dose of at or about or at least at or about 10, 15, 20, 25, 30, 35, 40, 45, 50, 55, 60, 65, 70, 80, 90, 100, 125, 150, 175, 200, 225, 250, 275, 300, 325, 350, 375, 400, 425, 450, 475, or 500 mg/kg. In some aspects of such embodiments, the agent is administered intravenously, e.g., by infusion. In some aspects of such embodiments, the agent is aadministered subcutaneously. In some aspects of such embodiments, the agent is administered every week or every other week. In another embodiment, the agent is administered every one, two, three, four, five, or six weeks. In yet another embodiment, the agent is administrated multiple times over a period of one, two, three, four, five, six, seven, eight, nine, or ten months.
In some embodiments, the methods increase or prolong survival of the subject, reduce or prevent an increase in bridging fibrosis, reduces or prevents an increase in alpha smooth muscle actin (αSMA) levels, reduce or prevent an increase in stellate cell activation, and/or reduce or prevent increase in alanine aminotransferase (ALT) or aspartate aminotransferase (AST). In some embodiments, the methods further include a step of assessing bridging fibrosis, alpha smooth muscle actin (αSMA) levels, stellate cell activation, alanine aminotransferase (ALT), aspartate aminotransferase (AST), gamma-glutamyltransferase (GGT), and/or inflammation and/or necrosis, e.g., inflammation and/or necrosis of the diseased or fibrotic tissue, e.g., liver. In some aspects, the method includes determining that such a parameter is decreased or that an increase in the parameter is prevented compared to the absence of treatment or a pre-treatmcnt measure of the parameter. In some cases, the method reduces ALT, AST, GGT, or ALT/AST ratio to less than the upper limit or normal (ULN), or to less than 2x, 5x, or 10x the upper limit of normal (ULN). In some cases, the method reduces inflammation and/or necrosis, e.g., in the fibrotic or diseased tissue.

In some aspects, the methods further include detection or monitoring steps, such as a detecting a level of a LOXL2 gene product, e.g., protein or mRNA. In some aspects, such detection and monitoring steps indicate efficacy of the treatment.

Also provided are methods of detecting lysyl oxidase-like 2 (LOXL2), e.g., LOXL2 polypeptides, and use thereof in diagnostic, prognostic, predictive, and therapeutic methods. For example, provided are assays to detect and/or quantify LOXL2, such as assays to detect and/or quantify circulating lysyl oxidase-like 2 (LOXL2) polypeptides in an individual. Also provided are methods and uses of such assays in diagnostic, prognostic, and predictive applications and assay devices and kits for use in the same.

Provided are methods for detecting LOXL2, typically circulating LOXL2, in an individual. Among the provided methods are detection, diagnostic, prediction, monitoring, and prognostic methods. In some examples, the methods are carried out by contacting a sample, generally a liquid sample, obtained from the individual with an antibody specific for LOXL2 and detecting binding of the antibody to polypeptide, *e.g*., LOXL2 polypeptide, present in the sample. In some examples, the assay detects LOXL2 in the liquid sample to 300, 250, 200, 175 pg/mL or less or detects LOXL2 in the sample at a concentration of as low as 300, 250, 200, 175 pg/mL, for example, as low as from about 150 pg/mL to about 175 pg/mL, from about 125 pg/mL to about 150 pg/mL, from about 100 pg/mL to about 125 pg/mL, from about 75 pg/mL to about 100 pg/mL, from about 50 pg/mL to about 75 pg/mL, or from about 40 pg/mL to about 50 pg/mL.

In some examples, the detected LOXL2 level indicates the presence or absence of a disease or condition. In some examples, it indicates the likelihood that the individual will respond to a particular treatment for the disease, or indicates efficacy of a treatment. In some examples, such as where the methods are prognostic methods, the detected level of LOXL2 indicates the likelihood of an outcome, event, or endpoint of the disease or condition. In some aspects, the disease or condition is characterized by or associated with circulating LOXL2 or with elevated circulating LOXL2. In some aspects, the individual has the disease or condition; in some aspects, the individual is suspected of having the disease or condition. In some aspects, the methods further include determining that the individual has or does not have the disease or condition, is likely or not to respond to a particular treatment, or is likely or not to have a particular outcome or event, or that a treatment has or has not been effective.

In some examples, the individual is undergoing a treatment for the disease or condition and a detected level of LOXL2 that is lower than a level determined at an earlier time point, such as a pre-treatment level, indicates efficacy of the treatment.

The sample typically is a liquid sample, such as blood, a blood fraction, such as serum or plasma, urine, saliva, sputum, or bronchoalveolar lavage.

In some examples, the antibody includes a detectable label; exemplary labels include a chemiluminescent agent, a particulate label, a colorimetric agent, an energy transfer agent, an enzyme, a fluorescent agent, and a radioisotope. In some examples, the LOXL2 present in the sample is immobilized on an insoluble support by contacting the liquid sample with a second antibody specific for LOXL2 to form a second antibody-LOXL2 complex. In one example, the second antibody is immobilized on the insoluble support. In another example, the second antibody-LOXL2 complex is formed before contacting the sample with the antibody. The immobilized antibody may be polyclonal or monoclonal. In some examples, the antibody binds LOXL2 when the LOXL2 is bound to an agent that inhibits enzymatic activity of the LOXL2, such as an allosteric inhibitor of LOXL2 enzymatic activity, e.g., an anti-LOXL2 monoclonal antibody, such as one that binds an epitope within an SRCR3-4 domain.

Exemplary of the anti-LOXL2 antibodies for use in connection with the provided methods and embodiments include, for example, AB0023, AB0024, antibodies having a heavy chain variable region with an amino acid sequence as forth in SEQ ID NO: 6, 8, 10, 11, or 12, or with 75% or more, 80% or more, 90% or more, 95% or more, or 99% or more homology to SEQ ID NO:6, 8, 10, 11, or 12, or with a CDR1, CDR2, and/or CDR3 of the variable region sequence set forth in SEQ ID NO: 6, 8, 10, 11, or 12, and/or having a variable light chain region having the amino acid sequence set forth in SEQ ID NO: 7, 9, 13, or 14, or with 75% or more, 80% or more, 90% or more, 95% or more, or 99% or more homology to SEQ ID NO: 7 or with a CDR1, CDR2, and/or CDR3 of the variable region sequence set forth in SEQ ID NO: 7; 9, 13, or 14, such as an antibody with a heavy chain having the CDR1, CDR2, and/or CDR3 or the entire sequence of the variable region sequence set forth in SEQ ID NO: 8 and a light chain variable region with the CDR1, CDR2, and/or CDR3 or the entire sequence of the variable region scqucnce set forth in SEQ ID NO: 9.

In some examples, the methods further include comparing the detected level with a normal control value, where a detected level higher than a normal control value is indicative of the presence of the disease or condition, a likelihood that the individual will respond to a treatment for the disease or condition, or a likelihood of a pathological outcome. For instance, in some examples, the methods detect pathological levels of circulating LOXL2. Such methods can include comparing the detected level with a normal control or other reference value, where a detected level that is higher than a normal control or reference value is indicative of a pathology.

Also provided are methods for determining whether an individual has a disease or condition characterized by or associated with elevated circulating lysyl oxidase like-2 (LOXL2), diagnosing such a disease or condition, or making a predictive or prognostic determination regarding such a disease or condition. In examples, such methods are carried out by detecting a level of LOXL2 in a sample, e.g., liquid sample, from the individual, for example, according to the assays and methods provided herein, such as those described above. Typically, a level of LOXL2 that is greater than a normal control level, reference level, or in some cases greater than baseline indicates that the individual has a disease characterized by elevated circulating LOXL2, or indicates prognostic or predictive information about the disease or condition, such as predicting the likelihood of a particular outcome or the likelihood that the individual will respond to a particular disease treatment.

In some aspects of the provided methods, the disease or condition is fibrosis or cancer or a disease associated therewith. Examples include pulmonary fibrosis (such as idiopathic pulmonary fibrosis (IPF)), liver fibrosis, kidney fibrosis, cardiac fibrosis, myelofibrosis, cirrhosis, chronic viral hepatitis, hepatitis C virus (HCV) and hepatitis B virus (HBV). In some aspects, the disease or condition is idiopathic pulmonary fibrosis (IPF). In one aspect, the disease or condition is liver fibrosis. In certain aspects, the disease or condition is hepatitis virus C (HCV) or chronic hepatitis virus C infections. In other aspects, the disease or condition is hepatitis virus B virus (HBV) or chronic hepatitis virus B virus (CHB) infections. In yet other aspects, the disease or condition is non-alcoholic steatohepatitis (NASH). In some other aspects, the disease or condition is primary biliary cirrhosis (PBC).

The methods can further include subjecting the individual to one or more further diagnostic tests, which can include pulmonary function tests, cardiac function tests, and liver function tests.

Also provided are methods for determining the likelihood that an individual having a fibrotic disease will exhibit a beneficial clinical response to a treatment for the fibrotic disease and/or that the individual will exhibit progression or regression with respect to a particular disease outcome, such as cirrhosis. Such methods can include determining a circulating level of lysyl oxidase like-2 (LOXL2), for example, in a liquid sample obtained from the individual, such as by the methods described above. In one aspect, a circulating level of LOXL2 that is greater than a normal control level indicates that the individual has an increased likelihood of exhibiting a beneficial clinical response to a treatment for the fibrotic disease. In some examples, reports are generated based on the determined likelihood. In some examples, the methods further include treating the individual for the fibrotic disease. In some examples, the individual has an active fibrotic disease, such as METAVIR F1 or F2 liver fibrosis, and/or an advanced stage fibrotic disease, such as METAVIR F4 liver fibrosis.

Also provided are methods for determining the efficacy of a treatment for a disease characterized by elevated lysyl oxidase like-2 (LOXL2) in an individual. In some examples, such methods are carried out by determining a circulating LOXL2 level at a time point in an individual undergoing treatment for the disease, according to the detection methods described above and herein. Typically, a level of circulating LOXL2 in the sample that is lower than a level obtained at an earlier time point, such as a pre-treatment level, from the individual indicates efficacy of the treatment. Alternatively, the level of circulating LOXL2 in the sample may increase initially followed by the clearance by the body.

Also among the provided methods are predictive and prognostic methods for idiopathic pulmonary fibrosis (IPF). In some examples, such methods are carried out by obtaining a sample from an individual; and detecting a level of LOXL2 in the sample, such as using the methods described herein. Generally, the level of LOXL2 indicates the likelihood of an IPF disease outcome or event in the individual. Also provided are predictive and prognostic methods for liver fibrosis. In some examples, such methods are carried out by obtaining a sample from an individual; and detecting a level of LOXL2 in the sample, such as using the methods described herein. Generally, the level of LOXL2 indicates the likelihood of liver fibrosis outcome or event (e.g. progression, regression, advance stage of fibrosis) in the individual.

These and others of the provided methods can also include a step of comparing the detected level to a normal control level of LOXL2, where an elevated LOXL2 level compared to the normal control level indicates an increased likelihood of the occurrence of an IPF disease outcome or event in the individual. In some embodiments of the provided methods, a level of LOXL2 that is higher than a threshold baseline level correlates with the negative outcome or mortality in a subject. Thus, in some embodiments, the methods include a step of determining whether the LOXL2 level in the sample is above or below the threshold level and/or whether the sample contains low or high levels of LOXL2. In one example, the threshold LOXL2 level in the sample is at least 800 picograms (pg) per milliliter (mL), at least 700 pg/mL, at least 750 pg/mL, at least between 700 and 800 pg/mL at least 600 pg/mL, at least 400 pg/mL, or at least 200 pg/mL. In another example, the threshold LOXL2 level in the sample is at least 440 pg/mL. In one example, the method indicates at least a 2-fold, 3-fold, 4-fold, 5-fold, 6-fold, or 7-fold increase in the likelihood of the IPF disease outcome in the individual compared with a subject having a LOXL2 level that is equal to the normal control LOXL2 level or baseline. In one example, a LOXL2 level that is at or above the threshold level, e.g., at or above or at or above about 700, 710, 720, 730, 740, 750, 760, 770, 780, 790, 800, or between 700-800, indicates at least a 2-fold, 3-fold, 4-fold, 5-fold, 6-fold, or 7-fold increase in the likelihood of the IPF disease outcome in the individual compared with a subject having a LOXL2 level that is equal to the normal control LOXL2 level or baseline.

In some embodiments, the detected level of LOXL2 is greater than about 700 pg/mL, is greater than between about 700 and between about 800 pg/mL, is between about 700 and about 800 pg/mL, or is greater than about 800 pg/mL.

Outcomes and events that may be predicted by the provided methods include, but are not limited to survival, cirrhosis, progression or regression thereof, progression to a more advanced stage of fibrosis, progression to decompensated liver disease, AST, ALT, inflammation, necrosis, efficacy of treatment, suppression of infection, progression to chronic or advanced disease, e.g., fibrosis, fibrotic stage, mortality from any cause, respiratory hospitalization, or a categorical decrease in lung function), lung function decline, respiratory hospitalization, transplant-free survival, death, and responsiveness to treatment, and/or amelioration of one or more symptom.

Symptoms that may be detected or the likelihood determined by the methods include fibrotic stage or score, e.g., as determined by any of the methods herein, cirrhosis, decompensated liver disease, inflammation, level of an enzyme, such as AST, ALT, necrosis, degree of lung or liver function, survival, hospitalization, and transplant-free survival.

In some embodiments, the level, e.g., threshold level, of detected LOXL2 determines or predicts the likelihood or the presence or absence of such a symptom or outcome of the disease or condition.

Among the IPF disease outcomes and events are IPF disease progression (such as that defined as mortality from any cause, respiratory hospitalization, or a categorical decrease in lung function), lung function decline, respiratory hospitalization, transplant-free survival, death, and responsiveness to treatment. In some cases, the methods predict an outcome, event, or endpoint, or the likelihood thereof, associated with IPF, in an individual. In some cases, the methods predict the outcome, endpoint, or likelihood thereof in an individual who has been deemed "negative" for such an output, endpoint, or likelihood by another method or assay, such as based on the Personal Clinical and Molecular Mortality index (PCMI) or level of one or more other biomarker, such as MMP7, ICAM1, IL8, VCAM1, and S100A12 (or for which such other method or assay does not detect or is incapable of detecting the outcome, event, endpoint, or likelihood thereof).

The predictive or prognostic IPF method can further include detecting a measure of IPF disease severity or functional status in the individual, selected from the group consisting of percent of predicted forced vital capacity (FVC), percent of predicted carbon monoxide diffusion capacity (DL_{CO}), 6-minute walk distance (6MWD), mean pulmonary artery pressure (mPAP), the lowest resting oxygen saturation (SpO2), the composite physiologic index (CPI), the St. George's Respiratory Questionnaire score (SGRQ), and the Transition Dyspnea Index (TDI) score, responsiveness to treatment, and biomarkers of IPF disease. In some examples, the methods further include analyzing the LOXL2 level and/or measure of disease severity or functional status using a predictive model.

Also provided are methods for monitoring response of an individual to IPF treatment or determining the likelihood that the individual will respond to treatment. In one example, such methods are carried out by obtaining a sample from an individual undergoing treatment for IPF; and detecting a level of LOXL2 in the sample. Typically, the level of LOXL2 indicates the responsiveness of the individual to the treatment or the likelihood that the individual will respond to the treatment.

In some cases, the methods further include initiating, altering, or discontinuing an IPF treatment in the individual. In some examples, treatment is initiated, altered, or discontinued based on the information determined by the methods, such as the level or relative level of LOXL2 or the prognostic or predictive information. In some examples, the treatment is initiated prior to determination of the LOXL2 levels.

Also provided are assay devices and kits for use in the provided methods, such as for use for use in determining the level of a lysyl oxidase-like 2 (LOXL2) polypeptide in a liquid biological sample obtained from an individual. In one embodiment, such a device includes a matrix defining an axial flow path, the matrix including i) a sample receiving zone at an upstream end of the flow path that receives the fluid sample; ii) one or more test zones positioned within the flow path and downstream from the sample receiving zone, each of the one or more test zones comprising a LOXL2-specific antibody, wherein each of the LOXL2-specific antibodies is capable of binding a LOXL2 polypeptide present in the liquid sample to form an anti-LOXL2 antibody/LOXL2 complex; and iii) one or more control zones positioned within the flow path and downstream from the sample receiving zone.

The one or more control zones can be positioned between the test zones when two test zones are present. The test zones and control zones can be positioned in an alternating format within the flow path beginning with a test zone positioned upstream of any control zone. In one example, one or more of the anti-LOXL2 antibodies is immobilized on the matrix in the test zone.

In some examples, the device further includes a label zone including a labeled antibody specific for a LOXL2-specific antibody. Generally, the labeled antibody is capable of binding an anti-LOXL2 antibody present in an anti-LOXL2 antibody/LOXL2 complex to form a labeled anti-LOXL2 antibody/LOXL2, and the labeled antibody is mobilizable in the presence of liquid sample. The labeled antibody can include a label component selected from among a chemiluminescent agent, a particulate label, a colorimetric agent, an energy transfer agent, an enzyme, a fluorescent agent, and a radioisotope.

In some examples of the devices, the matrix is positioned within a housing comprising a support and optionally a cover, wherein the housing contains an application aperture and one or more observation ports. Among the provided devices are test strips and dipstick assay devices.

Among the provided kits for determining the level of a lysyl oxidase-like 2 (LOXL2) polypeptide in a biological sample obtained from an individual are those including a first antibody specific for LOXL2 and a second antibody specific for LOXL2. The kit also can include purified LOXL2 for use in generating a standard curve. In one example, at least one of the antibodies in the kit includes a detectable label, such as a chemiluminescent agent, a particulate label, a colorimetric agent, an energy transfer agent, an enzyme, a fluorescent agent, and a radioisotope.

### Brief description of the drawings

**Figure 1** depicts effects administering an inhibitory anti-LOXL2 antibody (AB0023) in a CC14 mouse model of liver fibrosis.
**Figure 2** depicts alanine aminotransferase (ALT) levels in subjects with liver disease having elevated pretreatment ALT at indicated time-points following administration of an anti-LOXL2 antibody (AB0024) ("baseline" = pre-treatment).
**Figure 3** depicts aspartate aminotransferase (AST) levels in subjects with liver disease having elevated pretreatment ALT at indicated time-points following administration of an anti-LOXL2 antibody (AB0024) ("baseline" = pre-treatment).
**Figure 4** depicts mean ALT (left bars) and AST (right bars) levels in subjects with liver disease having elevated pretreatment ALT before ("baseline"), after a four-week treatment with the anti-LOXL2 antibody AB0024 ("end of treatment"), and at the end of a six-week follow-up period beginning at the end of the four-week treatment period.
**Figure 5** depicts the mean ALT, AST, and gamma-glutamyltransferase (GGT) levels by visit in ten subjects with liver disease at baseline and various indicated time-points following a four-week administration of the anti-LOXL2 antibody AB0024.
**Figure 6** depicts mean ALT (left bars) and AST (right bars) levels in subjects with elevated pretreatment AST/ALT levels at baseline, end of treatment, and end of follow-up period, p = 0.02 for AST at end of treatment versus baseline.
**Figure 7** depicts standard calibrator curves for a LOXL2 immunoassay, with raw ECL (electrochemiluminescence) counts plotted on the y-axis and LOXL2 concentration (nM/L) plotted on the x-axis. Purified recombinant full-length LOXL2 protein was added into pooled normal human serum, followed by serial dilution in serum to create a calibrator curve. Each data point represents the mean of three replicate wells; curves for four independent plates are shown.
**Figure 8** depicts LOXL2 levels in serum samples from patients with idiopathic pulmonary fibrosis.

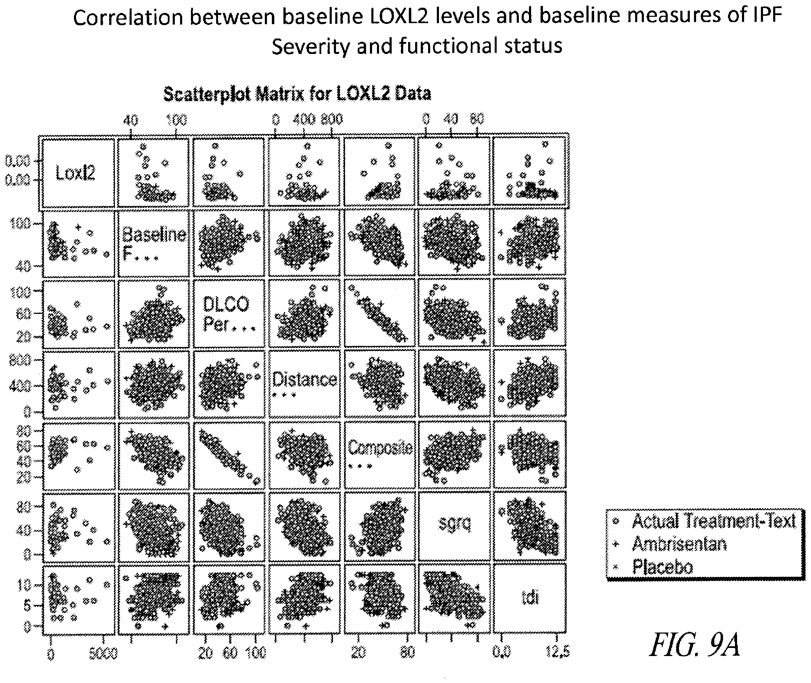

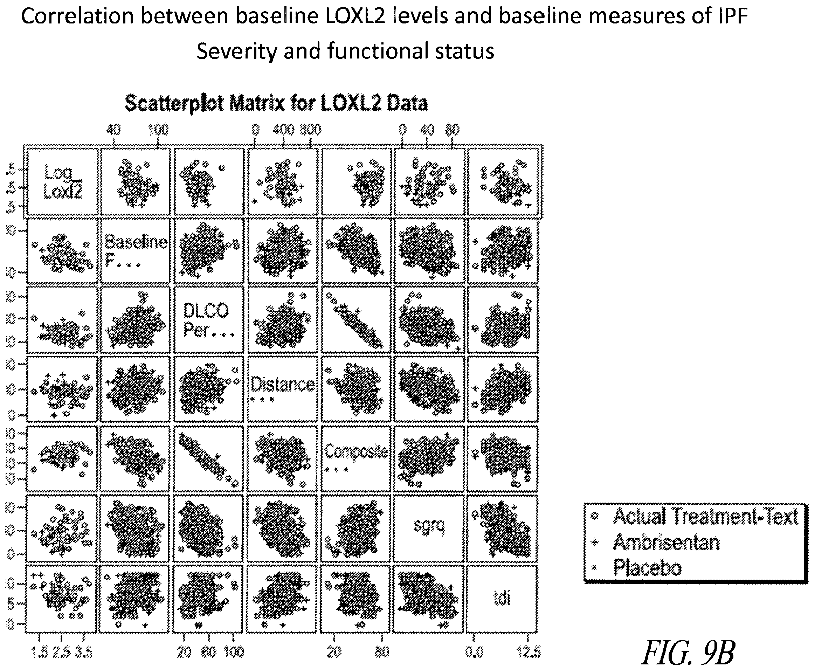

**Figure 9** depicts scatter plot matrices demonstrating correlation between baseline LOXL2 levels (with untransformed LOXL2 levels **in** **Figure 9A** and Log₁₀X-transformed LOXL2 levels in **Figure 9B**) and baseline measures of idiopathic pulmonary fibrosis (IPF) severity and functional status, as described in Example 5B. In each panel, the x- and y-axis of the first row and column, respectively, represent baseline LOXL2 levels; the x- and y-axis of the second row and column, respectively, represent baseline predicted forced vital capacity (FVC); the x-and y-axis of the third row and column, respectively, represent baseline percent of predicted carbon monoxide diffusion capacity (DL_{CO}); the x- and y-axis of the fourth row and column, respectively, represent the baseline 6-minute walk distance (6MWD); the x- and y-axis of the fifth row and column, respectively, represent the baseline composite physiologic index (CPI); the x- and y-axis of the sixth row and column, respectively, represent the baseline St. George's Respiratory Questionnaire score; and the x- and y-axis of the seventh row and column, respectively, represent the baseline Transition Dyspnea Index score. Correlation between LOXL2 and baseline measures of IPF severity and performance status are highlighted within the dark boxes at the top row of panels (a) and (b).

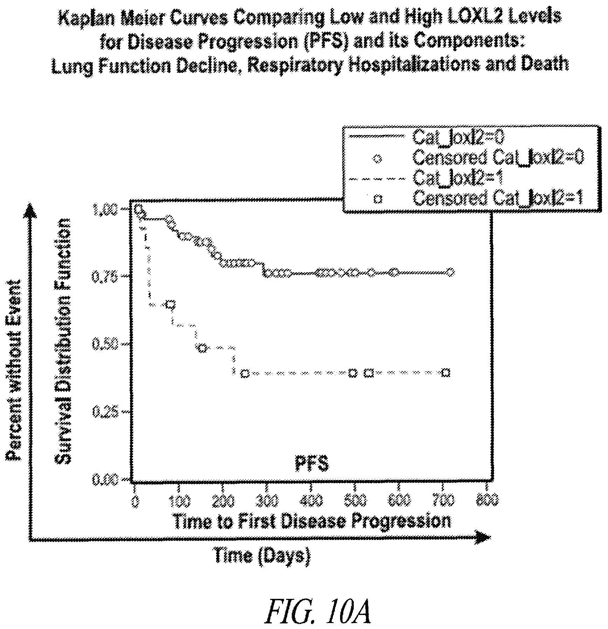

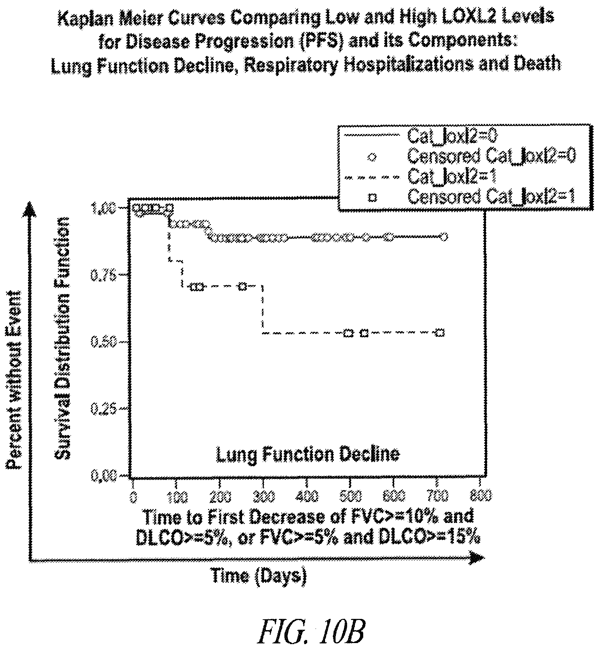

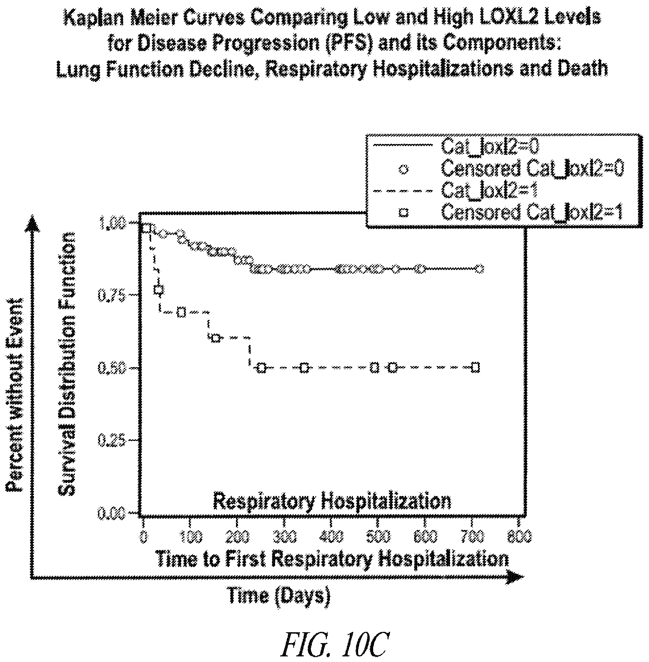

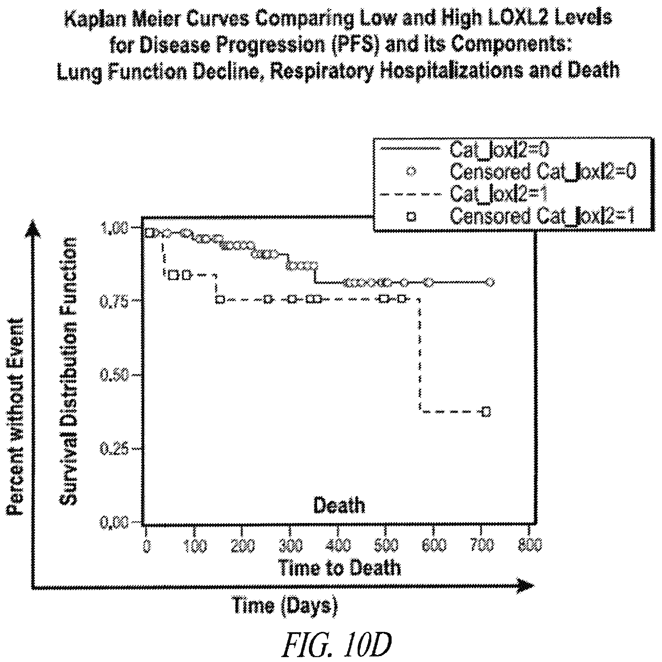

**Figure 10** depicts Kaplan Meier curves, comparing low (≤ 800 pg/mL) and high (> 800 pg/mL) LOXL2 levels for disease progression (PFS) (**Figure 10A**) and its components: lung function decline (Figure **10B**), respiratory hospitalizations (**Figure 10C**) and death (**Figure 10D**). In each panel, the top, darker line represents patients with low (≤ 800 pg/mL) baseline serum LOXL2 levels and the lower, lighter line represents patients with high (> 800 pg/mL) baseline LOXL2 levels. All patients were treated with ambrisentan. Each y-axis shows percent of patients without the given event (with 0, 25, 50, 75, and 100 marked along the axis) and each x-axis shows time in days (with 0, 100, 200, 300, 400, 500, 600, 700, and 800 days marked along the axis).
**Figure 11** depicts a comparison of baseline LOXL2 distribution in the ARTEMIS-IPF subjects (**Figure 11A**: placebo and Ambrisentan-treated subjects combined; **Figure 11B**: Ambrisentan only) and the GAP cohort subjects.

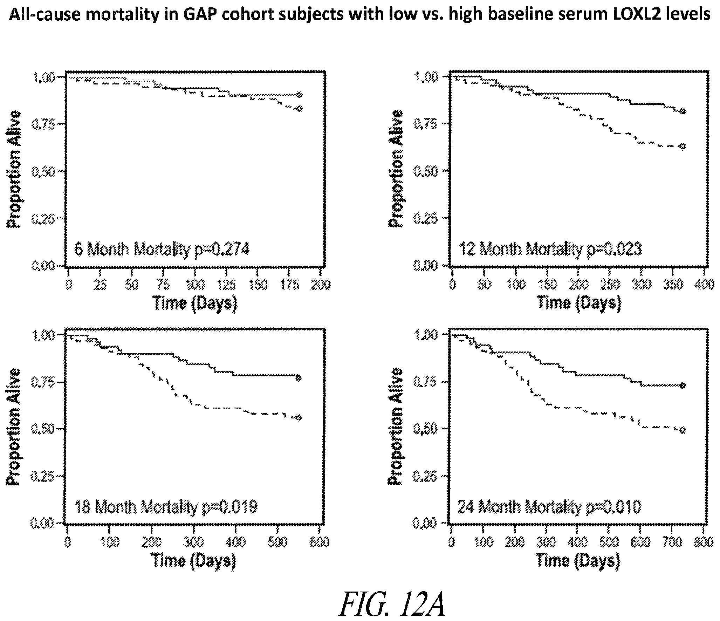

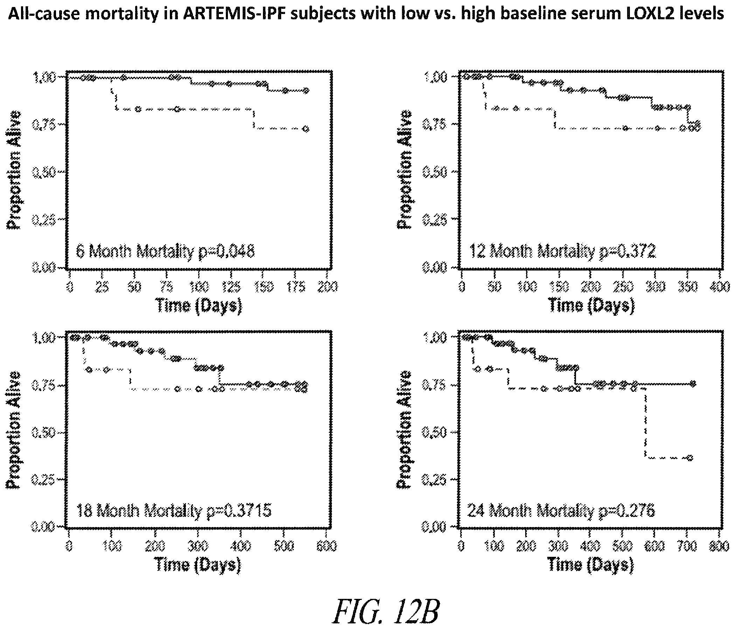

**Figure 12A** depicts Kaplan Meier curves for all-cause mortality according to low (upper line, ≤ 440 pg/mL) versus high (lower line, > 440 pg/mL) serum LOXL2 levels at 6-months (upper left panel), 12-months (upper right panel), 18-months (lower left panel) and 24-months (lower right panel) after baseline in the GAP cohort study. **Figure 12B** depicts Kaplan Meier curves for all-cause mortality according to low (upper line, ≤ 800 pg/mL) versus high (lower line, > 800 pg/mL) serum LOXL2 levels at 6-months (upper left panel), 12-months (upper right panel), 18-months (lower left panel) and 24-months (lower right panel) after baseline in the ARTEMIS-IPF study.
**Figure 13A** shows curves for disease progression (DP (including lung function (LF) decline (10% decrease in FVC and 5% decrease in DL_{CO} or 15 % decrease in DL_{CO} and 5% decrease in FVC), respiratory hospitalizations (RH) and mortality))) in subjects of the ARTEMIS-IPF study with low (lower line, ≤800 pg/mL) versus high (upper line, > 800 pg/mL) serum LOXL2 levels. **Figure 13B** shows curves for mortality in subjects of the GAP cohort with low (lower line, ≤ 700 pg/mL) versus high (upper line, > 700 pg/mL) serum LOXL2 levels.

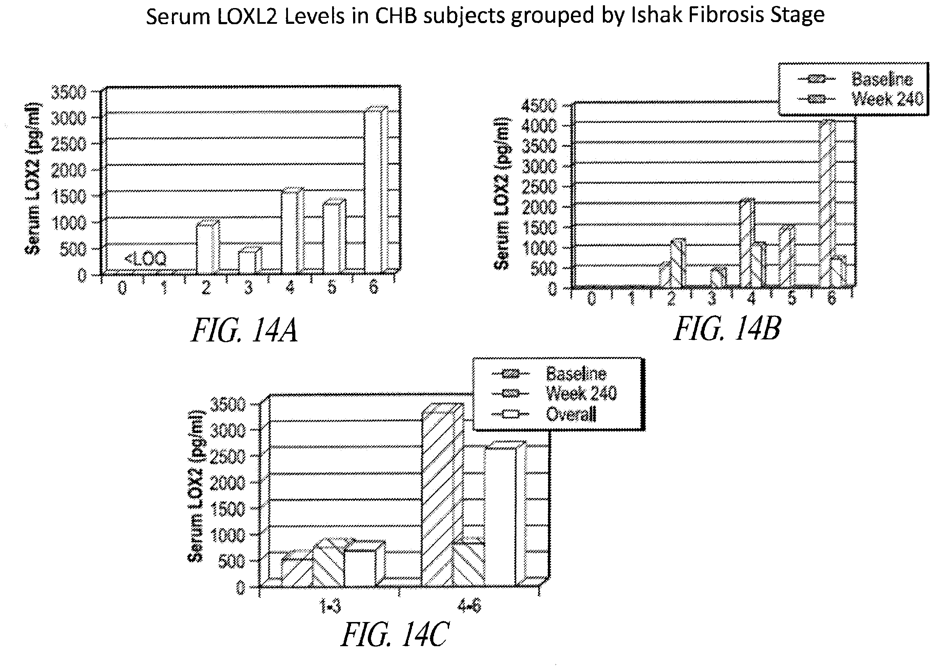

**Figure 14** shows mean serum LOXL2 levels (pg/mL) for various groups of subjects. **Figure 14A** shows mean serum LOXL2 levels for baseline and week 240 samples (total of 162 samples (one baseline and one week-240 for each of 81 subjects), grouped according to Ishak fibrosis score of the corresponding subject (0, 1, 2, 3, 4, 5, 6, left-right). LOQ = level of quantification. **Figure 14B** shows baseline and week-240 mean serum LOXL2 levels for subjects with given Ishak stages (0, 1, 2, 3, 4, 5, 6, left-right) at baseline and week 240. **Figure 14C** shows baseline, week-240, and overall serum levels of LOXL2 for patients with corresponding Ishak stages of between 1 and 3 and between 4 and 6.
**Figure 15** shows the percentage of subjects in the study with each given Ishak Stage (1, 2, 3, 4, 5, 6 (individual bars left to right)) that were determined to have a given level of serum LOXL2 (pg/mL). LOD=limit of detection; LOQ=limit of quantification. Each category shown extended from the upper limit of the previous category, for example, 1500=1001-1500 pg/mL.

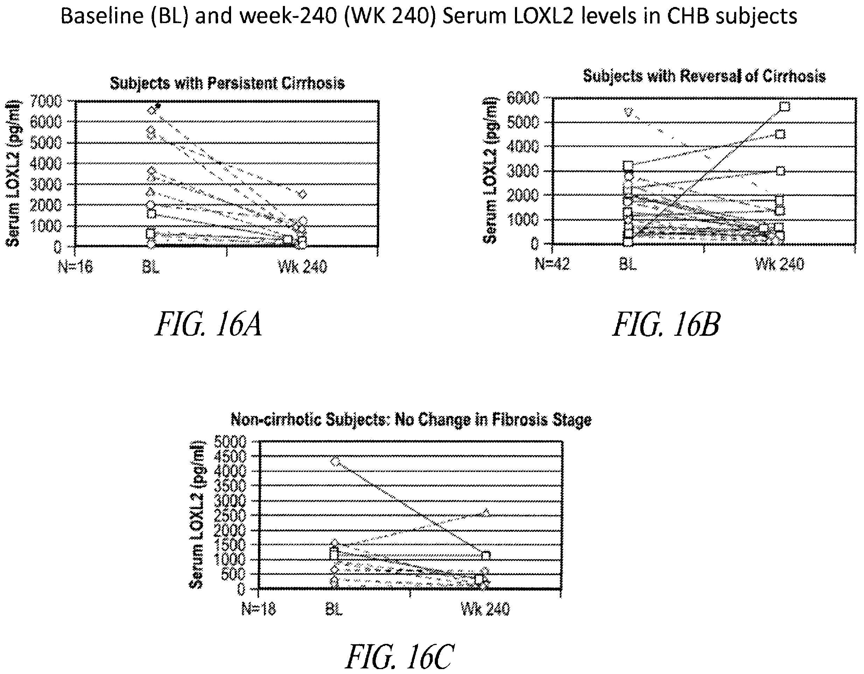

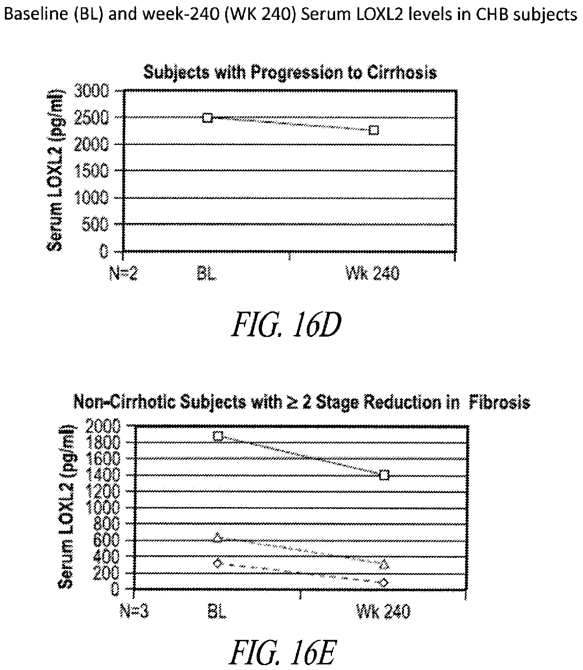

**Figure 16** shows serum LOXL2 levels (pg/mL) at baseline and week 240 following treatment for individual CHB subjects. **Figure 16A****:** subjects with persistent cirrhosis (n=16); **Figure 16B****:** subjects with reversal of cirrhosis by week 240 (n=42); **Figure 16C****:** non-cirrhotic subjects that did not experience a change in fibrotic stage (Ishak) by week 240); **Figure 16D****:** subjects that experienced a progression to cirrhosis over the course of the study; and **Figure** 16E: non-cirrhotic subjects with greater than or equal to 2-stage reduction in fibrosis (Ishak score). LOQ (limit of quantification)=440 pg/mL, LOD (limit of detection)=180 pg/mL.
**Figure 17** shows the percentage of cirrhotic CHB subjects that exhibited a histological improvement at week 240 ("Y") having given baseline serum LOXL2 levels (<1500, >1500, 1500-3000, <3000, and >3000 pg/mL) and the percentage of cirrhotic subjects determined not to have histological improvement at week 240 ("N") having the same given baseline serum LOXL2 levels.
**Figure 18** shows percentage of CHB subjects having varying Ishak fibrosis scores (1-6, bottom to top) at baseline, year 1, and year 5 following treatment.
**Figure 19** shows mean (squares) and median (triangles) sLOXL2 levels in CHB subjects over a 240-week treatment period. The dotted line shows limit of quantification in this study.
**Figure 20** shows baseline sLOXL2 levels according to baseline Ishak fibrosis score for CHB subjects. **Figure 20A** shows individual levels for subjects at the indicated fibrosis score. **Figure 20B** shows data for binned Ishak fibrosis scores.
**Figure 21** shows sLOXL2 levels in CHB subjects with compensated versus decompensated liver disease.
**Figure 22** shows sLOXL2 levels in decompensated CHB subjects with various MELD scores.
**Figure 23** shows results from immunohistochemistry (IHC) staining of liver explants from patients with PSC (**Figure 23A**) and patients with PBC (**Figure 23B**), and of liver tissue from *Mdr2*^{*-*/*-*} (**Figure 23C**) mice and mice subject to bile duct-ligation (BDL) (**Figure 23D**) mice (day 7 post-surgery).
**Figure 24** shows relative LOXL2 mRNA expression in liver tissues of animal models of cholestatic liver disease. **Figure 24A** shows relative LOXL2 mRNA levels measured in liver tissue from wild-type versus *Mdr2*^{*-*/*-*} mice. **Figure 24B** shows relative LOXL2 mRNA levels measured in liver tissue from mice subjected to sham surgery or bile duct ligation (BDL (day 3 and day 7 post-surgery)).
**Figure 25** depicts an amino acid sequence of human LOXL2 (SEQ ID NO:1).
**Figure 26** depicts LOXL2 serum concentration versus Ishak fibrosis score for 87 patients with chronic hepatitis C virus (HCV) infection.
**Figure 27** depicts LOXL2 levels (pg/ml) in serum samples from patients diagnosed with liver fibrosis.

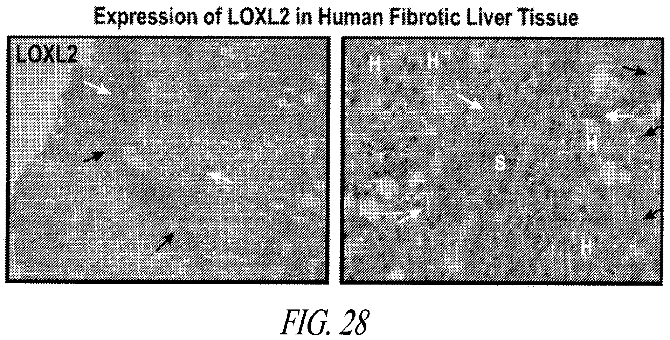

**Figure 28** shows expression of LOXL2 in human fibrotic liver tissue, as determined by Immunohistochemical (IHC) staining of liver tissues from a patient with chronic HCV infection. In the left panel (5x objective magnification), black arrows indicate areas of fibrous expansion into portal regions and tracts. White arrows indicate areas of short fibrous septa surrounding hepatic lobules. The right panel (40x objective magnification) shows LOXL2 immunoreactivity, observed in the fibrous septa (S) at the interface with hepatocytes (H), within the perisinusoidal space (arrows), and in the myofibroblasts within the liver parenchyma (arrows).
**Figure 29** shows LOXL2 serum levels by binned baseline Ishak fibrosis score and time. Each panel shows, for the indicated time point, LOXL2 concentration (pg/mL) for two groups of patients, grouped according to Ishak Fibrosis Score (1-3 and 5-6, respectively). Three outliers (LOXL2 concentration = 5529, 6621, 8845 pg/mL), with LOXL2 concentration out of plot ranges all were from the same subject, having an Ishak fibrosis score of 5.
**Figure 30** shows median within-subject LOXL2 serum levels, calculated as median LOXL2 serum concentration over weeks 4-30, for two groups of patients, grouped according to Ishak Fibrosis Score (1-3 and 5-6, respectively). The average within-subject coefficient of variation was 22 %.
**Figure 31** shows median LOXL2 serum concentration (pg/mL) over time (weeks), by binned baseline ishak fibrosis score, with 95% confidence intervals. Only one subject had a change greater than or equal to 2 in Ishak fibrosis score over the 25-28 weeks between study biopsies.
**Figure 32** shows median within-subject levels of LOXL2 vs. levels of Hyaluronic acid (HA) (top panel) and tissue inhibitor of metalloproteinases-1 (TIMP1) (bottom panel), for subjects having the indicated Ishak scores (1-6). Median within-subject expression was calculated as median expression over weeks 4 through 30. The curve was constructed using locally weighted scatter plot smoothing.

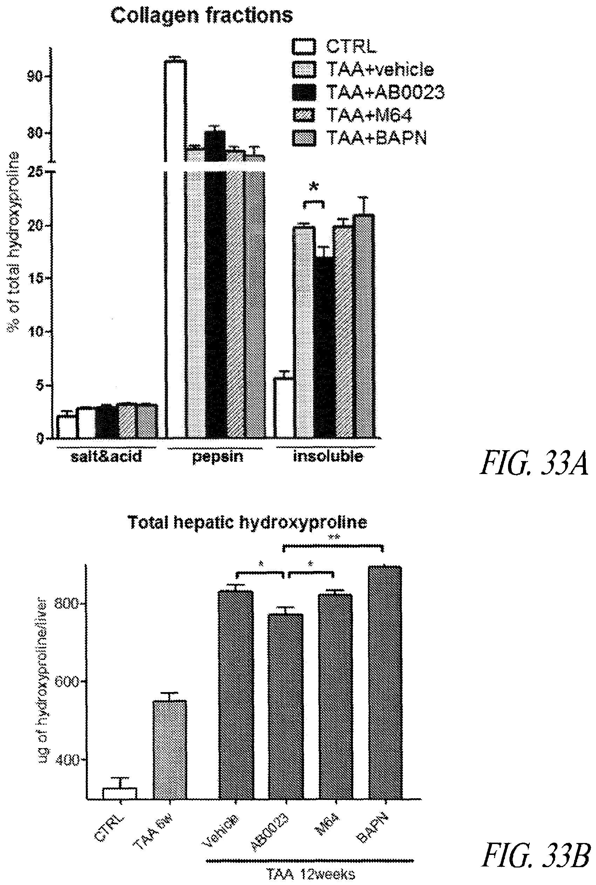

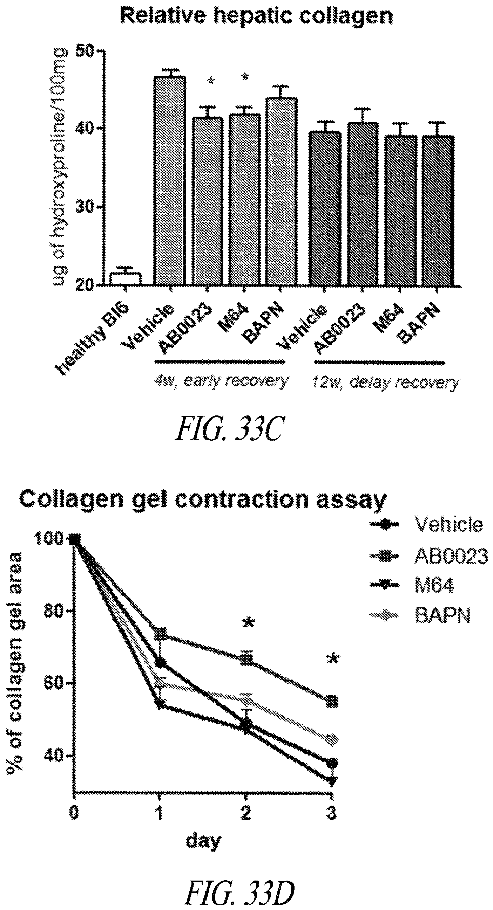

**Figure 33A** shows levels of total hydroxyproline of different collagen fractions obtained from the TAA mice administered with AB0023, M64, BAPN, or vehicle. **Figure 33B** shows levels of total hydrooxyproline showing hepatic collagen that was obtained from the TAA mice administered with AB0023, M64, BAPN, or vehicle at week 12. **Figure 33C** shows levels of total hydrooxyproline showing relative hepatic collagen that was obtained from the TAA mice administered with AB0023, M64, BAPN, or vehicle at weeks 6 and 12 during recovery. **Figure 33D** shows a collagen gel contraction assay in the TAA mice administered with AB0023, M64, BAPN, or vehicle during recovery.
**Figure 33A** shows levels of total hydroxyproline of different collagen fractions obtained from the TAA mice administered with AB0023, M64, BAPN, or vehicle. **Figure 33B** shows levels of total hydrooxyproline showing hepatic collagen that was obtained from the TAA mice administered with AB0023, M64, BAPN, or vehicle at week 12. **Figure 33C** shows levels of total hydrooxyproline showing relative hepatic collagen that was obtained from the TAA mice administered with AB0023, M64, BAPN, or vehicle at weeks 6 and 12 during recovery. **Figure 33D** shows a collagen gel contraction assay in the TAA mice administered with AB0023, M64, BAPN, or vehicle during recovery.

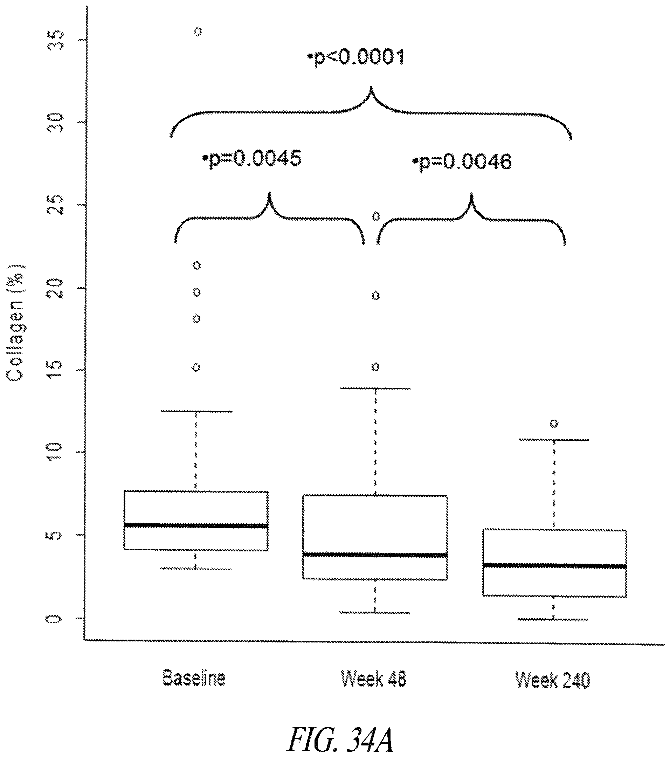

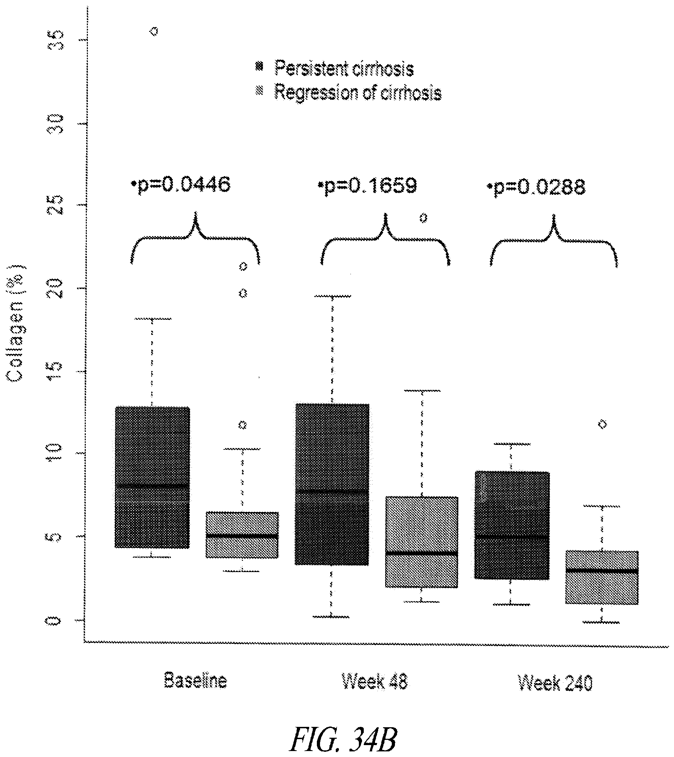

**Figure 34A** shows change in Percent Collagen Area (PCA). Mean PCA was 7.1% at Baseline, 5.3% at Week 48 and 3.9% at Week 240 for the entire population. **Figure 34B** shows change in PCA in Cirrhotic Subjects. Mean PCA was significantly higher in those with persistent cirrhosis than in those with histologic regression. The regressor groups also had a proportionally greater reduction in PCA over time.

### Detailed Description

### I. DEFINITIONS

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

When a trade name is used herein, reference to the trade name also refers to the product formulation, the generic drug, and the active pharmaceutical ingredient(s) of the trade name product, unless otherwise indicated by context.

The term "antibody" is used in the broadest sense unless clearly indicated otherwise, and specifically covers monoclonal antibodies (including full-length monoclonal antibodies), polyclonal antibodies, human antibodies, humanized antibodies, chimeric antibodies, nanobodies, diabodies, multispecific antibodies (e.g., bispecific antibodies), and antibody fragments including but not limited to Fv, scFv, Fab, Fab' F(ab')₂ and Fab₂, so long as they exhibit the desired biological activity. The term "human antibody" refers to antibodies containing sequences of human origin, except for possible non-human CDR regions, and does not imply that the full structure of an immunoglobulin molecule be present, only that the antibody has minimal immunogenic effect in a human (*i.e*., does not induce the production of antibodies to itself).

An "antibody fragment" comprises a portion of a full-length antibody, for example, the antigen binding or variable region of a full-length antibody. Such antibody fragments may also be referred to herein as "functional fragments: or "antigen-binding fragments". Examples of antibody fragments include Fab, Fab', F(ab')₂, and Fv fragments; diabodies; linear antibodies (Zapata et al. (1995) Protein Eng. 8(10): 1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen combining sites and is still capable of cross-linking antigen.

"Fv" is a minimum antibody fragment containing a complete antigen-recognition and -binding site. This region consists of a dimer of one heavy- and one light-chain variable domain in tight, non-covalent association. It is in this configuration that the three complementarity-determining regions (CDRs) of each variable domain interact to define an antigen-binding site on the surface of the V_{H}-V_{L} dimer. Collectively, the six CDRs confer antigen-binding specificity to the antibody. However, even a single variable domain (or an isolated V_{H} or V_{L} region comprising only three of the six CDRs specific for an antigen) has the ability to recognize and bind antigen, although generally at a lower affinity than does the entire Fᵥ fragment.

The "F_{ab}" fragment also contains, in addition to heavy and light chain variable regions, the constant domain of the light chain and the first constant domain (CH₁) of the heavy chain. Fab fragments were originally observed following papain digestion of an antibody. Fab' fragments differ from Fab fragments in that F(ab') fragments contain several additional residues at the carboxy terminus of the heavy chain CH₁ domain, including one or more cysteines from the antibody hinge region. F(ab')₂ fragments contain two Fab fragments joined, near the hinge region, by disulfide bonds, and were originally observed following pepsin digestion of an antibody. Fab'-SH is the designation herein for Fab' fragments in which the cysteine residue(s) of the constant domains bear a free thiol group. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains. Depending on the amino acid sequence of the constant domain of their heavy chains, immunoglobulins can be assigned to five major classes: IgA, IgD, IgE, IgG, and IgM, and several of these may be further divided into subclasses (isotypes), e.g., IgG1, IgG2, IgG3, IgG4, IgA1, and IgA2.

"Single-chain Fv" or "sFv" or "scFv" antibody fragments comprise the V_{H} and V_{L} domains of antibody, wherein these domains are present in a single polypeptide chain. In some embodiments, the Fv polypeptide further comprises a polypeptide linker between the V_{H} and V_{L} domains, which enables the sFv to form the desired structure for antigen binding. For a review of sFv, see Pluckthun, in The Pharmacology of Monoclonal Antibodies, vol. 113 (Rosenburg and Moore eds.) Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain, thereby creating two antigen-binding sites. Diabodies are additionally described, for example, in EP 404,097; WO 93/11161 and Hollinger et al. (1993) Proc. Natl. Acad. Sci. USA 90:6444-6448.

An "isolated" antibody is one that has been identified and separated and/or recovered from a component of its natural environment. Components of its natural environment may include enzymes, hormones, and other proteinaceous or nonproteinaceous solutes. In some embodiments, an isolated antibody is purified (1) to greater than 95% by weight of antibody as determined by the Lowry method, for example, more than 99% by weight, (2) to a degree sufficient to obtain at least 15 residues of N-terminal or internal amino acid sequence, e.g., by use of a spinning cup sequenator, or to homogeneity by gel electrophoresis (*e.g*., SDS-PAGE) under reducing or nonreducing conditions, with detection by Coomassie blue or silver stain. The term "isolated antibody" includes an antibody *in situ* within recombinant cells, since at least one component of the antibody's natural environment will not be present. In certain embodiments, isolated antibody is prepared by at least one purification step.

As used herein, "immunoreactive" refers to antibodies or fragments thereof that are specific to a sequence of amino acid residues ("binding site" or "epitope"), yet if are cross-reactive to other peptides/proteins, are not toxic at the levels at which they are formulated for administration to human use. "Epitope" refers to that portion of an antigen capable of forming a binding interaction with an antibody or antigen binding fragment thereof. An epitope can be a linear peptide sequence (i.e., "continuous") or can be composed of noncontiguous amino acid sequences (i.e., "conformational" or "discontinuous"). The term "preferentially binds" means that the binding agent binds to the binding site with greater affinity than it binds unrelated amino acid sequences.

The terms "complementarity determining region," and "CDR," are known in the art to refer to non-contiguous sequences of amino acids within antibody variable regions, which confer antigen specificity and binding affinity. In general, there are three (3) CDRs in a heavy chain variable region (CDRH1, CDRH2, CDRH3) and three (3) CDRs in a light chain variable region (CDRL1, CDRL2, CDRL3).

The precise amino acid sequence boundaries of a given CDR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), MacCallum et al., J. Mol. Biol. 262:732-745 (1996), "Antibody-antigen interactions: Contact analysis and binding site topography,"J. Mol. Biol. 262, 732-745." (Contact" numbering scheme), Lefrane MP et al., "IMGT unique numbering for immunoglobulin and T cell receptor variable domains and Ig superfamily V-like domains," Dev Comp Immunol, 2003 Jan;27(1):55-77 ("IMGT" numbering scheme), and Honegger A and Plückthun A, "Yet another numbering scheme for immunoglobulin variable domains: an automatic modeling and analysis tool," J Mol Biol, 2001 Jun 8;309(3):657-70, (AHo numbering scheme).

The boundaries of a given CDR may vary depending on the scheme used for identification. For example, the Kabat scheme is based structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. Table 1, below, lists the positions of CDRL1, CDRL2, CDRL3 and CDRH1, CDRH2, CDRH3 as identified by the Kabat, Chothia, and Contact schemes, respectively. For CDR-H1, residue numbering is given listed using both the Kabat and Chothia numbering schemes.

**Table 1 CDR Positions by different numbering schemes**

| **CDR** | **Kabat** | **Chothia** | **Contact** |
|---|---|---|---|
| CDR-L1 | L24--L34 | L24--L34 | L30--L36 |
| CDR-L2 | L50--L56 | L50--L56 | L46--L55 |
| CDR-L3 | L89--L97 | L89--L97 | L89--L96 |
| CDR-H1 (Kabat Numbering¹) | H31--H35B | H26--H32..34 | H30--H35B |
| CDR-H1 (Chothia Numbering²) | H31--H35 | H26--H32 | H30--H35 |
| CDR-H2 | H50--H65 | H52--H56 | H47--H58 |
| CDR-H3 | H95--H102 | H95--H102 | H93--H101 |

Thus, unless otherwise specified, the terms "CDR" and "complementary determining region" of a given antibody or region thereof, such as a variable region, as well as individual CDRs (*e.g*., "CDRH1, CDRH2) of the antibody or region thereof, should be understood to encompass the complementary determining region as defined by any of the known schemes described herein above. In some instances, the scheme for identification of a particular CDR or CDRs is specified, such as the CDR as defined by the Kabat, Chothia, or Contact method. In other cases, the particular amino acid sequence of a CDR is given.

As used herein, "treat" or "treatment" means stasis or a postponement of development of one or more symptoms associated with a disease or disorder described herein, or preventing additional symptoms, or ameliorating or preventing the underlying metabolic causes of symptoms. Thus, the terms denote that a beneficial result has been conferred on a mammalian subject with a disease or symptom, or with the potential to develop such disease or symptom. A response is achieved when the subject experiences partial or total alleviation, or reduction of one or more signs or symptoms of disease, condition, or illness, such as, but not limited to, prolongation of survival, or reduction of tumor progression, tumor growth, metastasis, invasion, or angiogenesis, or other symptom.

As used herein, "ameliorate" or "amelioration" means to reduce one or more symptoms associated with a disease or disorder described herein, including uncontrolled or unwanted symptoms. Thus, the terms denote an improved clinical state of a mammalian subject with a disease or symptom, such as a reduction in the severity of the symptom. An ameliorated response is achieved when the subject experiences partial alleviation, or reduction of one or more signs or symptoms of disease, condition or illness, such as, but not limited to, prolongation of survival, or reduction of tumor progression, tumor growth, metastasis, invasion, or angiogenesis, or other symptom.

As used herein, unless otherwise specified, the term "therapeutically effective amount" or "effective amount" refers to an amount of an agent or compound or composition that when administered (either alone or in combination with another therapeutic agent, as may be specified) to a subject is effective to prevent or ameliorate the disease condition or the progression of the disease, or result in amelioration of symptoms, e.g., treatment, healing, prevention or amelioration of the relevant medical condition, or an increase in rate of treatment, healing, prevention or amelioration of such conditions. When applied to an individual active ingredient administered alone, a therapeutically effective dose refers to that ingredient alone. When applied to a combination, a therapeutically effective dose refers to combined amounts of the active ingredients that result in the therapeutic effect, whether administered in combination, serially or simultaneously.

As used herein, the term "subject" means a mammalian subject. Exemplary subjects include, but are not limited to humans, monkeys, dogs, cats, mice, rats, cows, horses, goats and sheep. In some embodiments, the subject has cancer, an inflammatory disease or condition, or an autoimmune disease or condition, and can be treated with the agent of the present invention as described below.

As used herein, the term "biological sample" can refer to any of a variety of sample types obtained from an individual that can be used in a detection, diagnostic, prognostic, or monitoring assay. A liquid biological sample can include, for example, blood, a blood fraction (e.g., serum or plasma), urine, saliva, bronchoalveolar lavage, sputum, or cerebrospinal fluid. The definition also includes samples that have been manipulated in any way after their procurement, such as by treatment with reagents, solubilization, or enrichment for certain components, such as proteins.

"Axial flow" as used herein refers to lateral, vertical or transverse flow through a particular matrix or material comprising one or more test and/or control zones. The type of flow contemplated in a particular device, assay or method varies according to the structure of the device. Without being bound by theory, lateral, vertical or transverse flow may refer to flow of a fluid sample from the point of fluid contact on one end or side of a particular matrix (the upstream or proximal end) to an area downstream (or distal) of this contact. The downstream area may be on the same side or on the opposite side of the matrix from the point of fluid contact. For example, in vertical flow devices of certain embodiments of the present invention, axial flow may progress vertically from and through a first member (top to bottom) to a second member and from there on to an absorbent medium. By way of further example, and as will be appreciated by those of skill in the art, in a vertical flow device configured, for example, as a dipstick, a fluid sample may flow literally up the device, in which case however, the point of first contact of the fluid sample to the device is nonetheless considered the upstream (i.e., proximal) end and the point of termination of flow the downstream (i.e., distal) end.

As used herein the terms "upstream" and "downstream," in the context of axial flow, refer to the direction of fluid sample flow subsequent to contact of the fluid sample with a representative device of the present disclosure, wherein, under normal operating conditions, the fluid sample flow direction runs from an upstream position to a downstream position. For example, when fluid sample is initially contacted with the sample receiving zone, the fluid sample then flows downstream through the label zone and so forth.

Where a range of values is provided, it is understood that each intervening value, to the tenth of the unit of the lower limit unless the context clearly dictates otherwise, between the upper and lower limit of that range and any other stated or intervening value in that stated range, is encompassed. The upper and lower limits of these smaller ranges may independently be included in the smaller ranges, and are also encompassed, subject to any specifically excluded limit in the stated range. Where the stated range includes one or both of the limits, ranges excluding either or both of those included limits are also included.

It must be noted that as used herein and in the appended claims, the singular forms "a," "an," and "the" include plural referents unless the context clearly dictates otherwise. Thus, for example, reference to "a LOXL2-specific antibody" can include a plurality of such antibodies and reference to "the LOXL2 polypeptide" can include reference to one or more LOXL2 polypeptides and equivalents thereof known to those skilled in the art, and so forth.

It is appreciated that certain features of the invention, which are, for clarity, described in the context of separate embodiments, may also be provided in combination in a single embodiment. Conversely, various provided features, which are, for brevity, described in the context of a single embodiment, may also be provided separately or in any suitable sub-combination. All combinations of the provided embodiments are specifically embraced by the present disclosure and are disclosed herein just as if each and every combination was individually and explicitly disclosed. In addition, all sub-combinations of the various embodiments and elements thereof are also specifically embraced by the present disclosure and are disclosed herein just as if each and every such sub-combination was individually and explicitly disclosed herein.

### II. METHODS

LOXL2 promotes type I collagen cross-linking and is a core regulator of fibrogenesis in fibrosis of various etiologies and organs. See Mehal WZ, Iredale J, & Friedman SL., "Expressway to the core of fibrosis," Nat Med. 2011. 17: 552-553.

Allosteric inhibition of LOXL2 using a monoclonal antibody, e.g., AB0024, is efficacious in inhibiting fibrosis in a variety of disease models, including models of liver and lung (pulmonary) fibrosis. Inhibition of LOXL2 resulted in the down-regulation of TGFβ signaling and several key pro-fibrotic mediators (e.g. TGF-β1, CTGF, endothelin, CXCL12). Levels of circulating LOXL2 correlate with fibrotic stage. LOXL2 is a core pathway target in fibrotic disease. Mehal WZ, Iredale J, & Friedman SL., "Expressway to the core of fibrosis," Nat Med. 2011. 17: 552-553.

Lysyl oxidase-like 2 (LOXL2) is expressed in fibrotic human liver tissue, where it carries out cross-linking of collagen and other matrix components, resulting in increased stiffness, activation of pathologic fibroblasts and a dynamic process of matrix remodeling and fibrogenesis. Barry-Hamilton V, Spangler R, Marshall D, et al., "Allosteric inhibition of lysyl oxidase-like-2 impedes the development of a pathologic microenvironment," Nat Med. 2010. 16: 1009-1017. LOXL2 is expressed in fibrotic liver tissue from human diseases of diverse etiology, including hepatitis C infection, non-alcoholic steatohepatitis (NASH), alcoholic steatohepatitis (ASH), Wilson's disease (Vadasz Z, Kessler O, Akiri G, et al., "Abnormal deposition of collagen around hepatocytes in Wilson's disease is associated with hepatocyte specific expression of lysyl oxidase and lysyl oxidase like protein-2," J Hepatology. 2005. 43: 499-507), and primary biliary cirrhosis, in addition to mouse models of sclerosing cholangitis. Nakken KE, Nygard S, Haaland T, et al. "Multiple inflammatory-, tissue remodelling- and fibrosis genes are differentially transcribed in the livers of Abcb4 (-/-) mice harbouring chronic cholangitis," Scand J Gastroent. 2007. 42: 1245-1255.

Gradual accumulation of collagen in the hepatic parenchyma is a final common pathway of chronic liver disease. This progressive accumulation of fibrosis can ultimately lead to cirrhosis of liver and end-stage liver disease. LOXL2 catalyzes the cross linking of collagen fibrils and is a core regulatory protein of fibrogenesis. LOXL2 expression is increased in diseased liver tissue.

There is little LOXL2 expression in healthy adult tissues; and under normal (e.g., non-disease) conditions, the amount of circulating LOXL2 is low or undetectable. Under certain disease conditions, circulating LOXL2 is elevated. For example, LOXL2 can be elevated in the serum of patients with chronic liver disease, such as in chronic hepatitis C patients, with greater levels in patients with more advanced fibrosis. Detection of circulating LOXL2 is thus useful for determining whether an individual has a disease that results in elevated circulating LOXL2 levels. Such diseases include fibrosis and cancer.

It has been found that the level of circulating LOXL2 correlates with the stage of fibrosis. It has also been found that the level of circulating LOXL2 can provide an indication as to whether an individual having fibrosis is amenable to treatment for the fibrosis and provide other prognostic and predictive information regarding disease, such as the likelihood of a particular endpoint, outcome, or event, such as disease outcome or responsiveness to treatment. The present disclosure provides methods for determining the likelihood that an individual will respond to treatment for a fibrotic disease and/or the likelihood of such and outcome, endpoint, or event.

Treatment decisions for patients with HCV infection are increasingly based on non-invasive serum tests rather than liver biopsies. However, serum tests have not been entirely optimal. *See* Castera, L., "Invasive and non-invasive methods for the assessment of fibrosis and disease progression in chronic liver disease," Best Pract Res Clin Gastroent. 2011. 25: 291-303.

In some embodiments, provided are therapeutic and diagnostic methods related to LOXL2. For example, provided are detection, diagnostic, prognostic, and predictive methods for determining whether an individual has a disease associated with elevated circulating LOXL2 levels. Detection of circulating LOXL2 can be followed up with other diagnostic methods, to confirm a diagnosis or to exclude the possibility that an individual has a particular disease. Thus, in some embodiments, the present disclosure provides an assay to detect and/or quantify LOXL2, generally circulating lysyl oxidase-like 2 (LOXL2) polypeptides in an individual. The assay is useful, for example, in diagnostic and prognostic applications, which are also provided.

Also provided are methods for treating and/or ameliorating one or more symptoms of a disease or condition, such as a fibrotic disease or condition, e.g., liver disease or other LOXL2-associated disease or condition. In general, the methods are carried out by administering an agent that inhibits of and/or that binds to a lysyl oxidase-like 2 (LOXL2).

### III. AGENTS

The provided methods generally are carried out using one or more agents, such as those agents that specifically bind to, detect, and/or inhibit LOXL2. Such agents can include, for example, antibodies that specifically bind to LOXL2, including antibody fragments, small molecule inhibitors, siRNA, shRNA, and antisense polynucleotides. In some aspects, the agents, e.g., antibodies, are noncompetitive, uncompetitive, or competitive inhibitors, e.g., of LOXL2. In some instances, the agent that inhibits enzymatic activity of a LOXL2 polypeptide. Agents that inhibit LOXL2 enzymatic activity include an allosteric inhibitor of LOXL2 enzymatic activity.

### A. ANTIBODIES

Thus, in some embodiments, the agents are antibodies that specifically bind to a LOXL2 protein, including antibody fragments, polyclonal antibodies, and monoclonal antibodies. Among the antibodies for use in the provided methods are polyclonal antibodies, monoclonal antibodies, human antibodies, humanized antibodies, chimeric antibodies, nanobodies, diabodies, multispecific antibodies (e.g., bispecific antibodies), and antigen-binding antibody fragments.

In some aspects, the antibodies bind specifically to a LOXL2 polypeptide, e.g., where specific binding refers to binding with an affinity of at least about 10⁻⁷ M, at least about 10⁻⁸ M, at least about 10⁻⁹ M, at least about 10⁻¹⁰ M, at least about 10⁻¹¹ M, or at least about 10⁻¹² M, or greater than 10⁻¹² M. In some aspects, non-specific binding refers to binding with an affinity of less than about 10⁻⁷ M, e.g., binding with an affinity of 10⁻⁶ M, 10⁻⁵ M, 10⁻⁴ M, etc.

Non-limiting examples of LOXL2-specific antibodies include the LOXL2-specific antibodies disclosed in U.S. Patent Publication Nos. 2009/0104201, 2009/0053224, 2012/0087917, 2011/0200606, and International Patent Application Publication Nos. WO 2011/097513, WO 2009/017833, and WO 2009/035791.

In some aspects, the antibodies are non-competitive inhibitors and/or allosteric inhibitors of LOXL2, specifically bind to an epitope of LOXL2 outside the catalytic domain. In certain aspects, the antibodies are non-competitive inhibitors and/or allosteric inhibitors of LOXL2, specifically bind to an epitope within LOXL2. In certain aspects, the antibodies are non-competitive inhibitors that specifically bind to an epitope of LOXL2 within the SRCR3-4 domain of LOXL2, for example, within a region having the sequence set forth as SEQ ID: 19 (VRLRGGAYIGEGRVEVLKNGEWGTVCDDKWDLVSASVVCRELGFGSAKEAVTGSRL GQGIGPIHLNEIQCTGNEKSIIDCKFNAESQGCNHEEDAGVRCNLRLNGGRNPYEGRVE VLVERNGSLVWGMVCGQNWGIVEAMWCRQLGLGFASNAFQETWYWHGDVNSNKV VMSGVKCSGTELSLAHCRHDGEDVACPQGGVQYGAGVACS). Non-limiting examples include AB0023 and AB0024. In another aspects, the antibodies are competitive or uncompetitive inhibitors of LOXL2, specifically bind to an epitope of LOXL2 outside the catalytic domain. In certain other aspects, the antibodies are competitive or uncompetitive inhibitors that specifically bind to an epitope of LOXL2 catalytic domain. In other aspects, the antibodies are competitive inhibitors of LOXL2, specifically bind to an epitope within LOXL2.

For example, in some embodiments, the antibody is an antibody having a variable heavy chain region of AB0023:
MEWSRVFIFLLSVTAGVHSQVQLQQSGAELVRPGTSVKVSCKASGYAFTYYLIEWVKQ RPGQGLEWIGVINPGSGGTNYNEKFKGKATLTADKSSSTAYMQLSSLTSDDSAVYFCA RNWMNFDYWGQGTTLTVSS (SEQ ID NO: 6; sequences of CDR1, CDR2, and CDR3 underlined). In some embodiments, the antibody has a heavy chain variable region having an amino acid sequence with 75% or more, 80% or more, 90% or more, 95% or more, or 99% or more homology to SEQ ID NO:6. In some embodiments, the antibody has a heavy chain variable region with a CDR1, CDR2, and/or CDR3 of the variable region sequence set forth in SEQ ID NO: 6.

In some embodiments, the antibody is an antibody having a variable light chain region of AB0023:
MRCLAEFLGLLVLWIPGAIGDIVMTQAAPSVSVTPGESVSISCRSSKSLLHSNGNTYLYW FLQRPGQSPQFLIYRMSNLASGVPDRFSGSGSGTAFTLRISRVEAEDVGVYYCMQHLEY PYTFGGGTKLEIK (SEQ ID NO:7, sequences of CDR1, CDR2, and CDR3 underlined). In some embodiments, the antibody has a light chain variable region having an amino acid sequence with 75% or more, 80% or more, 90% or more, 95% or more, or 99% or more homology to SEQ ID NO: 7. In some embodiments, the antibody has a light chain variable region with CDR1, CDR2, and/or CDR3 of the variable region sequence set forth in SEQ ID NO: 7. In some embodiments, the antibody has a heavy chain variable region with CDR1, CDR2, and/or CDR3 of the variable region sequence set forth in SEQ ID NO: 6 and a light chain variable region with CDR1, CDR2, and/or CDR3 of the variable region sequence set forth in SEQ ID NO: 7. In some embodiments, the antibody competes for binding with AB0023 and/or an antibody having a heavy chain of SEQ ID NO: 6 and/or a light chain of SEQ ID NO: 7.

In some embodiments, the antibody is a humanized version of such an antibody, such as the antibody designated AB0024, an antibody that competes for binding with or binds to the same epitope as such an antibody, and/or one having a heavy or light chain variable region of such an antibody or having a heavy chain having the CDRs (CDR1, CDR2, and CDR3) of AB0024 and/or having a light chain having the CDRs (COR1, CDR2, and CDR3) of AB0024.

For example, in one embodiment, the antibody is an antibody having the variable heavy chain region of AB0024:
QVQLVQSGAEVKKPGASVKVSCKASGYAFTYYLIEWVRQAPGQGLEWIGVINPGSGGT NYNEKFKGRATITADKSTSTAYMELSSLRSEDTAVYFCARNWMNFDYWGQGTTVTVS S(SEQ ID NO:8, with the sequences of CDR1, CDR2, and CDR3 underlined). In some embodiments, the antibody has a heavy chain variable region having an amino acid sequence with 75% or more, 80% or more, 90% or more, 95% or more, or 99% or more homology to SEQ ID NO: 8. In some embodiments, the antibody has a heavy chain variable region with CDR1, CDR2, and/or CDR3 of the variable region sequence set forth in SEQ ID NO: 8.

In some embodiments, the antibody has a heavy chain variable region having the amino acid sequence set forth in SEQ ID NO: 8, SEQ ID NO: 10
(QVQLVQSGAELKKPGASVKVSCKASGYAFTYYLIEWVKQAPGQGLEWIGVINPGSGG TNYNEKFKGRATLTADKSTSTAYMELSSLRSEDSAVYFCARNWMNFDYWGQGTTVTV SS), SEQ ID NO: 11
(QVQLVQSGAEVKKPGASVKVSCKASGYAFTYYLIEWVRQAPGQGLEWIGVINPGSGG TNYNEKFKGRATLTADKSTSTAYMELSSLRSEDTAVYFCARNWMNFDYWGQGTTVTV SS), or SEQ ID NO: 12
(QVQLVQSGAEVKKPGASVKVSCKASGYAFTYYLIEWVRQAPGQGLEWIGVINPGSGG TNYNEKFKGRVTITADKSTSTAYMELSSLRSEDTAVYYCARNWMNFDYWGQGTTVTV SS) or an amino acid having 75% or more, 80% or more, 90% or more, 95% or more, or 99% or more homology to SEQ ID NO: 8, 10, 11, or 12, and/or a light chain variable region having the amino acid sequence set forth in SEQ ID NO: 9, SEQ ID NO: 13
(DIVMTQTPLSLSVTPGQPASISCRSSKSLLHSNGNTYLYWFLQKPGQSPQFLIYRMSNLA SGVPDRFSGSGSGTAFTLKISRVEAEDVGVYYCMQHLEYPYTFGGGTKVEIK), or SEQ ID NO: 14
(DIVMTQTPLSLSVTPGQPASISCRSSKSLLHSNGNTYLYWYLQKPGQSPQFLIYRMSNL ASGVPDRFSGSGSGTDFTLKISRVEAEDVGVYYCMQHLEYPYTFGGGTKVEIK), or an amino acid having 75% or more, 80% or more, 90% or more, 95% or more, or 99% or more homology to SEQ ID NO: 9, 13, or 14.

In some embodiments, the antibody contains a light chain having an amino acid sequence having 75% or more, 80% or more, 90% or more, 95% or more, or 99% or more identity to SEQ ID NO: 22, or having the amino acid sequence of SEQ ID NO: 22. In some aspects, the light chain has an amino acid sequence having 75% or more, 80% or more, 90% or more, 95% or more, or 99% or more identity to SEQ ID NO: 20 or has an amino acid sequence of SEQ ID NO: 20.

In some embodiments, the antibody contains a heavy chain having an amino acid sequence having 75% or more, 80% or more, 90% or more, 95% or more, or 99% or more identity to SEQ ID NO: 26 or having an amino acid sequence of SEQ ID NO: 26. In some aspects, the light chain has an amino acid sequence having 75% or more, 80% or more, 90% or more, 95% or more, or 99% or more identity to SEQ ID NO: 24, or having an amino acid sequence of SEQ ID NO: 24. For example, in some cases, the antibody has a light chain with identity to SEQ ID NO: 22 and a heavy chain having identity to SEQ ID NO: 26. In some cases, the antibody has a light chain with identity to SEQ ID NO: 20 and a heavy chain having identity to SEQ ID NO: 24.

In some aspects, the light chain contains an amino acid sequence encoded by SEQ ID NO: 21 or SEQ ID NO: 23. In some aspects, the heavy chain contains an amino acid sequence encoded by SEQ ID NO: 25 or 27.

In some embodiments, the antibody contains an amino acid sequence (or has 75% or more, 80% or more, 90% or more, 95% or more, or 99% or more identity to an amino acid sequence) of SEQ ID NO: 28 or 29. In some embodiments, the antibody contains a constant region having 75% or more, 80% or more, 90% or more, 95% or more, or 99% or more identity to or containing the amino acid sequence set forth as SEQ ID NO: 30.

In some embodiments, the antibody is an antibody having a variable light chain region of AB0024:
DIVMTQTPLSLSVTPGQPASISCRSSKSLLHSNGNTYLYWFLQKPGQSPQFLIYRMSNLA SGVPDRFSGSGSGTDFTLKISRVEAEDVGVYYCMQHLEYPYTFGGGTKVEIK (SEQ ID NO: 9, sequences of CDR1, CDR2, and CDR3 underlined). In some embodiments, the antibody has a light chain variable region having an amino acid sequence with 75% or more, 80% or more, 90% or more, 95% or more, or 99% or more homology to SEQ ID NO: 9. In some embodiments, the antibody has a light chain variable region with CDR1, CDR2, and/or CDR3 of the variable region sequence set forth in SEQ ID NO: 9. In some embodiments, the antibody has a heavy chain variable region with CDR1, CDR2, and/or CDR3 of the variable region sequence set forth in SEQ ID NO: 8 and a light chain variable region with CDR1, CDR2, and/or CDR3 of the variable region sequence set forth in SEQ ID NO: 9. In some aspects, the antibody has a light chain containing SEQ ID NO: 20 or 22 and a heavy chain containing SEQ ID NO: 24 or 26.

Whether an agent inhibits LOXL2 enzymatic activity can be determined using any known assay. For example, an assay for LOXL2 enzymatic activity can be carried out using diaminopentane (DAP) as a substrate, or using collagen as a substrate. In both assays, enzymatic activity of LOXL2 can be measured using an assay that couples production of hydrogen peroxide (liberated by LOXL2 upon deamination of substrate) to horseradish peroxidase-catalyzed conversion of Amplex® Red (Invitrogen, Carlsbad, CA) to resorufin (a fluorescent product).

In some embodiments, a suitable anti-LOXL2 antibody inhibits enzymatic activity of a LOXL2 polypeptide. In other embodiments, a suitable anti-LOXL2 antibody does not inhibit enzymatic activity of a LOXL2 polypeptide.

Thus, exemplary of the anti-LOXL2 antibodies for use in connection with the provided methods and embodiments include, for example, AB0023, AB0024, antibodies having a heavy chain variable region with an amino acid sequence as forth in SEQ ID NO: 6, 8, 10, 11, or 12, or with 75% or more, 80% or more, 90% or more, 95% or more, or 99% or more homology to SEQ ID NO:6, 8, 10, 11, or 12, or with a CDR1, CDR2, and/or CDR3 of the variable region sequence set forth in SEQ ID NO: 6, 8, 10, 11, or 12, and/or having a variable light chain region having the amino acid sequence set forth in SEQ ID NO: 7, 9, 13, or 14, or with 75% or more, 80% or more, 90% or more, 95% or more, or 99% or more homology to SEQ ID NO: 7 or with a CDR1, CDR2, and/or CDR3 of the variable region sequence set forth in SEQ ID NO: 7; 9, 13, or 14, such as an antibody with a heavy chain having the CDR1, CDR2, and/or CDR3 or the entire sequence of the variable region sequence set forth in SEQ ID NO: 8 and a light chain variable region with the CDR1, CDR2, and/or CDR3 or the entire sequence of the variable region sequence set forth in SEQ ID NO: 9.

In some embodiments, the antibody specifically binds an epitope in the LOXL2 SRCR1 domain, in the LOXL2 SRCR2 domain, in the LOXL2 SRCR3 domain, in the LOXL2 SRCR4 domain, and/or in the LOXL2 catalytic domain. In some cases, an antibody binds to a full-length LOXL2 polypeptide without the signal sequence, e.g., including SRCR1-2, SRCR3-4, and the catalytic domain. In some instances, an antibody binds to mature LOXL2 polypeptide (i.e., without the signal sequence and without SRCR1-2), including only the SRCR3-4 domain and the catalytic domain. In other instances, an antibody binds to an N-terminal LOXL2 fragment, which N-terminal LOXL2 fragment includes the SRCR1-2 domains and not the SRCR3-4 or catalytic domains.

In some instances, a suitable antibody specifically binds an epitope in the LOXL2 catalytic domain, such as an antibody that binds to a catalytic domain comprising the amino acid sequence of SEQ ID NO: 2, 3, 4, or 5. In some instances, a suitable antibody (e.g., a polyclonal antibody) specifically binds multiple epitopes in one, two, three, or more LOXL2 domains.

In some embodiments, the antibody specifically binds to an epitope within the SRCR3-linker-SRCR4 region ("SRCR3-4"), for example, an SRCR3-4 region including an amino acid sequence with at least about 90%, at least about 95%, at least about 98%, at least about 99%, or 100%, amino acid sequence identity with amino acids 325 to 544, with amino acids 325 to 547, with amino acids 303 to 544, or with amino acids 303 to 547, of SEQ ID NO:1; an epitope within the linker-SRCR3-linker-SRCR4-linker region, such as an epitope within an amino acid sequence that has at least about 90%, at least about 95%, at least about 98%, at least about 99%, or 100%, amino acid sequence identity with amino acids 303 to 544, amino acids 303 to 545, amino acids 303 to 546, or amino acids 303 to 547 of SEQ ID NO:1; an epitope within the SRCR3-linker-SRCR4-linker region, such as an epitope within an amino acid sequence that has at least about 90%, at least about 95%, at least about 98%, at least about 99%, or 100%, amino acid sequence identity with amino acids 325 to 544, amino acids 325 to 545, amino acids 325 to 546, or amino acids 325 to 547, of SEQ ID NO:1; an epitope within the SRCR3 region (and not within SRCR4), such as an SRCR3 region including an amino acid sequence that has at least about 90%, at least about 95%, at least about 98%, at least about 99%, or 100%, amino acid sequence identity with amino acids 325 to 425, with amino acids 303 to 425, with amino acids 303 to 434, or with amino acids 325 to 434, of SEQ ID NO:1; an epitope within the linker-SRCR3 region, such as an epitope within an amino acid sequence that has at least about 90%, at least about 95%, at least about 98%, at least about 99%, or 100%, amino acid sequence identity with amino acids 303 to 425 of SEQ ID NO:1; an epitope within the SRCR3-linker region, such as an epitope within an amino acid sequence that has at least about 90%, at least about 95%, at least about 98%, at least about 99%, or 100%, amino acid sequence identity with amino acids 325 to 434 of SEQ ID NO:1; an epitope within the linker-SRCR3-linker region, such as an epitope within an amino acid sequence that has at least about 90%, at least about 95%, at least about 98%, at least about 99%, or 100%, amino acid sequence identity with amino acids 303 to 434 of SEQ ID NO:1; an epitope within the linker-SRCR4-linker region, such as an epitope within an amino acid sequence that has at least about 90%, at least about 95%, at least about 98%, at least about 99%, or 100%, amino acid sequence identity with amino acids 426 to 544, amino acids 426 to 545, amino acids 426 to 546, or amino acids 426 to 547, of SEQ ID NO:1; an epitope within the SRCR4 region (and not within SRCR3), such as an SRCR4 region including an amino acid sequence that has at least about 90%, at least about 95%, at least about 98%, at least about 99%, or 100%, amino acid sequence identity with amino acids 435 to 544, amino acids 435 to 545, amino acids 435 to 546, or with amino acids 435 to 547, of SEQ ID NO:1; an epitope within the SRCR1-linker-SRCR2 region ("SRCR1-2"), such as an SRCR1-2 region including an amino acid sequence having at least about 90%, at least about 95%, at least about 98%, at least about 99%, or 100%, amino acid sequence identity with amino acids 58 to 302, or 58 to 324, of the amino acid sequence depicted in SEQ ID NO:1; an epitope within an amino acid sequence that has at least about 90%, at least about 95%, at least about 98%, at least about 99%, or 100%, amino acid sequence identity with amino acids 58 to 324 of the amino acid sequence depicted in SEQ ID NO:1; an epitope within the SRCR1 region (and not within SRCR2) such as an SRCR1 region including an amino acid sequence that has at least about 90%, at least about 95%, at least about 98%, at least about 99%, or 100%, amino acid sequence identity with amino acids 58 to 159 of the amino acid sequence depicted in SEQ ID NO:1; an epitope within the SRCR1-linker region, such as an SRCR1-linker region including an amino acid sequence that has at least about 90%, at least about 95%, at least about 98%, at least about 99%, or 100%, amino acid sequence identity with amino acids 58 to 187 of the amino acid sequence depicted in SEQ ID NO:1; or an epitope within the SRCR1 region (and not within SRC2), where an SRCR2 region can comprise an amino acid sequence that has at least about 90%, at least about 95%, at least about 98%, at least about 99%, or 100%, amino acid sequence identity with amino acids 188 to 302 of the amino acid sequence depicted in SEQ ID NO:1. In certain aspects, the antibody specifically binds to an epitope within the SRCR3-linker-SRCR4 region of human LOXL2, for example, within a region having the sequence set forth in SEQ ID NO 15 (VWGMVCGQNWGIVEA), SEQ ID NO: 17 (VEAMWCRQLGLGFA), or SEQ ID NO: 18 (GFASNAFQETWYWHG).

Other non-limiting examples include a monoclonal antibody AB0030 (RPDS1-M20), which binds specifically an epitope in the LOXL2 catalytic domain (see, e.g., US 2009/0053224, where antibody AB0030 corresponds to proBM20), RPDS-1M1, RPDS-1M3, RPDS-1M8, RPDS-1M9, RPDS-1M11, RPDS-1M15, RPDS-1M17, RPDS-1M19, RPDS-1M20 (AB0030), RPDS-1M22, RPDS-1M24, RPDS-1M25, RPDS-1M27, RPDS-1M28, RPDS-1M29, RPDS-1M30, RPDS-1M31, RPDS-1M32, RPDS-2M1, RPDS-2M2, RPDS-2M3, RPDS-2M4, RPDS-2M5, RPDS-2M6, RPDS-2M7, RPDS-2M8, RPDS-2M9, RPDS-2M10, RPDS-2M11, RPDS-2M12, RPDS-2M13, RPDS-2M14, RPDS-2M15, RPDS-2M16, RPDS-2M17, RPDS-2M18, and RPDS-2M19. See International Patent Application Publication No. WO 2011/097513 and monoclonal antibodies RPDS-1M1, RPDS-1M3, RPDS-1M8, RPDS-1M9, RPDS-1M11, RPDS-1M15, RPDS-1M17, RPDS-1M19, RPDS-1M20 (AB0030), RPDS-1M22, RPDS-1M24, RPDS-1M25, RPDS-1M27, RPDS-1M28, RPDS-1M29, RPDS-1M30, RPDS-1M31, RPDS-1M32, RPDS-2M1, RPDS-2M2, RPDS-2M3, RPDS-2M4, RPDS-2M5, RPDS-2M6, RPDS-2M7, RPDS-2M8, RPDS-2M9, RPDS-2M10, RPDS-2M11, RPDS-2M12, RPDS-2M13, RPDS-2M14, RPDS-2M15, RPDS-2M16, RPDS-2M17, RPDS-2M18, and RPDS-2M19, for example, those that bind within the catalytic domain of LOXL2. In some embodiments, the antibodies include those listed in the following table and/or antibodies that compete for binding with such antibodies.

| **Material Deposited** | **Date of Deposit** | **Accession Number** |
|---|---|---|
| RPDS1-M20 (AB0030) | March 26, 2010 | 050210-04 |
| RPDS-1M19 | March 26, 2010 | 050210-02 |
| RPDS-1M21 | March 26, 2010 | 050210-03 |
| RPDS-1M27 | March 26, 2010 | 050210-01 |
| RPDS1-M31 | March 26, 2010 | 260310-01 |
| RPDS-2M2 | March 26, 2010 | 260310-02 |
| RPDS-2M4 | March 26, 2010 | 260310-03 |

In some embodiments, the LOXL2-specific antibody does not substantially bind to any other lysyl oxidase-like polypeptide other than a LOXL2 polypeptide, e.g., a LOXL2-specific antibody does not substantially bind to a LOXL1, LOXL3, or LOXL4 polypeptide, or to a lysyl oxidase (LOX) polypeptide.

In some embodiments, the antibody specifically binds to LOXL2 when LOXL2 is bound to an agent that inhibits LOXL2 enzymatic activity. Agents that inhibit LOXL2 enzymatic activity include an allosteric inhibitor of LOXL2 enzymatic activity. In some cases, the allosteric inhibitor is an anti-LOXL2 monoclonal antibody, e.g., an anti-LOXL2 monoclonal antibody that binds an epitope within an "SRCR3-4" domain of LOXL2. Non-limiting examples of a monoclonal antibody that inhibits LOXL2 enzymatic activity, and that binds an epitope within an SRCR3-4 domain, are AB0023 and AB0024; see, e.g., US 2009/0053224. In some embodiments, a suitable anti-LOXL2 antibody: a) specifically binds an epitope within SRCR3-4; and ii) does not compete with an AB0023 antibody and/or an AB0024 antibody for binding to an epitope within SRCR3-4.

In some embodiments, a LOXL2-specific antibody binds an epitope(s) that is accessible for binding when the LOXL2 polypeptide is in a liquid biological sample, e.g., the epitope(s) bound by the LOXL2-specific antibody is surface accessible and/or not masked by one or more non-LOXL2 proteins that may be present in the liquid biological sample.

In some cases, the anti-LOXL2 antibody includes a detectable label. Suitable detectable labels include, but are not limited to, magnetic beads (e.g. Dynabeads™), fluorescent dyes (e.g., fluorescein isothiocyanate, texas red, rhodamine, a green fluorescent protein, a red fluorescent protein, a yellow fluorescent protein, and the like), radiolabels (e.g., ³H, ¹²⁵I, ³⁵S, ¹⁴C, or ³²P), enzymes (e.g., horse radish peroxidase, alkaline phosphatase, luciferase, and other enzymes commonly used in an enzyme-linked immunosorbent assay (ELISA)), and colorimetric labels such as colloidal gold or colored glass or plastic (e.g. polystyrene, polypropylene, latex, etc.) beads.

In some aspects, where an anti-LOXL2 antibody comprises a detectable label, the anti-LOXL2 antibody can be detected by detecting a signal produced by the label (e.g., a chromophore, luminophore, etc., produced as a product of an enzyme attached to the anti-LOXL2 antibody; a signal produced directly by the label; etc.). In some cases, an anti-LOXL2 antibody does not comprise a detectable label but may be detected using a secondary antibody comprising a detectable label. Suitable secondary antibodies include monoclonal and polyclonal antibodies specific for epitope(s) in the constant region domain(s) of an anti-LOXL2 antibody. A secondary antibody can comprise any of a variety of detectable labels, including, but not limited to, magnetic beads (e.g. Dynabeads™), fluorescent dyes (e.g., fluorescein isothiocyanate, texas red, rhodamine, a green fluorescent protein, a red fluorescent protein, a yellow fluorescent protein, and the like), radiolabels (e.g., ³H, ¹²⁵I, ³⁵S, ¹⁴C, or ³²P), enzymes (e.g., horse radish peroxidase, alkaline phosphatase, luciferase, and other enzymes commonly used in an enzyme-linked immunosorbent assay (ELISA)), and colorimetric labels such as colloidal gold or colored glass or plastic (e.g. polystyrene, polypropylene, latex, etc.) beads. Also suitable for use as a detectable label is the SULFO-TAG™ label from MesoScale Discovery. The SULFO-TAG™ label is a ruthenium(II) tris-bipyridal tag, which can be attached to a polypeptide (e.g., a secondary antibody) via reaction of Ruthenium (II) tris-bipyridine-(4-methylsulfone) N-hydroxysuccinimide (NHS)-ester with a primary amine (e.g., a lysine side chain).

In some instances, the anti-LOXL2 antibody, e.g., for use in a provided detection or assay method, is immobilized on an insoluble support. Suitable insoluble supports can comprise various materials including, but not limited to, polyvinyl difluoride (PVDF), cellulose, nitrocellulose, nylon, glass, polystyrene, polyvinyl chloride, polypropylene, silicon dioxide, polyethylene, polycarbonate, dextran, amylose, natural and modified celluloses, polyacrylamides, silica embedded in a polyacrylamide gel, agaroses, gabbros, magnetite, and the like. The insoluble support can be in any of a variety of formats (e.g., dimensions, shapes), e.g., sheets, such as used in a test strip; a dipstick assay format; a multi-well plate (e.g., such as those used in an ELISA); and the like.

### IV. SUBJECTS

In some embodiments, the provided methods are carried by administering agents to subjects, e.g., those having, being suspected of having, having been diagnosed with, and/or who is undergoing or has undergone treatment for a particular disease or condition. In some embodiments, the methods are carried out using samples obtained from such subjects. Among the diseases and conditions are cancer, fibrosis, fibrotic diseases and conditions, liver diseases, and diseases and conditions associated with LOXL2. Among the subjects are those that have the disease or condition but have not yet been diagnosed with it and those currently or previously being treated for the disease or condition.

### A. FIBROSIS

In some embodiments, the disease or condition is fibrosis or a fibrotic disease or condition e.g., liver fibrosis, kidney fibrosis, pulmonary fibrosis, myelofibrosis, cardiac fibrosis, or other type of fibrosis. In some cases, the disease or condition is associated with desmoplasia. Fibrosis can include abnormal accumulation of fibrous tissue that can occur, e.g., as a part of the wound-healing process in damaged tissue, which can result, for example, from physical injury, inflammation, infection, exposure to toxins, and other causes. Examples of fibrosis include dermal scar formation, keloids, liver fibrosis, lung fibrosis (e.g., silicosis, asbestosis), kidney fibrosis (including diabetic nephropathy), scleroderma, and glomerulosclerosis.

### 1. Liver disease and liver fibrosis

In some embodiments, the disease or condition is a liver disease or condition, such as a fibrotic liver disease or condition or liver (hepatic) fibrosis, for example, any hepatic fibrosis, regardless of underlying liver disease. Liver fibrosis and liver diseases associated with fibrosis include, but are not limited to, hepatitis C virus (HCV), NASH (nonalcoholic steatohepatitis), PSC (primary sclerosing cholangitis), cirrhosis, liver fibrosis, and portal hypertension, and can also include PBC (primary biliary cirrhosis), autoimmune hepatitis, alcoholic cirrhosis, alpha 1 antitrypsin deficiency disease, hereditary hemochromatosis, Wilson's disease, hepatitis B virus (HBV), and HIV associated steatohepatitiscirrhosis, and associated conditions such as chronic viral hepatitis, non-alcoholic fatty liver disease (NAFLD), alcoholic steatohepatitis (ASH), non-alcoholic steatohepatitis (NASH), primary biliary cirrhosis (PBC), biliary cirrhosis, primary sclerosing cholangitis, and autoimmune hepatitis. In certain embodiments, the disease or condition is non-alcoholic steatohepatitis (NASH), primary biliary cirrhosis (PBC), or primary sclerosing cholangitis (PSC). In some embodiments, the disease or condition is a viral hepatitic disease or condition that is acute or chronic. Among the exemplary diseases and conditions are hepatitis C virus (HCV), hepatitis B virus (HBV), with or without HCV infection- or HBV infection-associated liver damage. Thus, among the provided methods are methods for antifibrotic therapy in patients with liver disease, such as viral hepatitis. In some aspects, the liver disease is compenstated liver disease. On other aspects, it is decompenstated liver disease, such as liver disease associated with ascites, esophageal varices, encephalopathy, and/or jaundice.

Liver (hepatic) fibrosis is implicated in the pathology of numerous hepatic diseases and can occur as can occur as a part of the wound-healing response to chronic liver injury, as a complication of haemochromatosis, Wilson's disease, alcoholism, schistosomiasis, viral hepatitis, bile duct obstruction, exposure to toxins, and metabolic disorders. Liver fibrosis is characterized by the accumulation of extracellular matrix that can be distinguished qualitatively from that in normal liver. Left unchecked, hepatic fibrosis progresses to cirrhosis (defined by the presence of encapsulated nodules), liver failure, and death. Chronic insults to the liver from sources including parasites and viral infection (e.g. hepatitis B virus (HBV), HCV, human immunodeficiency virus (HIV), schistosomiasis) or the long term stress from alcohol consumption typically result in remodeling of the liver, presumably to encapsulate the damaged area and protect the remaining liver tissue from damage. (Li and Friedman, Gastroenterol. Hepatol. 14:618-633, 1999). Liver fibrosis results in extracellular matrix changes, including 3-10 fold increases in total collagen content and replacement of the low density basement membrane with high-density matrix, which impair the metabolic and synthesis function of hepatocytes, hepatic stellate cells and endothelial cells. (Girogescu, M., Non-invasive Biochemical Markers of Liver Fibrosis, J. Gastrointestin. Liver Dis., 15(2): 149-159 (2006)).

Gradual accumulation of collagen in the hepatic parenchyma is a final common pathway of chronic liver disease. This progressive accumulation of fibrosis can ultimately lead to cirrhosis of liver and end-stage liver disease. LOXL2 expression is increased in diseased liver tissue.

Therapeutic strategies for liver fibrosis include removal of the underlying cause (e.g., toxin or infectious agent), suppression of inflammation (using, e.g., corticosteroids, IL-1 receptor antagonists, or other agents), down-regulation of stellate cell activation using, e.g., gamma interferon or antioxidants), promotion of matrix degradation, or promotion of stellate cell apoptosis. Li and Friedman (Gastroenterol. Hepatol. 14:618-633, 1999). Treatments are needed that address the underlying biochemical process as opposed to merely suppressing inflammation. Embodiments of the provided methods address this need.

### a. Liver fibrosis scoring and staging

A number of standardized scoring systems exist which provide a quantitative assessment of the degree and severity of liver fibrosis. These include the METAVIR, Knodell, Scheuer, Ludwig, and Ishak scoring systems. Individuals with liver fibrosis include individuals with any degree or severity of liver fibrosis, based on any of the METAVIR, Knodell, Scheuer, Ludwig, and Ishak scoring systems.

The METAVIR scoring system is based on an analysis of various features of a liver biopsy, including fibrosis (portal fibrosis, centrilobular fibrosis, and cirrhosis); necrosis (piecemeal and lobular necrosis, acidophilic retraction, and ballooning degeneration); inflammation (portal tract inflammation, portal lymphoid aggregates, and distribution of portal inflammation); bile duct changes; and the Knodell index (scores of periportal necrosis, lobular necrosis, portal inflammation, fibrosis, and overall disease activity). The definitions of each stage in the METAVIR system are as follows: score: 0, no fibrosis; score: 1, stellate enlargement of portal tract but without septa formation; score: 2, enlargement of portal tract with rare septa formation; score: 3, numerous septa without cirrhosis; and score: 4, cirrhosis.

Knodell's scoring system, also called the Histology Activity Index, classifies specimens based on scores in four categories of histologic features: I. Periportal and/or bridging necrosis; II. Intralobular degeneration and focal necrosis; III. Portal inflammation; and IV. Fibrosis. In the Knodell staging system, scores are as follows: score: 0, no fibrosis; score: 1, mild fibrosis (fibrous portal expansion); score: 2, moderate fibrosis; score: 3, severe fibrosis (bridging fibrosis); and score: 4, cirrhosis. The higher the score, the more severe the liver tissue damage. Knodell (1981) Hepatol. 1:431. See Knodell RG, Conrad ME, Ishak KG. Development of chronic liver disease after acute non-A, non-B, post transfusion hepatitis. Gastroenterology 1977;72:902-909. In some embodiments, scoring includes analyzing overall Knodell necroinflammatory index, and/or individual components thereof, susch as Knodell inflammation score and/or necrosis score.

In the Scheuer scoring system scores are as follows: score: 0, no fibrosis; score: 1, enlarged, fibrotic portal tracts; score: 2, periportal or portal-portal septa, but intact architecture; score: 3, fibrosis with architectural distortion, but no obvious cirrhosis; score: 4, probable or definite cirrhosis. Scheuer (1991) J. Hepatol. 13:372.

The Ishak scoring system is described in Ishak (1995) J. Hepatol. 22:696-699. Stage 0, No fibrosis; Stage 1, Fibrous expansion of some portal areas, with or without short fibrous septa; stage 2, Fibrous expansion of most portal areas, with or without short fibrous septa; stage 3, Fibrous expansion of most portal areas with occasional portal to portal (P--P) bridging; stage 4, Fibrous expansion of portal areas with marked bridging (P--P) as well as portal-central (P--C); stage 5, Marked bridging (P--P and/or P--C) with occasional nodules (incomplete cirrhosis); stage 6, Cirrhosis, probable or definite.

In some aspects, the liver disease or fibrosis is assessed by determining the Model for End-stage Liver Disease (MELD) score. In some aspects, the methods predict or determine that or the likelihood that the individual has or has at least a particular MELD score.

In some aspects, the liver disease is determined to be compenstated or decompensated liver disease. For example, decompenstated liver disease may be associated with ascites, esophageal varices, encephalopathy, and/or jaundice.

### 2. Kidney fibrosis

In some embodiments, the disease or condition is or is associated with kidney fibrosis. Like liver fibrosis, kidney fibrosis can result from various diseases and insults to the kidneys. Examples of such diseases and insults include chronic kidney disease, metabolic syndrome, vesicoureteral reflux, tubulointerstitial renal fibrosis, diabetes (including diabetic nephropathy), and resultant glomerular nephritis (GN), including, but not limited to, focal segmental glomerulosclerosis and membranous glomerulonephritis, mesangiocapillary GN.

It has become recognized that metabolic syndrome is a cluster of abnormalities including diabetic hallmarks such as insulin resistance, as well as central or visceral obesity and hypertension. In nearly all cases, dysregulation of glucose results in the stimulation of cytokine release and upregulation of extracellular matrix deposition. Additional factors contributing to chronic kidney disease, diabetes, metabolic syndrome, and glomerular nephritis include hyperlipidemia, hypertension, and proteinuria, all of which result in further damage to the kidneys and further stimulate the extracellular matrix deposition. Thus, regardless of the primary cause, insults to the kidneys may result in kidney fibrosis and the concomitant loss of kidney function. (Schena, F. and Gesualdo, L., Pathogenic Mechanisms of Diabetic Nephropathy, J. Am. Soc. Nephrol., 16: S30-33 (2005*);* Whaley-Connell, A., and Sower, J.R., Chronic Kidney Disease and the Cardiometabolic Syndrome, J. Clin. Hypert., 8(8): 546-48 (2006*)*).

### 3. Lung fibrosis

In some embodiments, the disease or condition is or is associated with lung fibrosis. Fibrosis of the lung includes many syndromes and diseases. Exemplary diseases include idiopathic pulmonary fibrosis (IPF), idiopathic interstitial pneumonia, and acute respiratory distress syndrome (ARDS). Lung fibrosis also includes, but is not limited to, cryptogenic fibrosing alveolitis, chronic fibrosing interstitial pneumonia, interstitial lung disease (ILD), and diffuse parenchymal lung disease (DPLD).

The pathogenesis of most lung fibroses, including the aforementioned diseases are not well understood, however all are characterized by an influx of inflammatory cells and a subsequent increase in the synthesis and deposition of collagen-rich extracellular matrix. (Chua et al., Am J. Respir. Cell. Mol. Biol., 33:9-13 (2005*);* Tzortzaki et al., J. Histochem. & Cytochem., 54(6): 693-700 (2006*);* Armstrong et al., Am. J. Respir. Crit. Care Med., 160: 1910-1915 (1999*)*).

IPF is characterized by inflammation, and eventually fibrosis, of lung tissue; although these two symptoms can also be dissociated. The cause of IPF is unknown; it may arise either from an autoimmune disorder or as a result of infection. Symptoms of IPF include dyspnea (*i.e*., shortness of breath) which becomes the major symptom as the disease progresses, and dry cough. Death can result from hypoxemia, right-heart failure, heart attack, lung embolism, stroke or lung infection, all of which can be brought on by the disease.

### 4. Myelofibrosis

In some embodiments, the disease or condition is or is associated with myelofibrosis. Pathogenic processes in primary myelofibrosis involve a primary megakaryocyte-weighted clonal myeloproliferation and a paraneoplastic stromal reaction that includes bone marrow fibrosis, osteosclerosis, angiogenesis, and extramedullary hematopoiesis. The bone marrow reaction includes excess deposition of extracellular matrix proteins such as fibrillar collagen, hypocellularity, activation and recruitment of bone marrow fibroblasts, excessive cytokine and growth factor production, and other changes that result in a reduction of hematopoietic capacity. Secondary myelofibrosis can result from polycythemia rubra vera or essential thrombocytosis.

### B. CANCER

In some embodiments, the disease or condition is or is associated with a cancer or tumor. Thus, in some embodiments, the subject is an oncology patient. Such diseases and conditions and cancers include carcinomas, sarcomas, benign tumors, primary tumors, tumor metastases ,solid tumors, non-solid tumors, blood tumors, leukemias and lymphomas, and primary and metastatic tumors.

Carcinomas include, but are not limited to, esophageal carcinoma, hepatocellular carcinoma, basal cell carcinoma (a form of skin cancer), squamous cell carcinoma (various tissues), bladder carcinoma, including transitional cell carcinoma (a malignant neoplasm of the bladder), bronchogenic carcinoma, colon carcinoma, colorectal carcinoma, gastric carcinoma, lung carcinoma, including small cell carcinoma and non-small cell carcinoma of the lung, adrenocortical carcinoma, thyroid carcinoma, pancreatic carcinoma, breast carcinoma, ovarian carcinoma, prostate carcinoma, adenocarcinoma, sweat gland carcinoma, sebaceous gland carcinoma, papillary carcinoma, papillary adenocarcinoma, cystadenocarcinoma, medullary carcinoma, renal cell carcinoma, ductal carcinoma in situ or bile duct carcinoma, choriocarcinoma, seminoma, embryonal carcinoma, Wilm's tumor, cervical carcinoma, uterine carcinoma, testicular carcinoma, osteogenic carcinoma, epithelial carcinoma, and nasopharyngeal carcinoma, etc.

Sarcomas include, but are not limited to, fibrosarcoma, myxosarcoma, liposarcoma, chondrosarcoma, chordoma, osteogenic sarcoma, osteosarcoma, angiosarcoma, endotheliosarcoma, lymphangiosarcoma, lymphangioendotheliosarcoma, synovioma, mesothelioma, Ewing's sarcoma, leiomyosarcoma, rhabdomyosarcoma, and other soft tissue sarcomas.

Solid tumors include, but are not limited to, glioma, astrocytoma, medulloblastoma, craniopharyngioma, ependymoma, pinealoma, hemangioblastoma, acoustic neuroma, oligodendroglioma, mcnangioma, melanoma, neuroblastoma, and retinoblastoma.

Leukemias include, but are not limited to, a) chronic myeloproliferative syndromes (neoplastic disorders of multipotential hematopoietic stem cells); b) acute myelogenous leukemias (neoplastic transformation of a multipotential hematopoietic stem cell or a hematopoietic cell of restricted lineage potential; c) chronic lymphocytic leukemias (CLL; clonal proliferation of immunologically immature and functionally incompetent small lymphocytes), including B-cell CLL, T-cell CLL prolymphocytic leukemia, and hairy cell leukemia; and d) acute lymphoblastic leukemias (characterized by accumulation of lymphoblasts). Lymphomas include, but are not limited to, B-cell lymphomas (e.g., Burkitt's lymphoma); Hodgkin's lymphoma; and the like.

Benign tumors include, e.g., hemangiomas, hepatocellular adenoma, cavernous hemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas.

Primary and metastatic tumors include, e.g., lung cancer (including, but not limited to, lung adenocarcinoma, squamous cell carcinoma, large cell carcinoma, bronchioloalveolar carcinoma, non-small-cell carcinoma, small cell carcinoma, mesothelioma); breast cancer (including, but not limited to, ductal carcinoma, lobular carcinoma, inflammatory breast cancer, clear cell carcinoma, mucinous carcinoma); colorectal cancer (including, but not limited to, colon cancer, rectal cancer); anal cancer; pancreatic cancer (including, but not limited to, pancreatic adenocarcinoma, islet cell carcinoma, neuroendocrine tumors); prostate cancer; ovarian carcinoma (including, but not limited to, ovarian epithelial carcinoma or surface epithelial-stromal tumor including serous tumor, endometrioid tumor and mucinous cystadenocarcinoma, sex-cord-stromal tumor); liver and bile duct carcinoma (including, but not limited to, hepatocellular carcinoma, cholangiocarcinoma, hemangioma); esophageal carcinoma (including, but not limited to, esophageal adenocarcinoma and squamous cell carcinoma); non-Hodgkin's lymphoma; bladder carcinoma; carcinoma of the uterus (including, but not limited to, endometrial adenocarcinoma, uterine papillary serous carcinoma, uterine clear-cell carcinoma, uterine sarcomas and leiomyosarcomas, mixed mullerian tumors); glioma, glioblastoma, medulloblastoma, and other tumors of the brain; kidney cancers (including, but not limited to, renal cell carcinoma, clear cell carcinoma, Wilm's tumor); cancer of the head and neck (including, but not limited to, squamous cell carcinomas); cancer of the stomach (including, but not limited to, stomach adenocarcinoma, gastrointestinal stromal tumor); multiple myeloma; testicular cancer; germ cell tumor; neuroendocrine tumor; cervical cancer; carcinoids of the gastrointestinal tract, breast, and other organs; and signet ring cell carcinoma.

### V. THERAPEUTIC METHODS

Among the provided methods are methods for treating and/or ameliorating one or more symptoms of a disease or condition, such as a disease or condition as described herein, for example, a fibrotic disease or condition, e.g., liver disease. In general, the methods are carried out by administering an agent that inhibits of and/or that binds to a lysyl oxidase-like 2 (LOXL2). Among the diseases and conditions are fibrotic diseases and conditions and diseases and conditions associated with fibrosis. Such diseases and conditions can include, for example, liver fibrosis (hepatic fibrosis) and fibrotic liver diseases, such as viral hepatitis, such as chronic viral hepatitis, hepatitis B virus (HBV) and hepatitis C virus (HCV), and HIV associated steatohepatitiscirrhosis and associated conditions such as chronic viral hepatitis, lung or pulmonary fibrosis, such as idiopathic pulmonary fibrosis (IPF), kidney fibrosis, cardiac fibrosis, myelofibrosis, and cancers, such as pancreatic adenocarcinoma and colorectal adenocarcinoma. Other examples of liver fibrotic diseases or conditions are NASH (nonalcoholic steatohepatitis), PSC (primary sclerosing cholangitis), cirrhosis, portal hypertension, PBC (primary biliary cirrhosis), autoimmune hepatitis, alcoholic cirrhosis, alpha 1 antitrypsin deficiency disease, hereditary hemochromatosis, Wilson's disease, non-alcoholic fatty liver disease (NAFLD), an dalcoholic steatohepatitis (ASH). In certain embodiments, the disease or condition is non-alcoholic steatohepatitis (NASH), primary biliary cirrhosis (PBC), or primary sclerosing cholangitis (PSC). In some embodiments, the disease or condition is an active fibrotic disease, such as METAVIR F1 or F2 liver fibrosis, early stage liver fibrosis (e.g., METAVIR F1 or F2), and/or an advanced stage fibrotic disease, such as METAVIR F4 liver fibrosis.

### A. AGENTS THAT BIND TO, DETECT, AND/OR INHIBIT LOXL2

In general, the methods are carried out using agents that are inhibitors of and/or bind to and/or detect LOXL2, such as a LOXL2 polypeptide as described herein. In some aspects, the LOXL2 is a full-length or processed form (resulting from protyolitic processing or cleavage), a proenzyme form or a mature form, a secreted form, and/or an active form. Other lysyl oxidases include LOXL1, LOXL3, and LOXL4. In some embodiments, the agent inhibits and/or binds to both active LOX and LOXL2. In other embodiments, the agent is specific for one form of LOX or LOXL, such as an inhibitor or binder of LOXL2 which does not substantially bind to or inhibit another lysyl oxidase or lysyl oxidase-like protein.

The agents include, for example, antibodies that specifically bind to LOXL2, including antibody fragments, small molecule inhibitors, siRNA, shRNA, and antisense polynucleotides. In some aspects, the agents, e.g., antibodies, are noncompetitive inhibitors, e.g., of LOXL2. In some instances, the agent that inhibits enzymatic activity of a LOXL2 polypeptide. Agents that inhibit LOXL2 enzymatic activity include an allosteric inhibitor of LOXL2 enzymatic activity.

Thus, in some embodiments, the LOXL2 inhibitors and/or binding agents are antibodies that specifically bind a LOXL2 protein, such as any of those antibodies described herein, including antibody fragments and monoclonal antibodies.

If needed, for treatments, methods can further include additional therapies. For example, in some embodiments, the methods further include treating the subject with another therapeutic agent such as an anti-viral agent, e.g., an agent suitable for treating an HCV or HBV infection or other hepatitis virus infection. For example, an HCV infection can be treated with an interferon-alpha (IFN-α), viramidine, ribavirin, levovirin, an HCV NS3 inhibitor, an HCV NS5B inhibitor, or combinations of one or more of the foregoing.

### B. DOSAGE AND ADMINISTRATION

In general, the agents are administered in a therapeutically effective amount, e.g., in an amount to effect treatment of a particular disease or condition, such as to effect a reduction or elimination of a symptom thereof, and/or an amount effective to inhibit LOXL2, e.g., LOXL2 activity. In some examples, the agents are administered at an amount effective to increase or prolong survival, compared to the absence of the treatment, reduce or prevent an increase in bridging fibrosis, reduce or prevent an increase in alpha smooth muscle actin (αSMA) levels, and/or reduce or prevent an increase in stellate cell activation, reduce fibrosis, and/or reduce inflammation and/or necrosis in the diseased or fibrotic tissue. In some examples, the agents are administered at an amount effective to reduce or prevent increase in alanine aminotransferase (ALT) or aspartate aminotransferase (AST), and/or for example, to reduce ALT/AST ratio or AST/ALT ratio, for example, to reduce such parameters to less than the upper limit or normal (ULN), or to less than 2x, 5x, or 10x the upper limit of normal (ULN).

The selected dosage regimen will depend upon a variety of factors, which may include the activity of the antibody, the route of administration, the time of administration, the rate of excretion of the particular compound being employed, the duration of the treatment, other drugs, compounds and/or materials used in combination with the particular composition employed, the age, sex, weight, condition, general health and prior medical history of the patient being treated, and like factors well known in the medical arts.

A clinician having ordinary skill in the art can readily determine and prescribe the effective amount (ED50) of the pharmaceutical composition required. For example, the physician or veterinarian can start doses of the compounds of the invention employed in the pharmaceutical composition at levels lower than that required in order to achieve the desired therapeutic effect and gradually increase the dosage until the desired effect is achieved.

In some aspects, the agent, e.g., antibody is administered at a dose between 1 ng/kg body weight and 20 mg/kg body weight per dose, e.g. between 0.1 mg/kg body weight to 10 mg/kg body weight, e.g. between 0.5 mg/kg body weight to 5 mg/kg body weight, and/or 1 µg to 10 mg per kilogram of body weight per minute. In some examples, the dosage is at or about or at least at or about, or up to at or about 10 or 20 mg/kg, such as at or about or at least at or about, or up to at or about 10 or 20 mg/kg IV every other week. In some examples, the dosage is at or about 200 mg, e.g., at or about 200 mg IV, at or about 700 mg, e.g., at or about 700 mg IV (every other week), between at or about 200 and at or about 700 or 1000 mg, e.g., IV, every other week. In other examples, the dosage is at or about 75 mg, 125 mg, or at or about between 75 and 125 mg, e.g., at or about 75 mg, 125 mg subcutaneously (SC). In another examples, the dosage is at or about 1, 5, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, or 500 mg that is administered by IV or SC. The agent is administered every one, two, three, four, five, or six weeks by either IV or SC. Also, the agent is administered by either IV or SC over a period of one, two, three, four, five, six, seven, eight, nine, ten, eleven, twelve, fifteen, eighteen, twenty, twenty-four, thirty, or thirty-six months.

In some cases, the methods of treatment include parenteral administration, e.g., intravenous, intra-arterial, intramuscular, or subcutaneous administration, or oral administration of the antibody or composition containing the same. The antibodies may also be administered locally.

### C. COMPOSITIONS, DEVICES, SYSTEMS, AND KITS

Also provided herein are compositions, devices, systems, and kits for delivering inhibitors of LOX/LOXL locally to the site of fibrosis. Medical devices such as catheters and stents may be used to deliver locally, thus substantially reducing the risk of toxicity or other side effects associated with systemic delivery of such inhibitors of LOX/LOXL.

For example, provided are pharmaceutical compositions for use in connection with the methods, such as those containing any of the agents, e.g., antibodies, described herein. Compositions can be suitable for administration locally or systemically by any suitable route.

### D. DETECTION AND MONITORING METHODS

In some aspects, the therapeutic methods include diagnostic, detection, and/or prognostic steps, such as for the detection of LOXL2 gene products (e.g., mRNA, protein) and/or products of fibrosis-related genes. Such methods may be performed, for example, in conjunction with the provided therapeutic methods, for example to monitor effects of treatment.

For example, in some embodiments, the treatment methods include steps for monitoring treatment, including for monitoring efficacy or activity and/or detecting or measuring the presence, absence, levels, and/or expression of markers, including LOXL2 and/or other markers of the disease or condition of interest. In some examples, the methods include assessing intrahepatic LOXL2 expression, e.g., mRNA or protein expression, and/or posttreatment vs. pretreatment levels of other fibrosis-related genes are assessed.

In some aspects, the methods determine a likelihood that an individual having the disease or condition will exhibit a beneficial clinical response to a treatment, such as treatment with a LOXL2 inhibitor or binding agent, and/or for assessing efficacy of the treatment. Such methods can include determining a circulating level of lysyl oxidase like-2 (LOXL2), for example, in a liquid sample obtained from the individual. In one aspect, a circulating level of LOXL2 that is greater than a normal control level indicates that the individual has an increased likelihood of exhibiting a beneficial clinical response to a treatment for the disease or condition. In some examples, reports are generated based on the determined likelihood. In some examples, the methods further include treating or altering or discontinuing treatment of the individual for the disease or condition. In some embodiments, the methods provide predictive information regarding the disease or condition, such as the likelihood of a particular endpoint, outcome, or event, such as disease outcome or responsiveness to treatment.

In some examples, the method include assessing the individual for markers of liver function, such as synthesis of proteins such as serum proteins (e.g., albumin, clotting factors, alkaline phosphatase, aminotransferases (e.g., alanine transaminase, aspartate transaminase), 5'-nucleosidase, γ-glutaminy|transpeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics. In one example, levels of serum alanine aminotransferase (ALT) are measured, using standard assays. In some examples, an ALT level of less than about 45 international units, less than about 50, 55, 60, 61, 62, 63, 64, or 65, is considered normal. Elevated ALT levels can indicate compromised liver function. Quantitative tests of functional liver reserve can also be used to assess liver function, where such test include, e.g., indocyanine green clearance (ICG), galactose elimination capacity (GEC), aminopyrine breath test (ABT), antipyrine clearance, monoethylglycine-xylidide (MEG-X) clearance, and caffeine clearance.

### V. DETECTION. DIAGNOSTIC. PROGNOSTIC. AND PREDICTIVE METHODS

Also among the provided methods are detection, diagnostic, prognostic, and predictive methods. In some embodiments, such methods include detection and/or quantification of LOXL2, e.g., LOXL2 polypeptides, in an individual (e.g., a subject as described herein) and/or in a sample, such as a sample obtained from such an individual. In some aspects, the LOXL2 is circulating LOXL2. Thus, provided in some aspects are assays for detection and/or quantification of circulating LOXL2.

In some embodiments, the LOXL2 is detected in a sample, e.g., a liquid sample, obtained from an individual being tested. The liquid sample can be blood or a blood fraction such as plasma or serum, or other liquid sample.

In some embodiments, the provided methods and assays are useful for non-invasive surrogate measurement of the degree of liver fibrosis, such as in patients with chronic HCV infection or HBV infection.

### A. LOXL2 POLYPEPTIDES

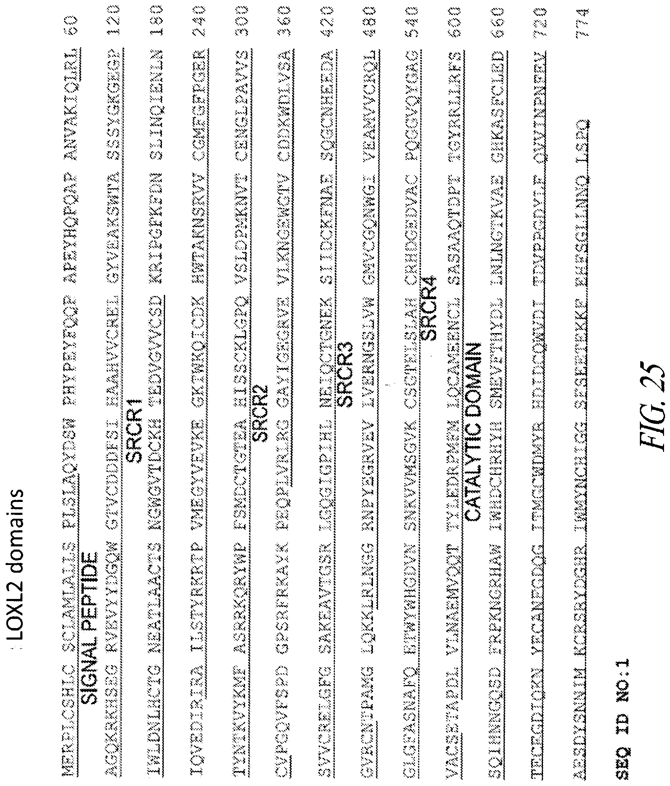

A "LOXL2 polypeptide" refers to a polypeptide comprising an amino acid sequence having at least about 90%, at least about 95%, at least about 97%, at least about 98%, at least about 99%, or 100%, amino acid sequence identity to a contiguous stretch of from about 100 amino acids (aa) to about 200 aa, from about 200 aa to about 300 aa, from about 300 aa to about 400 aa, from about 400 aa to about 500 aa, from about 500 aa to about 600 aa, from about 600 aa to about 700 aa, or from about 700 aa to 774 aa, of the amino acid sequence depicted in Figure 25. "LOXL2" also refers to the human LOXL2 amino acid sequence depicted in Figure 25, and naturally-occurring variants (polymorphisms) thereof.

Figure 25 depicts an amino acid sequence of human LOXL2, showing the four scavenger receptor cysteine rich (SRCR) domains. A LOXL2 polypeptide can be a full-length polypeptide or a mature (cleavage form; processed form) LOXL2 polypeptide. The predicted signal cleavage is between Ala25-Gln26. Cleavage of the signal peptide from the prepropeptide results in a LOXL2 propeptide. LOXL2 propeptide is cleaved between SRCR2 and SRCR3 (e.g., between amino acids 301 and 326 of the sequence depicted in Figure 25), leaving a LOXL2 polypeptide comprising SRCR3, SRCR4, and the lysyl oxidase (catalytic) domain.

A LOXL2 polypeptide may be enzymatically active. For example, a LOXL2 polypeptide can catalyze oxidative deamination of ε-amino groups of lysine and hydroxylysine residues, resulting in conversion of peptidyl lysine to peptidyi-α-aminoadipic-δ-semialdehyde (allysine) and the release of stoichiometric quantities of ammonia and hydrogen peroxide. This reaction most often occurs extracellularly, e.g., on lysine residues in collagen and elastin.

In some cases, the LOXL2 polypeptide that is detected using a subject LOXL2 assay is a full-length LOXL2 polypeptide without the signal sequence, e.g., including SRCR1-2, SRCR3-4, and the catalytic domain. In some instances, the LOXL2 polypeptide that is detected using a subject LOXL2 assay is a mature LOXL2 polypeptide (i.e., without the signal sequence and without SRCR1-2), including only the SRCR3-4 domain and the catalytic domain. Alternatively, or in addition to, detecting the mature LOXL2 polypeptide (SRCR3-4 and catalytic domains; without the signal sequence and SRCR1 -2 domains), a subject LOXL2 assay can detect an N-terminal LOXL2 fragment, which N-terminal LOXL2 fragment includes the SRCR1-2 domains and not the SRCR3-4 or catalytic domains.

### B. SAMPLES

In some embodiments, the methods are performed on samples, such as biological samples, such as liquid biological samples. Such samples include, but are not limited to, whole blood; blood fractions (also referred to as "blood products"), where suitable blood fractions include, but are not limited to, serum and plasma; saliva; urine; bronchoalveolar lavage; cerebrospinal fluid; sputum; and the like. The biological sample can be fresh blood or stored blood (e.g. in a blood bank) or blood fractions. The biological sample can be a liquid sample expressly obtained for an assay of the present disclosure or a liquid sample obtained for another purpose which can be subsampled for an assay of the present disclosure.

As one example, the biological sample can be whole blood. Whole blood can be obtained from the subject using standard clinical procedures. In another embodiment, the biological sample is plasma. Plasma can be obtained from whole blood samples by centrifugation of anti-coagulated blood. Such process provides a buffy coat of white cell components and a supernatant of the plasma. In another embodiment, the biological sample is serum.

The sample can be pretreated as necessary by dilution in an appropriate buffer solution, heparinized, concentrated if desired, or fractionated by any number of methods including but not limited to ultracentrifugation, fractionation by fast protein liquid chromatography (FPLC), or precipitation. The sample can be fractionated, e.g., by an immunoaffinity method, to remove one or more non-LOXL2 proteins or other non-LOXL2 components from the sample; e.g., an anti-albumin antibody can be used to remove albumin from the sample. Any of a number of standard aqueous buffer solutions, employing one of a variety of buffers, such as phosphate, Tris, or the like, at physiological pH can be used.

### C. AGENTS

The methods generally are performed using agents that bind to and/or detect LOXL2, such as an anti-LOXL2 antibody, including any of the antibodies described herein. In some embodiments, the method uses an antibody specific for LOXL2 to immobilize and/or detect LOXL2 in a liquid sample. The antibody can be any antibody described herein.

### D. ASSAY FORMATS

In some aspects, a subject assay for detecting circulating LOXL2 in an individual involves: a) contacting a liquid sample obtained from the individual with an antibody specific for LOXL2; and b) detecting binding of the antibody with LOXL2 present in the liquid sample. Suitable assay methods include an enzyme-linked immunosorbent assay (ELISA), a radioimmunoassay (RIA), an immunoprecipitation assay, a lateral or axial flow assay, mass spectrometry, and the like.

In some embodiments, a subject assay method can detect LOXL2 in a liquid sample to 175 pg/ml or less, e.g., a subject assay method can detect LOXL2 in a liquid sample to from about 150 pg/ml to about 175 pg/ml, to from about 125 pg/ml to about 150 pg/ml, to from about 100 pg/ml to about 125 pg/ml, to from about 75 pg/ml to about 100 pg/ml, to from about 50 pg/ml to about 75 pg/ml, or to from about 40 pg/ml to about 50 pg/ml. For example, a subject assay method can detect LOXL2 in a liquid sample when the LOXL2 is present in the liquid sample in a concentration of less than 10 ng/ml, e.g., in a concentration of from about 10 ng/ml to about 5 ng/ml, from about 5 ng/ml to about 1 ng/ml, from about 1 ng/ml to about 500 pg/ml, from about 500 pg/ml to about 400 pg/ml, from about 400 pg/ml to about 300 pg/ml, from about 300 pg/ml to about 200 pg/ml, from about 200 pg/ml to about 175 pg/ml, from about 200 pg/ml to about 150 pg/ml, from about 150 pg/ml to about 100 pg/ml, from about 100 pg/ml to about 75 pg/ml, from about 75 pg/ml to about 50 pg/ml, or from about 50 pg/ml to about 40 pg/ml. In some cases, a subject assay method detects LOXL2 in a liquid sample when the LOXL2 is present in the liquid sample in a concentration range of from about 175 pg/ml to about 5 ng/ml (or more than 5 ng/ml). In some cases, a subject assay method detects LOXL2 in a liquid sample when the LOXL2 is present in the liquid sample in a concentration range of from about 40 pg/ml to about 5 ng/ml (or more than 5 ng/ml). In some cases, a subject assay method detects LOXL2 in a liquid sample to a detection limit of average background plus 2.5 x SD (standard deviation of the background).

In some cases, a subject assay method involves the use of two LOXL2-specific antibodies. The two LOXL2-specific antibodies can both be monoclonal antibodies; the two LOXL2-specific antibodies can be a polyclonal antibody and a monoclonal antibody; or some other such combination.

For example, a first LOXL2-specific antibody is contacted with a liquid sample, where the first LOXL2-specific antibody forms a complex with LOXL2 present in the liquid sample. The first LOXL2-specific antibody can be immobilized on an insoluble support, such that the first LOXL2-specific antibody/LOXL2 complex is immobilized on the insoluble support. Alternatively, the first LOXL2-specific antibody can be in solution, and the first LOXL2-specific antibody/LOXL2 complex can be insoluble, such that the first LOXL2-specific antibody/LOXL2 complex immunoprecipitates. The first LOXL2-specific antibody/LOXL2 complex can be detected using a second LOXL2-specific antibody. In some cases, the first LOXL2-specific antibody is a polyclonal antibody; and the second LOXL2-specific antibody is a monoclonal antibody.

In some embodiments, a subject assay method involves contacting a liquid sample, obtained from an individual, with an immobilized LOXL2-specific antibody, where the immobilized LOXL2-specific antibody is immobilized on an insoluble support. Any LOXL2 present in the sample will bind to the immobilized LOXL2-specific antibody, forming an immobilized anti-LOXL2/LOXL2 complex. The immobilized anti-LOXL2/LOXL2 complex can be detected using a second (non-immobilized) LOXL2-specific antibody. The second LOXL2-specific antibody can be detectably labeled, or can be detected using a detectably labeled secondary antibody.

Thus, in some embodiments, a subject method of detecting circulating LOXL2 in an individual involves: a) contacting a liquid sample obtained from the individual with a first antibody specific for LOXL2, such that the first antibody and the LOXL2 form a complex; b) contacting the LOXL2-first antibody complex with a second antibody specific for LOXL2; and c) detecting binding of the second antibody to the LOXL2-first antibody complex.

The insoluble support can be one or more wells of a multi-well plate, a test strip, a dipstick format, and the like. In any of the above-described assay formats, one or more washing steps can be carried out to remove unbound components.

An assay method of the present disclosure can detect a pathological level of circulating LOXL2 in an individual. For example, a subject assay method can involve: a) contacting a liquid sample obtained from an individual with an antibody specific for LOXL2; b) detecting binding of the antibody with LOXL2 present in the liquid sample; and c) comparing the detected level with a normal control value. A detected level that is higher than a normal control value is indicative of pathology (e.g., cancer or fibrosis).

### E. CONTROL VALUES

In some embodiments, a level or levels of LOXL2 in a sample obtained from a test subject is compared to a normal control value(s) or range of normal control values. The control value can be based on levels of LOXL2 in comparable samples (e.g., blood, plasma, or serum sample, or other liquid biological sample) obtained from a control population, e.g., the general population or a select population of human subjects. For example, the select population may be comprised of apparently healthy subjects, e.g., individuals who have not previously had any signs or symptoms of fibrosis or cancer. Apparently healthy individuals also generally do not otherwise exhibit symptoms of disease. In other words, such individuals, if examined by a medical professional, would be characterized as healthy and free of symptoms of disease.

The control value can take a variety of forms. The control value can be a single cutoff value, such as a median or mean. A normal control value can be a normal control range.

In some cases, the control, normal value is below the detection limit of a subject assay method, e.g., a normal value can be less than about 175 pg/ml, less than about 150 pg/ml, less than about 100 pg/ml, less than about 75 pg/ml, less than about 50 pg/ml, or less than about 40 pg/ml.

### F. TEST SUBJECTS

As noted above, in some embodiments, a sample, e.g., a liquid sample, obtained from an individual (e.g., subject) is tested using a subject LOXL2 assay. Individual, e.g., subjects, suitable for testing with the methods include any of the subjects described herein, such as those who have not yet been diagnosed as having a disease or condition, but who present with symptoms and/or complaints to a physician (e.g., individuals with an undiagnosed disorder or disease); individuals who have been diagnosed with a disease or condition (e.g., cancer, fibrosis, hepatitis C virus (HCV) infection, such as chronic HCV, or a hepatitis B virus (HBV) infection, such as chronic HBV (CHB), or other disease or condition described herein); individuals suspected of having the disease or condition but who have not yet been diagnosed as having it; individuals who are apparently healthy and who are undergoing routine screening, and individuals who are undergoing treatment for the disease or condition.

In some aspects, the subjects for testing using a subject LOXL2 assay include individuals who have been diagnosed as having cancer include individuals having a benign tumor, individuals having a primary tumor, individuals having tumor metastasis, and individuals having a non-solid tumor type of cancer. Individuals who are suitable for testing using a subject LOXL2 assay include individuals who have a cancer, but who have not yet been diagnosed as having cancer. Thus, individuals who are suitable for testing using a subject LOXL2 assay include individuals having a wide variety of cancers, including carcinomas, sarcomas, leukemias, and lymphomas.

In some cases, an oncology patient is one who is currently undergoing treatment for the cancer. In some instances, the treatment comprises administration of an agent that inhibits enzymatic activity of a LOXL2 polypeptide. Agents that inhibit LOXL2 enzymatic activity include an allosteric inhibitor of LOXL2 enzymatic activity. In some cases, the allosteric inhibitor is an anti-LOXL2 monoclonal antibody, e.g., an anti-LOXL2 monoclonal antibody that binds an epitope within an "SRCR3-4" domain of LOXL2. Non-limiting examples of a monoclonal antibody that inhibits LOXL2 enzymatic activity, and that binds an epitope within an SRCR3-4 domain, are AB0023 and AB0024; see, e.g., US 2009/0053224.

In some aspects, individuals who are suitable for testing using a subject assay method include individuals in whom an epithelial-to-mesenchymal transition (EMT) of epithelial cells has taken place. Individuals who are suitable for testing using a subject assay method include individuals in whom desmoplasia and fibroblast activation (which are considered factors in generating a pathologic microenvironment of tumors and fibrotic disease) have occurred. Such individuals may have precancerous cells and/or be at an early stage of cancer development.

In some aspects, individuals who are suitable for testing using a subject LOXL2 assay method include individuals who have been diagnosed as having fibrosis (a fibrotic disease), e.g., liver fibrosis, kidney fibrosis, pulmonary fibrosis, myelofibrosis, cardiac fibrosis, or other type of fibrosis. Individuals who are suitable for testing using a subject LOXL2 assay method include individuals who have a fibrotic disease (e.g., liver fibrosis, kidney fibrosis, pulmonary fibrosis, myelofibrosis, cardiac fibrosis, or other type of fibrosis), but who have not yet been diagnosed as having the fibrotic disease.

In some cases, a suitable test subject has an advanced form of fibrosis, but might still be suitable for treatment with a treatment regimen for fibrosis. For example, a suitable test subject includes a subject with active (not end-stage) fibrosis. In some cases, a suitable test subject is one who has fibrosis, and who might be anticipated to experience rapid disease progression.

In some cases, an individual who is to be tested using a subject LOXL2 assay is one who is currently undergoing treatment for a fibrotic disease. In some instances, the treatment comprises administration of an agent that inhibits enzymatic activity of a LOXL2 polypeptide. Agents that inhibit LOXL2 enzymatic activity include an allosteric inhibitor of LOXL2 enzymatic activity. In some cases, the allosteric inhibitor is an anti-LOXL2 monoclonal antibody, e.g., an anti-LOXL2 monoclonal antibody that binds an epitope within an "SRCR3-4" domain of LOXL2. Non-limiting examples of a monoclonal antibody that inhibits LOXL2 enzymatic activity, and that binds an epitope within an SRCR3-4 domain, are AB0023 and AB0024; see, e.g., US 2009/0053224.

In some cases, an individual who is to be tested using a subject LOXL2 assay is one who is currently undergoing treatment for IPF. In other cases, an individual who is to be tested using a subject LOXL1 assay is one who is currently undergoing treatment for liver fibrosis. In some instances, the treatment comprises administration of an agent that inhibits enzymatic activity of a LOXL2 polypeptide. Agents that inhibit LOXL2 enzymatic activity include an allosteric inhibitor of LOXL2 enzymatic activity. In some cases, the allosteric inhibitor is an anti-LOXL2 monoclonal antibody, e.g., an anti-LOXL2 monoclonal antibody that binds an epitope within an "SRCR3-4" domain of LOXL2. Non-limiting examples of a monoclonal antibody that inhibits LOXL2 enzymatic activity, and that binds an epitope within an SRCR3-4 domain, are AB0023 and AB0024; see, e.g., US 2009/0053224.

In some cases, an individual who is to be tested using a subject LOXL2 assay is one who is currently undergoing treatment for a fibrotic disease or for a cancer. In some instances, the treatment comprises administration of an agent that inhibits enzymatic activity of a LOXL2 polypeptide. Agents that inhibit LOXL2 enzymatic activity include an allosteric inhibitor of LOXL2 enzymatic activity. In some cases, the allosteric inhibitor is an anti-LOXL2 monoclonal antibody, e.g., an anti-LOXL2 monoclonal antibody that binds an epitope within an SRCR3-4 domain of LOXL2. Non-limiting examples of a monoclonal antibody that inhibits LOXL2 enzymatic activity, and that binds an epitope within an SRCR3-4 domain, are AB0023 and AB0024; see, e.g., US 2009/0053224.

### G. DIAGNOSTIC METHODS

Among the provided methods are various diagnostic methods for diseases and conditions associated with LOXL2, including diseases and conditions associated with or characterized by elevated levels of LOXL2, such as elevated circulated LOXL2. For example, provided are methods for determining whether an individual has a disease characterized by elevated circulating LOXL2. Also provided are methods for assessing the activity or severity of such a disease or condition. The diagnostic methods generally involve detecting a level of circulating LOXL2 in the individual, using a subject LOXL2 assay method, as described above. Diseases characterized by elevated circulating LOXL2 include cancer and fibrosis.

The level of LOXL2 in a given sample may be expressed in terms of concentration, by weight, or other readout of a detection assay as described herein. In one aspect, a level of circulating LOXL2 that is greater than a normal control level or other reference level indicates that the individual has a disease characterized by elevated circulating LOXL2. For example, a level of circulating LOXL2 that is at least 10%, at least 15%, at least 20%, at least 25%, at least 30%, at least 40%, at least 50%, or more than 50%, higher than a normal control or other reference level, can indicate that the individual has a disease characterized by elevated circulating LOXL2. As another example, a level of circulating LOXL2 that is greater than about 40 pg/ml, greater than about 50 pg/ml, greater than about 75 pg/ml, greater than about 100 pg/ml, greater than about 150 pg/ml, greater than about 175 pg/ml, greater than about 200 pg/ml, greater than about 250 pg/ml, greater than about 300 pg/ml, greater than about 350 pg/ml, greater than about 400 pg/ml, greater than about 450 pg/ml, greater than about 500 pg/ml, greater than about 550 pg/mL, greater than about 600 pg/mL, greater than about 650 pg/mL, greater than about 700 pg/ml, greater than about 750 pg/mL, or greater than about 800 pg/mL, or greater than between at or about 700 pg/mL and at or about 800 pg/mL, can indicate that the individual has a disease characterized by elevated circulating LOXL2, and/or give prognostic or predictive information about the disease or condition, such as by indicating active disease or a particular activity level. Thus, in some embodiments, the methods include a step of determining whether or that a circulating level of LOXL2 is at or above such an amount, such as that it is greater than at or about 40 pg/ml, greater than at or about 50 pg/ml, greater than about 75 pg/ml, greater than at or about 100 pg/ml, greater than at or about 150 pg/ml, greater than at or about 175 pg/ml, greater than at or about 200 pg/ml, greater than at or about 250 pg/ml, greater than at or about 300 pg/ml, greater than at or about 350 pg/ml, greater than at or about 400 pg/ml, greater than at or about 450 pg/ml, greater than at or about 500 pg/ml, greater than at or about 550 pg/mL, greater than at or about 600 pg/mL, greater than at or about 650 pg/mL, greater than at or about 700 pg/ml, greater than at or about 750 pg/mL, greater than at or about 800 pg/mL, or greater than between at or about 700 pg/mL and at or about 800 pg/mL. In other embodiments, the method include a step of determining whether or that a circulating level of LOXL2 is at, above, or below a level of about 100, 200, 300, 400, 500, 600, 700, 800, 900, 1000, 1100, 1200, 1300, 1400,1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, or 4000 pg/mL. In some cases, the level indicates active fibrogenesis, fibrosis stage, progression or regression of fibrosis, progression or regression of cirrhosis, compensate or decompensate stage in the subject. As used herein, the terms "normal control level," and "reference level," in the context of LOXL2, refer to the level of LOXL2 to which the LOXL2 level in a sample, e.g., a test sample, is compared. In one embodiment, the circulating level of LOXL2 at or above 3000 pg/mL indicates that the subject is less likely to have cirrhosis regression. In other embodiment, the circulating level of LOX2 at or below 1500 pg/mL is more likely to have cirrhosis regression.

In one example, the normal control or reference level is a level generally observed in a sample from a healthy individual, such as an individual not having the subject disease or condition, e.g., LOXL2-associated disease or condition. In another example, it is a level observed in an individual having a LOXL2-associated disease or condition, such as an individual with less active disease, a relatively better prognosis, or more favorable chances associated with a particular outcome, endpoint, or event, such as survival or responsivness to treatment. For example, the reference or normal control level may be a level observed at a particular time point, such as a baseline level, in a sample from an individual that ultimately showed a favorable outcome, endpoint, or event. In another example, the normal control or reference level is a level observed in a sample taken from the same individual, at a different time point compared to the sample being assayed, for example, a baseline level, prior to treatment, or a level earlier in disease progression or before disease was detected. In another example, the normal or reference level is a standard level, such as a level in a sample prepared to have a pre-defined concentration of LOXL2 or simply a pre-defined level. As used herein, "baseline" refers to an amount, level, or measurement of a particular variable at a point in time that is prior to a particular event or period, such as a point in time prior to treatment or prior to the commencement of a study monitoring disease progression. Thus, in one aspect, the reference or normal control level of LOXL2 is a baseline level, such as a baseline level from the same individual or from another individual.

### 1. Control values

Levels of LOXL2 in a liquid sample obtained from a test subject can be compared to a normal control value(s) or range of normal control values. The control value can be based on levels of LOXL2 in comparable samples (e.g., blood, plasma, or serum sample, or other liquid biological sample) obtained from a control population, e.g., the general population or a select population of human subjects. For example, the select population may be comprised of apparently healthy subjects, e.g., individuals who have not previously had any signs or symptoms of fibrosis or cancer. Apparently healthy individuals also generally do not otherwise exhibit symptoms of disease. In other words, such individuals, if examined by a medical professional, would be characterized as healthy and free of symptoms of disease. Alternatively, the assessed values may be compared to other reference values, such as an average, mean, or median value or values observed for a population of subjects having a particular disease or condition. For example, such a reference value may be used in comparison to levels assessed for particular individuals who then are determined, for example, to have more active disease compared to the overall patient cohort from whom the reference value was obtained.

The control value can take a variety of forms. The control value can be a single cut-off value, such as a median or mean. A normal control value can be a normal control range.

### 2. Individuals to be tested

Test subjects include those listed above. Individuals who are suitable for testing using a subject assay include, but are not limited to, individuals who have not yet been diagnosed as having a disease, but who present with symptoms and/or complaints to a physician (e.g., individuals with an undiagnosed disorder or disease); individuals who have been diagnosed with cancer; individuals suspected of having a cancer but who have not yet been diagnosed as having cancer; individuals who are apparently healthy and who are undergoing routine screening; individuals who have been diagnosed as having fibrosis; individuals suspected of having fibrosis but who have not yet been diagnosed as having fibrosis; individuals who have been diagnosed as having a hepatitis C virus (HCV) or hepatitis B virus (HBV) infection (and optionally also diagnosed as having HCV infection- or HBV infection-associated liver damage); and individuals who are undergoing treatment for a cancer or a fibrotic disease.

In some cases, the individual to be tested is an individual with an undiagnosed disorder or disease, e.g., an individual who presents with symptoms and/or complaints. A subject diagnostic method can be used to determine whether such an individual might have a fibrotic disease or a cancer. A subject diagnostic method can be part of differential diagnosis; and in some cases can be used in conjunction with one or more diagnostic tests, e.g., to confirm or to rule out a diagnosis.

### 3. Generating a report

A subject diagnostic method can include generating a report that provides an indication as to whether an individual is likely to have a fibrotic disease or a cancer. Such a report can include information such as a recommendation regarding further evaluation; a recommendation regarding therapeutic drug treatment; and the like.

A subject report can further include one or more of: 1) service provider information; 2) patient data; 3) data regarding the level of LOXL2; 4) follow-up evaluation recommendations; 5) therapeutic drug treatment; and 6) other features.

### 4. Further evaluation

Based on detection of a level of LOXL2, and/or based on a report (as described above), a physician or other qualified medical personnel can determine whether further evaluation of the test subject (the patient) is required. Further evaluation can include, e.g., lung function tests (e.g., where pulmonary fibrosis is suspected); liver function tests (e.g., where liver fibrosis is suspected); and various tests for cancer, which tests may vary, depending on the type of cancer suspected.

As one example, where an individual is suspected of having a cancer, any of a variety of tests for a cancer can be performed, where such tests include, e.g., histochemical analysis of a tissue biopsy for the presence of cancerous cells; tests for the presence of a tumor associated antigen; and the like.

As another example, where an individual is suspected of having a pulmonary fibrotic disorder, the individual can be assessed for symptoms of the pulmonary fibrotic disorder. Symptoms of a pulmonary fibrotic disorder can include, but are not limited to, decreased body weight, increased lung weight, pulmonary fibrosis, pathologic lung architecture (*e.g*., "honeycomb" lung), increased Ashcroft score, increased pulmonary collagen levels, increased number of CD45⁺/collagen⁺ cells, pneumocyte proliferation and expansion and increased leukocyte number in bronchioalveolar lavage (BAL) fluid. Symptoms can also include, for example, increased pulmonary levels of one or more of the following molecules: LOXL2, α-smooth muscle actin (a-SMA), transforming growth factor β-1 (TGFβ-1), stromal derived factor-1 (SDF-1) (*e.g*., SDF-1α), endothelin-1 (ET-1) and phosphorylated SMAD2.

As a further example, where an individual is suspected of having liver fibrosis, the individual can be assessed for markers of liver function. Liver functions include, but are not limited to, synthesis of proteins such as serum proteins (e.g., albumin, clotting factors, alkaline phosphatase, aminotransferases (e.g., alanine transaminase, aspartate transaminase), 5'-nucleosidase, γ-glutaminyltranspeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics; and the like. For example, levels of serum alanine aminotransferase (ALT) are measured, using standard assays. In general, an ALT level of less than about 45 international units is considered normal. Elevated ALT levels can indicate compromised liver function. Quantitative tests of functional liver reserve can also be used to assess liver function, where such test include, e.g., indocyanine green clearance (ICG), galactose elimination capacity (GEC), aminopyrine breath test (ABT), antipyrine clearance, monoethylglycine-xylidide (MEG-X) clearance, and caffeine clearance. The presence of ascites, esophageal varices, encephalopathy, and/or jaundice can indicate decompensated liver disease.

### 5. Therapy

Based on detection of a level of LOXL2, and/or based on a report (as described above), a physician or other qualified medical personnel can determine whether appropriate therapeutic drug treatment is advised, e.g., to treat a fibrotic disease, to treat a cancer, etc.

For example, an individual who has been determined to have an early stage cancer, based on circulating levels of LOXL2 and optionally on further evaluation (e.g., histochemical analysis of a tissue biopsy), can be started on a cancer chemotherapeutic drug regimen and/or can be treated with radiation therapy and/or can undergo surgical removal of the cancer.

Cancer chemotherapeutic agents ("chemotherapeutics") include cytotoxic and cytostatic drugs. Chemotherapeutics may include those which have other effects on cells such as reversal of the transformed state to a differentiated state or those which inhibit cell replication. Examples of known cytotoxic agents are listed, for example, in Goodman et al., "The Pharmacological Basis of Therapeutics," Sixth Edition, A.B. Gilman et al., eds./Macmillan Publishing Co. New York, 1980. These include taxanes, such as paclitaxel and docetaxel; nitrogen such as mechlorethamine, melphalan, uracil mustard and chlorambucil; ethylenimine derivatives, such as thiotepa; alkyl sulfonates, such as busulfan; nitrosoureas, such as lomustine, semustine and streptozocin; triazenes, such as dacarbazine; folic acid analogs, such as methotrexate; pyrimidine analogs, such as fluorouracil, cytarabine and azaribine; purine analogs, such as mercaptopurine and thioguanine; vinca alkaloids, such as vinblastine and vincristine; antibiotics, such as dactinomycin, daunorubicin, doxorubicin, and mitomycin; metal complexes, such as platinum coordination complexes, such as cisplatin; substituted urea, such as hydroxyurea; methyl hydrazine derivatives, such as procarbazine; adrenocortical suppressants, such as mitotane; hormones and antagonists, such as adrenocortisteroids (prednisone), progestins (hydroxyprogesterone caproate, acetate and megestrol acetate), estrogens (diethylstilbestrol and ethinyl estradiol), and androgens (testosterone propionate and fluoxymesterone).

As another example, an individual who has been determined to have IPF, for example, based on circulating levels of LOXL2 and optionally on further evaluation (e.g., lung function tests), can be treated with pharmaceutical treatment for IPF and/or other treatment for IPF. Primary treatment for IPF is pharmaceutical, the most common drugs used for treatment of IPF being corticosteroids (*e.g*., prednisone), penicillamine, and various anti neoplastics (*e.g*., cyclophosphamide, azathiporene, chlorambucil, vincristine and colchicine). Other treatments include oxygen administration and, in extreme cases, lung transplantation.

As a further example, an individual who has been determined to have liver fibrosis, based on circulating levels of LOXL2 and optionally on further evaluation (e.g., liver functions tests; tests for infection with HCV, HBV, etc.), can be treated with, e.g., an anti-viral agent, e.g., an agent suitable for treating an HCV or HBV infection or other hepatitis virus infection. For example, an HCV infection can be treated with an interferon-alpha (IFN-α), viramidine, ribavirin, levovirin, an HCV NS3 inhibitor, an HCV NS5B inhibitor, or combinations of one or more of the foregoing.

### H. METHODS FOR MONITORING EFFICACY OF TREATMENT

The present disclosure provides methods for monitoring efficacy of treatment for a LOXL2-associated disease or condition, such as a disease characterized by elevated circulating LOXL2, the method generally involving determining a circulating LOXL2 level in the individual at a time point, using a subject LOXL2 assay. In one aspect, a level of LOXL2 in the sample that is lower than a level obtained at an earlier time point from the individual indicates efficacy of the treatment. In another aspect, a lower level compared to a control or reference sample indicates treatment efficacy. In another aspect, the level of LOXL2, *e.g*., a high level of LOXL2, indicates that an individual will respond favorably to treatment, such as treatment with a LOXL2-targeting therapy.

For example, a circulating LOXL2 level is determined at a first time point and at a second time point in the individual, where the second time point is later than the first time point. The first time point can be before the start of treatment; and the second time point can be during treatment (e.g., after a treatment regimen has begun). The first time point can be during treatment; and the second time point can be at a later time during treatment. The second time point can be from about 1 hour to about 1 year after the first time point, e.g., the second time point can be from about 1 hour to about 2 hours, from about 2 hours to about 4 hours, from about 4 hours to about 8 hours, from about 8 hours to about 16 hours, from about 16 hours to about 24 hours, from about 24 hours to about 36 hours, from about 36 hours to about 72 hours, from about 72 hours to about 4 days, from about 4 days to about 1 week, from about 1 week to about 2 weeks, from about 2 weeks to about 1 month, from about 1 month to about 3 months, from about 3 months to about 6 months, or from about 6 months to about 1 year, or more than 1 year, after the first time point.

Thus, e.g., in some embodiments, a subject method of determining efficacy of treatment for a disease characterized by elevated circulating LOXL2 comprises: a) determining the circulating level of LOXL2 in an individual at a first time point (by determining the level of LOXL2 in a liquid sample obtained from the individual at the first time point); b) determining the circulating level of LOXL2 in the individual at a second time point (by determining the level of LOXL2 in a liquid sample obtained from the individual at the second time point); and comparing the level of LOXL2 from the first and second time points.

If the circulating LOXL2 level at the second time point is lower than the circulating LOXL2 level at the first time, point, it may be concluded that the treatment for the disease characterized by elevated circulating LOXL2 was effective; in these cases, a recommendation may be made to continue with the treatment regimen. If the circulating LOXL2 level at the second time point is higher than the circulating LOXL2 level at the first time, point, it may be concluded that the treatment for the disease characterized by elevated circulating LOXL2 was not effective; in these cases, a recommendation may be made to discontinue the treatment regimen, to increase the dose of a drug used in the treatment regimen, to increase the frequency of dosing, or to administer an alternative treatment regimen. If the circulating LOXL2 level at the second time point is not significantly different than the circulating LOXL2 level at the first time, point, it may be concluded that the treatment for the disease characterized by elevated circulating LOXL2 was not effective, or that the treatment regimen should be altered; in these cases, a recommendation may be made to discontinue the treatment regimen, to increase the dose of a drug used in the treatment regimen, to increase the frequency of dosing, or to administer an alternative treatment regimen.

In some embodiments, provided are methods for assessing treatments (e.g., efficacy thereof) for and/or other effects on LOXL2-associated diseases and conditions, such as a disease characterized by elevated circulating LOXL2, using animal models. In some aspects, the disease or condition is a cholestatic liver disease, such as PSC or PBC. In some such aspects, the animal model is a *Mdr2*^{*-*/*-*} or bile duct-ligated (BDL) mice. In some embodiments, the methods include administering the treatment to the animal model and assessing efficacy or other outcome of treatment. In one example, the assessing includes measuring LOXL2 levels in a sample from the animal, for example, using a detection method as described herein.

### 1. Test subjects

A subject method for monitoring efficacy of treatment can be used to test any of a variety of individuals, including, e.g., individuals who have been diagnosed with cancer and who are undergoing treatment for; individuals who have been diagnosed as having fibrosis and who are undergoing treatment for the fibrosis; individuals who have been diagnosed as having IPF and who are undergoing treatment thereof; individuals who have been diagnosed as having NASH and who are undergoing treatment thereof; individuals who have been diagnosed as having PBC and who are undergoing treatment thereof; individuals who have been diagnosed as having liver fibrosis and are undergoing treatment thereof; individuals who have been diagnosed as having an HCV or HBV infection and who are undergoing treatment for the HCV or HBV infection; individuals who have been diagnosed as having HCV or HBV infection-associatcd liver damage, and who are undergoing treatment for the HCV or HBV infection and/or the liver damage; and the like. These subjects may be referred to as the subject in need of treatment or the subject in need of diagnosis.

In some cases, an individual who is to be tested using a subject LOXL2 assay is one who is currently undergoing treatment for a cancer. The cancer chemotherapy can be any of a variety of cytotoxic agents. Such cytotoxic agents include taxanes, such as paclitaxel and docetaxel; nitrogen such as mechlorethamine, melphalan, uracil mustard and chlorambucil; ethylenimine derivatives, such as thiotepa; alkyl sulfonates, such as busulfan; nitrosoureas, such as lomustine, semustine and streptozocin; triazenes, such as dacarbazine; folic acid analogs, such as methotrexate; pyrimidine analogs, such as fluorouracil, cytarabine and azaribine; purine analogs, such as mercaptopurine and thioguanine; vinca alkaloids, such as vinblastine and vincristine; antibiotics, such as dactinomycin, daunorubicin, doxorubicin, and mitomycin; metal complexes, such as platinum coordination complexes, such as cisplatin; substituted urea, such as hydroxyurea; methyl hydrazine derivatives, such as procarbazine; adrenocortical suppressants, such as mitotane; hormones and antagonists, such as adrenocortisteroids (prednisone), progestins (hydroxyprogesterone caproate, acetate and megestrol acetate), estrogens (diethylstilbestrol and ethinyl estradiol), and androgens (testosterone propionate and fluoxymesterone).

In some instances, the cancer treatment comprises administration of an agent that inhibits enzymatic activity of a LOXL2 polypeptide. Agents that inhibit LOXL2 enzymatic activity include an allosteric inhibitor of LOXL2 enzymatic activity. In some cases, the allosteric inhibitor is an anti-LOXL2 monoclonal antibody, e.g., an anti-LOXL2 monoclonal antibody that binds an epitope within an "SRCR3-4" domain of LOXL2. Non-limiting examples of a monoclonal antibody that inhibits LOXL2 enzymatic activity, and that binds an epitope within an SRCR3-4 domain, are AB0023 and AB0024; see, e.g., US 2009/0053224.

As another example, an individual undergoing treatment for liver fibrosis, or who is undergoing treatment for a disease that can result in liver fibrosis, is suitable for testing using a subject method. As an example, an individual undergoing treatment for an HCV infection is suitable for testing using a subject method. For example, an HCV infection can be treated with an IFN-α, viramidine, ribavirin, levovirin, an HCV NS3 inhibitor, an HCV NS5B inhibitor, or combinations of one or more of the foregoing.

As another example, an individual undergoing treatment for IPF is suitable for testing using a subject method. Drugs commonly used to treat IPF include, e.g., corticosteroids (*e.g*., prednisone), penicillamine, and various anti neoplastics (*e.g*., cyclophosphamide, azathiporene, chlorambucil, vincristine and colchicine).

### 2. Control values

Levels of LOXL2 in a liquid sample obtained from a test subject can be compared to a normal control value(s) or range of normal control values or other reference values as described herein. The control value can be based on levels of LOXL2 in comparable samples (e.g., blood, plasma, or serum sample, or other liquid biological sample) obtained from a control population, e.g., the general population or a select population of human subjects. For example, the select population may be comprised of apparently healthy subjects, e.g., individuals who have not previously had any signs or symptoms of fibrosis or cancer. Apparently healthy individuals also generally do not otherwise exhibit symptoms of disease. In other words, such individuals, if examined by a medical professional, would be characterized as healthy and free of symptoms of disease.

The control value can take a variety of forms. The control value can be a single cut-off value, such as a median or mean. A normal control value can be a normal control range. In some cases, the control, normal value is below the detection limit of a subject assay method, e.g., less than about 175 pg/ml less than about 150 pg/ml, less than about 125 pg/ml, less than about 100 pg/ml, less than about 75 pg/ml, less than about 50 pg/ml, or less than about 40 pg/ml.

### I. PROGNOSTIC METHODS

Also provided are various prognostic and predictive methods. For example, the present disclosure provides determining the likelihood that an individual having a fibrotic disease will exhibit a beneficial clinical response to a treatment for the fibrotic disease. In another example, the method determines the likelihood or risk of a particular disease output or endpoint or responsiveness to treatment. The method generally involves detecting a circulating level of LOXL2, such as in a liquid sample obtained from the individual, using a subject LOXL2 assay. In one aspect, a level of LOXL2 that is greater than a normal control or other reference level indicates that the individual has an increased likelihood of exhibiting a beneficial clinical response to a treatment for the fibrotic disease. In another aspect, a comparatively low level indicates a relatively lower likelihood or risk of developing a particular disease outcome or endpoint, or other prognostic information. Likewise, comparatively high LOXL2 levels can indicate poorer prognosis, such as increased risk or likelihood of developing a particular disease or condition output or reaching a particular endpoint. Fibrotic diseases include pulmonary fibrosis, liver fibrosis, cardiac fibrosis, and myelofibrosis, as described above. In some cases, e.g., where the circulating LOXL2 levels indicate that the subject is likely to exhibit a beneficial clinical response to a treatment for the fibrotic disease, a subject method further involves treating the individual for the fibrotic disease.

Individuals who are suitable for testing using a subject assay method include individuals who have been diagnosed as having fibrosis, e.g., liver fibrosis, kidney fibrosis, pulmonary fibrosis, myelofibrosis, cardiac fibrosis, or other type of fibrosis. Liver fibrosis includes, but is not limited to cirrhosis, and associated conditions such as chronic viral hepatitis (resulting from, e.g., HCV or HBV infection), NAFLD, ASH, NASH, cholestatic liver disease, primary biliary cirrhosis (PBC), biliary cirrhosis, primary sclerosing cholangitis (PSC), and autoimmune hepatitis. Kidney fibrosis can result from a variety of diseases and insults, where examples of such diseases and insults include chronic kidney disease, metabolic syndrome, vesicoureteral reflux, tubulointerstitial renal fibrosis, diabetes (including diabetic nephropathy), and resultant glomerular nephritis (GN), including, but not limited to, focal segmental glomerulosclerosis and membranous glomerulonephritis, mesangiocapillary GN. Fibrosis of the lung includes many syndromes and diseases, where exemplary diseases include IPF, idiopathic interstitial pneumonia, and ARDS. Lung fibrosis also includes, but is not limited to, cryptogenic fibrosing alveolitis, chronic fibrosing interstitial pneumonia, ILD, and DPLD. In some aspects, the liver disease is compensated liver disease. On other aspects, it is decompensated liver disease, such as liver disease associated with ascites, esophageal varices, encephalopathy, and/or jaundice.

In some cases, a suitable test subject has an advanced form of fibrosis, but might still be suitable for treatment with a treatment regimen for fibrosis. For example, a suitable test subject includes a subject with active (not end-stage) fibrosis. In some cases, a suitable test subject is one who has fibrosis, and who might be anticipated to experience rapid disease progression. As an example, an individual may have an advanced stage, e.g., METAVIR F4, of liver fibrosis; an individual with METAVIR F4 fibrosis and a positive LOXL2 (e.g., greater than normal levels of LOXL2 in liquid sample, as determined using a subject LOXL2 assay) may still be a candidate for treatment for the fibrosis. A METAVIR F4 liver fibrosis patient with a negative LOXL 2 (e.g., normal levels of LOXL2 in liquid sample, as determined using a subject LOXL2 assay) may not be considered a candidate for treatment for the fibrosis. As another example, an individual with elevated LOXL2 (e.g., greater than normal levels of LOXL2 in liquid sample, as determined using a subject LOXL2 assay) who has an early stage of liver fibrosis (e.g., METAVIR F1 or F2) may be considered a candidate for treatment for the fibrosis.

In some aspects, the methods indicate that or the likelihood that the individual has an advanced stage of fibrosis, cirrhosis, poor prognosis, decompensated liver disease, inflammation, necrosis, and/or will respond to treatment.

### 1. Control values

Levels of LOXL2 in a liquid sample obtained from a test subject can be compared to a normal control value(s) or range of normal control values. The control value can be based on levels of LOXL2 in comparable samples (e.g., blood, plasma, or serum sample, or other liquid biological sample) obtained from a control population, e.g., the general population or a select population of human subjects. For example, the select population may be comprised of apparently healthy subjects, e.g., individuals who have not previously had any signs or symptoms of fibrosis. Apparently healthy individuals also generally do not otherwise exhibit symptoms of disease. In other words, such individuals, if examined by a medical professional, would be characterized as healthy and free of symptoms of disease.

The control value can take a variety of forms. The control value can be a single cut-off value, such as a median or mean. A normal control value can be a normal control range. In some cases, the control, normal value is below the detection limit of a subject assay method, e.g., less than about 175 pg/ml less than about 150 pg/ml, less than about 125 pg/ml, less than about 100 pg/ml, less than about 75 pg/ml, less than about 50 pg/ml, or less than about 40 pg/ml.

### 2. Generating a report

The likelihood that a patient will exhibit a beneficial clinical response to treatment for a fibrotic disease is assessed by determining a circulating level of LOXL2. The patient's likelihood of exhibiting a beneficial clinical response to treatment for a fibrotic disease is provided in a report. The report may further include information regarding the patient's likelihood of response. For example, a subject method can further include a step of generating or outputting a report providing the results of a subject response likelihood assessment, which report can be provided in the form of an electronic medium (e.g., an electronic display on a computer monitor), or in the form of a tangible medium (e.g., a report printed on paper or other tangible medium).

A "report," as described herein, is an electronic or tangible document which includes report elements that provide information of interest relating to a subject likelihood assessment and its results. A subject report includes at least a likelihood assessment, e.g., an indication as to the likelihood that a patient having a fibrotic disease will exhibit a beneficial clinical response to a treatment for the fibrotic disease. A subject report can be completely or partially electronically generated. A subject report can further include one or more of: 1) information regarding the testing facility; 2) service provider information; 3) patient data; 4) sample data; 5) an interpretive report, which can include various information including: a) indication; b) test data, e.g., circulating LOXL2 level; and 6) other features.

### 3. Prognostic and predictive IPF methods

In some embodiments, provided are diagnostic, prognostic, and predictive methods for idiopathic pulmonary fibrosis (IPF). As shown in the examples herein, increased expression of LOXL2 is detected in the sera of IPF patients compared with normal control samples; additionally, increased circulating LOXL2 levels indicate an active IPF phenotype and an increased risk of various disease outcomes. Higher LOXL2 expression also is detected in the lung tissue of IPF patients. Accordingly, provided are methods using LOXL2 as a marker of IPF disease, such as a marker of IPF disease activity or of the active IPF phenotype. Thus, in some embodiments of the provided methods, LOXL2 is used as a diagnostic, prognostic, and/or predictive marker for IPF. In one aspect, LOXL2 levels are used to evaluate fibrogenesis and/or various IPF stages, severity, or outcomes, such as the likelihood of particular disease outcomes or responsiveness to treatment.

In another aspect, LOXL2 levels are indicative of active disease or a level of disease activity. In another aspect, LOXL2 levels, typically serum levels, that are higher in comparison to a control or other reference sample indicate a risk of developing a particular disease outcome or developing a particular disease outcome in a particular period of time. In other aspects, LOXL2 levels indicate the likelihood that a patient will respond to a particular treatment or gives information regarding the responsiveness to ongoing treatment, such as treatment with a LOXL2 inhibitor or other treatment. Thus, in some embodiments, the methods further include initiating, discontinuing, or altering a disease treatment approach, based on the prediction or detected LOXL2 levels.

Exemplary disease outcomes that are assessed or predicted using the methods include IPF disease progression (a composite endpoint defined as one of the following: mortality from any cause), poor progression-free survival (PFS), respiratory hospitalization, decrease in lung function, *e.g*., categorical decrease in lung function (which may be defined as either a 10% decrease in forced vital capacity (FVC) with a 5% decrease in the diffusion capacity for carbon monoxide (DL_{CO}) or a 15 % decrease in DL_{CO} with a 5% decrease in FVC), and death.

The methods generally involve obtaining a patient sample and/or determining a LOXL2 level in the sample (for example, using the methods described herein) and performing various statistical analyses based on this and other information. In one example, it is determined whether the patient or a sample has a high or low level of LOXL2, for example, a low or high circulating or serum LOXL2 level. This information can be determined, for example, by dichotomizing LOXL2 levels based on a distribution of determined LOXL2 levels in a given population, such as a collection of samples, designating cutoff points for "low" and "high" levels of LOXL2. For example, a high level of LOXL2 can be deemed a level at least or above a particular concentration in a given sample, such as greater than at or about 800 picograms (pg) LOXL2 per milliliter (mL) of sera. Alternatively, a high LOXL2 serum level may be defined based on a distribution of levels for samples within a population or based on a particular fold change compared to a control or reference sample.

In some aspects, the methods are carried out by determining LOXL2 levels in connection with other measurements, such as markers of disease severity or functional status, e.g., baseline measurements of IPF, such as those reflective of IPF severity, such as percent of predicted forced vital capacity (FVC), percent of predicted carbon monoxide diffusion capacity (DL_{CO}), 6-minute walk distance (6MWD), mean pulmonary artery pressure (mPAP), the lowest resting oxygen saturation (SpO2), the composite physiologic index (CPI), the St. George's Respiratory Questionnaire score (SGRQ), and the Transition Dyspnea Index (TDI) score, responsiveness to treatment, and/or other biomarkers of disease or disease severity. Thus, in some aspects of the predictive models and methods, LOXL2 is a biomarker of IPF disease outcome integrated with measures of disease severity or functional status and/or other biomarkers.

### J. STATISTICAL ANALYSES FOR THE DIAGNOSTIC, PROGNOSTIC AND PREDICTIVE METHODS

In some examples, statistical analyses are carried out based on the LOXL2 level and other determinations. In one example, levels of LOXL2 are evaluated, for example, using standard histograms to evaluate untransformed or log₁₀x transformed levels of LOXL2. Statistical analyses can include determining various values, such as mean, *e.g*., geometric mean, or median values for LOXL2 expression levels and/or baseline variables, for individual samples and/or patients, and calculating standard deviations and fold changes among various samples or conditions, and comparing expression levels and/or other variables using any of a number of well-known tests, such as the student's t-test, which, for example, may be used to compare distribution of baseline variables and LOXL2 expression levels.

In some aspects, Pearson's Correlation (PC) is used to assess linear relationships (correlations) between pairs of values (*e.g*., by calculating PC coefficients), such as between LOXL2 expression levels and other variables, such as baseline IPF variable(s) as described herein. Such analysis may be used to linearly separate distribution in expression patterns, by calculating PC coefficients for individual pairs of variables (plotted on x- and y- axes of individual matrices, as shown in Example 9).

### 1. Predictive modeling

In some embodiments, the predictive methods further comprise further use of statistical analysis and use of predictive models and systems. In some aspects, such models and systems are used to predict disease outcomes, endpoints, responsiveness, and/or events, based on LOXL2 levels and typically other information, such as variables indicative of disease severity and other biomarkers. For example, survival models may be used to examine the relationship between LOXL2 levels and other covariates and one or more events, endpoints, or outcomes, such as disease outcomes, *e.g*., IPF outcome(s) and responsiveness to one or more treatment(s); such models may be used to predict the likelihood that a particular patient will have the event, endpoint, or outcome, or that such outcome will occur within a particular amount of time.

In one such example, Cox proportional hazard modeling, *e.g*., stepwise Cox proportional hazard modeling, is carried out to examine the relationship between LOXL2 levels (and optionally other covariates, such as baseline IPF variables described herein and other variables that may be associated with disease outcomes, such as other disease biomarkers) and outcomes, such as IPF outcomes. Using well-known statistical methods, hazard ratios (HRs) are calculated, representing the relationship between the covariate, *e.g*., LOXL2 level, and the subject outcome, endpoint, or event. Thus, in some aspects, the provided methods include using such models to predict outcomes, endpoints, and/or events, *e.g.*, IPF disease outcomes, in individual patients based on LOXL2 levels and values for other covariates. In one example, the model includes LOXL2 levels (for example, the presence or absence of "high" LOXL2 levels), 6MWD, and/or CPI.

IPF outcomes, events, and endpoints for use in such modeling include endpoints or events indicative of disease progression or severity, such as any endpoint typically specified in IPF clinical trials or treatment regimen, such IPF disease progression, lung function decline, respiratory hospitalization, and death. In some aspects, disease progression represents a composite endpoint defined as one of the following: mortality from any cause, respiratory hospitalization, or a categorical decrease in lung function, defined as either a 10 % decrease in forced vital capacity (FVC) with a 5% decrease in the diffusion capacity for carbon monoxide (DL_{CO}) or a 15 % decrease in DL_{CO} with a 5% decrease in FVC). Lung function endpoints may be determined using pulmonary function tests. In some examples, at least two tests are used, conducted at least 4 weeks apart. Other exemplary endpoints are all cause mortality, transplant free survival, and death. The outcome can be defined as the time that elapses before such an endpoint is reached.

Receiver Operating Characteristic (ROC) Curves may be used to evaluate sensitivity versus specificity of the systems. Area Under the Curve (AUC) is computed using well-known methods.

In some examples of the predictive models, LOXL2 is significantly associated with one or more outcome or event, such as disease progression, for example, at a particular confidence interval (CI) and confidence level, such as a 95 % confidence interval, for example, based on a P-value less than a particular threshold amount, *e.g*., 0.05. The hazard ratio may be used to determine the fold-change in risk of a particular outcome, for a given covariate, such as high LOXL2 levels. In some aspects, a given LOXL2 level is associated, *e.g.*, statistically significantly associated, with at least a 2-fold, 3-fold, 4-fold, 5-fold, 6-fold, or 7-fold risk in developing a particular outcome, such as disease progression, hospitalization, decrease in lung function, or other outcome as described herein. The fold-change in risk, for example, can be expressed in terms of comparison to a normal subject, such as one not having an elevated level of LOXL2 or one having a "low" LOXL2 level. In one example, LOXL2 levels, *e.g*., "high" LOXL2 levels, are statistically significantly associated with the outcome, *e.g*., disease progression, when other covariates are included in the model, such as 6MWD and CPI.

### K. KITS AND ASSAY DEVICES

The present disclosure provides kits and assay devices for carrying out the detection, diagnostic, prognostic, and predictive methods, such as for carrying out a subject assay for circulating LOXL2.

In some embodiments, a subject kit includes: a) a first antibody specific for LOXL2; and b) a second antibody specific for LOXL2. In some cases, the first antibody is a polyclonal LOXL2-specific antibody; and the second antibody is a monoclonal LOXL2-specific antibody. In other cases, the first antibody is a monoclonal LOXL2-specific antibody; and the second antibody is a monoclonal LOXL2-specific antibody. In other cases, the first antibody is a polyclonal LOXL2-specific antibody; and the second antibody is a polyclonal LOXL2-specific antibody. The first and/or the second antibody will in some cases comprise a detectable label. In some cases, neither the first nor the second antibody comprises a detectable label.

The first antibody will in some embodiments be immobilized on an insoluble support. Alternatively, an insoluble support is provided with the kit, and the user will effect immobilization of the first antibody onto the insoluble support. Thus, in some cases, a subject kit includes: a) a first antibody specific for LOXL2; b) a second antibody specific for LOXL2; and c) an insoluble support. The insoluble support can be provided in any of a variety of materials and formats, as described above. For example, in some instances, the insoluble support is a plastic multi-well plate, a test strip, or a dipstick.

As noted above, in some instances, neither the first nor the second antibody comprises a detectable label. In these cases, a third antibody that comprises a detectable label, and that binds to the second antibody, may be provided; such an antibody is generally referred to as a secondary antibody. The detectable label can be, e.g., a chemiluminescent agent, a particulate label, a colorimetric agent, an energy transfer agent, an enzyme, a fluorescent agent, or a radioisotope. Thus, in some embodiments, a subject kit comprises: a) a first antibody specific for LOXL2; b) a second antibody specific for LOXL2; and c) a third antibody, where the third antibody comprises a detectable label, and binds to the second antibody. In some cases, a subject kit comprises: a) a first antibody specific for LOXL2; b) a second antibody specific for LOXL2; c) a third antibody, where the third antibody comprises a detectable label, and binds to the second antibody; and d) an insoluble support. The insoluble support can be provided in any of a variety of materials and formats, as described above. For example, in some instances, the insoluble support is a plastic multi-well plate, a test strip, or a dipstick.

A subject kit can further include purified LOXL2, for use in generating a standard curve.

A subject kit can further include one or more additional components, e.g., a buffer; a protease inhibitor; a detectable label; wash reagents; blocking agents; etc. The various components of the kit may be present in separate containers or certain compatible components may be pre-combined into a single container, as desired.

In addition to above-mentioned components, a subject kit can include instructions for using the components of the kit to practice a subject method. The instructions for practicing a subject method are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. compact disc-read only memory (CD-ROM), digital versatile disk (DVD), diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### 1. Assay device

The present disclosure further provides an assay device for use in detecting LOXL2 in a liquid biological sample obtained from an individual. The device can include a matrix defining an axial flow path.

The matrix can comprise: i) a sample receiving zone at an upstream end of the flow path that receives the liquid sample; ii) one or more test zones positioned within the flow path and downstream from the sample receiving zone, each of the one or more test zones comprising immobilized therein an antibody specific for LOXL2 in each of the test zones, to form an immobilized anti-LOXL2/LOXL2 complex; and iii) one or more control zones positioned within the flow path and downstream from the sample receiving zone, where the one or more control zones can include positive and/or negative controls. The test zones and control zones can be positioned in an alternating format within the flow path beginning with a test zone positioned upstream of any control zone.

The matrix can comprise: i) a sample receiving zone at an upstream end of the flow path that receives the liquid sample; ii) one or more test zones positioned within the flow path and downstream from the sample receiving zone, each of the one or more test zones comprising an antibody specific for LOXL2 in each of the test zones, to form an anti-LOXL2/LOXL2 complex; and iii) one or more control zones positioned within the flow path and downstream from the sample receiving zone, where the one or more control zones can include positive and/or negative controls. The test zones and control zones can be positioned in an alternating format within the flow path beginning with a test zone positioned upstream of any control zone. In some embodiments, the antibody specific for LOXL2 is not immobilized; and, when the anti-LOXL2 antibody binds any LOXL2 present in the sample, the anti-LOXL2 antibody/LOXL2 complex is mobilizable. For example, the anti-LOXL2 antibody/LOXL2 complex formed in a first test zone can be mobilized such that it enters a second test zone comprising an immobilized anti-LOXL2 antibody, where the anti-LOXL2 antibody/LOXL2 complex binds to the immobilized anti-LOXL2 antibody, forming an immobilized anti-LOXL2 antibody/LOXL2 complex.

In using such an assay device, in some embodiments, a labeled antibody specific for LOXL2 can first be mixed with a liquid sample before the liquid sample is applied to the sample receiving zone of the device, where such mixing results in a labeled antibody/LOXL2 complex. In these embodiments, the liquid sample comprising the labeled antibody/LOXL2 complex is applied to the sample receiving zone of the assay device. The liquid sample flows along the device until the liquid sample reaches a test zone. Antibody present in the test zone binds LOXL2 present in the labeled antibody/LOXL2 complex; and can then be detected.

The assay device can further include a label zone comprising a labeled antibody specific for LOXL2, where the labeled antibody is capable of binding LOXL2 present in an immobilized LOXL2/anti-LOXL2 antibody complex, to form a labeled LOXL2/anti-LOXL2 antibody complex, where the labeled antibody is mobilizable in the presence of liquid sample. In using such an assay device, a liquid sample which may comprise LOXL2 is applied to the sample receiving zone of the device; anti-LOXL2 antibody present in the label zone binds the LOXL2, forming labeled antibody/LOXL2 complex, which, like the labeled antibody, is mobilizable; and the labeled antibody/LOXL2 complex flows alone the device until the liquid sample reaches a test zone. Anti-LOXL2 antibody present in the test zone binds the LOXL2 present in the labeled antibody/LOXL2 complex; and can then be detected.

Alternatively, the assay device can include a label zone comprising a labeled antibody specific for an anti-LOXL2 antibody, where the labeled antibody binds to any anti-LOXL2 antibody/LOXL2 complexes formed in the test zone(s). In some cases, the labeled antibody is mobilizable.

The labeled antibody can comprise a label such as a chemiluminescent agent, a particulate label, a colorimetric agent, an energy transfer agent, an enzyme, a fluorescent agent, or a radioisotope.

Control zones include positive control zones and negative control zones.

The matrix is generally an insoluble support, where suitable insoluble supports include, but are not limited to, polyvinyl difluoride (PVDF), cellulose, nitrocellulose, nylon, and the like. The matrix can be flexible, or can be relatively inflexible. The matrix can be positioned within a housing comprising a support and optionally a cover, where the housing contains an application aperture and one or more observation ports. The assay device can be in any of a variety of formats, e.g., a test strip, a dipstick; etc.

Various aspects of the invention are further described and illustrated by way of the examples which follow, none of which are intended to limit the scope of the invention.

### Examples

The following examples are not intended to limit the scope of what the inventors regard as their invention; nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly).

### Example 1

### Administration of Anti-LOXL2 Antibody in a Mouse Model of Liver Fibrosis

### A. Carbon tetrachloride mouse model of liver fibrosis

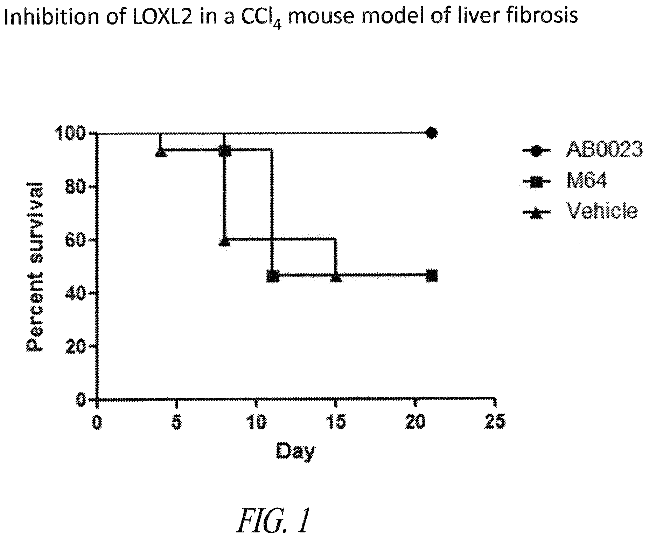

Fibrosis was induced in BALB/C mice using carbon tetrachloride (CCl₄). A murine anti-LOXL2 antibody (AB0023) was administered to the animals at a dose of 30 mg/kg twice weekly over three weeks. Control mice received CCl₄ only (vehicle) or a sham antibody (M64). As shown in Figure 1, mice receiving the anti-LOXL2 antibody AB0023 exhibited a survival benefit compared to control mice. Additionally, immunohistochemistry of liver tissues demonstrated that mice administered the anti-LOXL2 antibody exhibited a significant reduction of bridging fibrosis (p=0.01 vs. vehicle) (assessed via Sirius red staining) and a reduction in LOXL2 and alpha smooth muscle actin (αSMA; demonstrating a reduction in stellate cell activation) (p=0.008 vs. vehicle). See Barry-Hamilton V, et al. Nat Med 2010;16:1009-17.

Using the same fibrosis model, mice were administered with 30 mg/kg of BAPN (LOX inhibitor) or vehicle for 6 weeks. After 8 weeks, the treated mice exhibited reduced collagen crosslinking and net collagen deposition compared to the mice treated with vehicle (data not shown).

### B. TTA mouse model of fibrosis

In a different model, liver fibrosis was induced in C57B/6 mice by injection thioacetamide (TAA) three times per week for 12 weeks. Liver fibrosis was observed at week 6 and continued to advance through the study duration of 12 weeks. LOXL2 was not detected in normal liver but was detected in the mice showing liver fibrosis. Also, the levels of LOXL2 increased as the liver fibrosis advanced. From week 6 to 12, the mice were administered twice per week with anti-LOXL2 antibody AB0023 (30 mg/kg), anti-LOX antibody M64 antibody (30 mg/kg), BAPN (100 mg/kg), or vehicle.

After 6 weeks of treatment, the mice were examined for collagen content and connective tissue staining in liver. Compared to the mice treated with M64 or vehicle, the mice treated with AB0023 exhibited about 20% reduction in net collagen reduction (Figure 33A and Figure 33B) and attenuated bridging fibrosis (data not shown).

In another study, the mice were treated with TAA for 6 weeks followed by treatment with AB0023 (30 mg/kg), M64 (30 mg/kg), BAPN (100mg/kg) or vehicle from week 6 to 18. At week 10, the mice exhibit recovery from liver fibrosis. Also, the mice treated with AB0023 exhibited earlier recovery compared to the mice treated with BAPN or vehicle (Figure 33C). The mice treated with AB0023 also showed reduced collagen gel contraction by hepatic stellate cells in a dose-dependent manner *in vitro* compared to other LOX inhibitors (Figure 33D).

### Example 2

### Administration of Anti-LOXL2 Antibody to Human Subjects with Liver Disease

Groups of human subjects between the ages of 18 and 60 having liver fibrosis and a Metavir fibrosis stage between 1 and 3 were administered an anti-LOXL2 antibody. Each of the subjects had a BMI less than 36 kg/m², no history of decompensated liver disease, liver disease (various etiologies), Creatinine levels less than 2.0 mg/dL, alanine aminotransferase (ALT) / aspartate aminotransferase (AST) ratio of less than 10x upper limit of normal (ULN), and was in general good health. Subjects with viral hepatitis on treatment were excluded. All Metavir fibrosis scores were assessed by the same hepatopathologist.

### 10-subject cohort

A group of ten such subjects included nine males and one female. Nine of the ten had chronic hepatitis C infection, four with HIV co-infection; the other had nonalcoholic steatohepatitis and HIV. The ten subjects had a mean age of 53 years (36-60 years), a mean weight of 81.4 kg (64.5-98.9 kg), and a mean Metavir fibrosis stage of 2.1 (three stage 1; three stage 2; four stage 3).

An anti-LOXL2 antibody (AB0024, a humanized IgG4 monoclonal antibody that specifically binds to LOXL2) was administered to each subject by intravenous infusion at a dose of 10 mg/kg (mean dose of 806.5 mg per infusion (630-953 mg)), infused over 1 hour, every 2 weeks for a total of 3 infusions over a four-week period. Subjects subsequently were monitored for six weeks. Subjects underwent liver biopsy at screening and liver FNAC prior to the first infusion and at the end of the treatment period. All ten subjects completed the regimen of 3 infusions uneventfully, with no iinfusion or dose interruptions, no early terminations, and no dose reductions.

No deaths, serious adverse events (SAEs), or adverse events leading to discontinuation of dosing were reported. Of the 28 total adverse events reported, in nine subjects, none was grade 3 or 4. The most frequently reported adverse events were abdominal pain (5 subjects, 50 %; apparently unrelated to treatment, likely related to FNA procedures), fatigue (2 subjects, 20 %), musculoskeletal pain (2 subjects, 20 %), and headache (2 subjects each). Each of the following was reported in one subject each: nausea, vomiting, salivary gland enlargement, chest pain, influenza-like illness, pain, sialoadenitis, weight increase, arthralgia, hypoesthesia, presyncope, epididymal cyst, epididymitis, cough, and rash. No lab abnormalities were noted.

### Liver enzymes, ALT

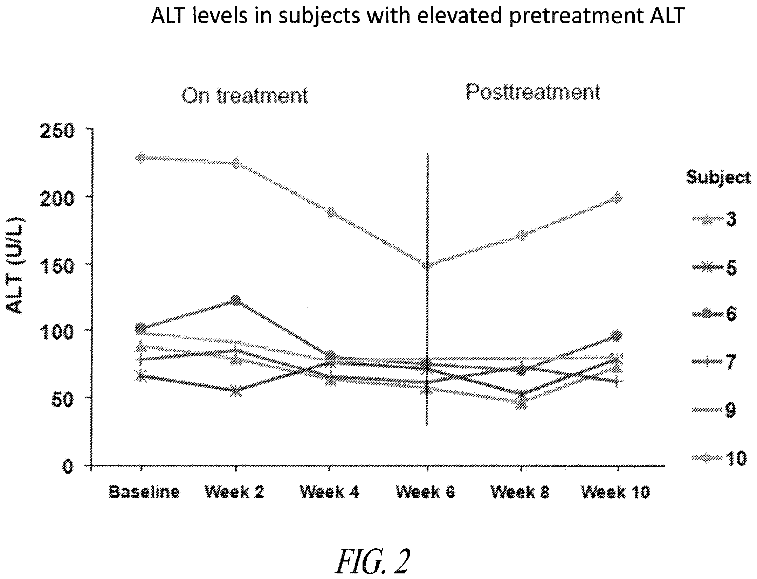

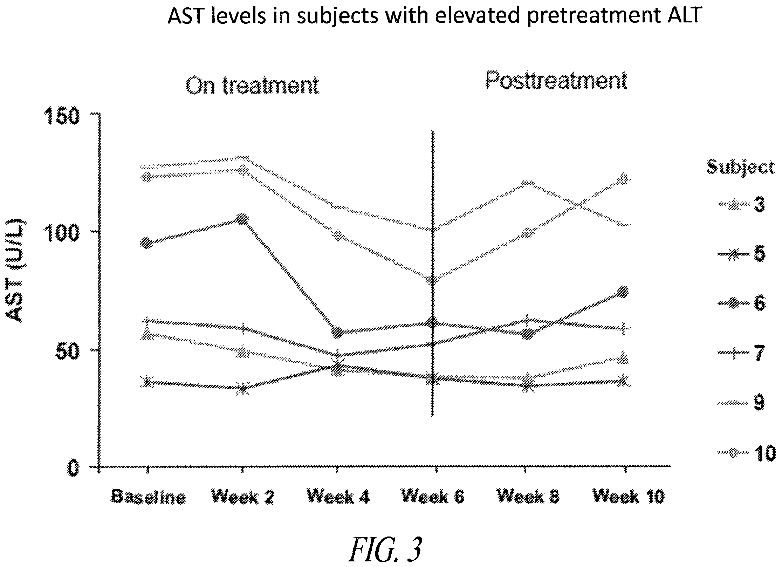

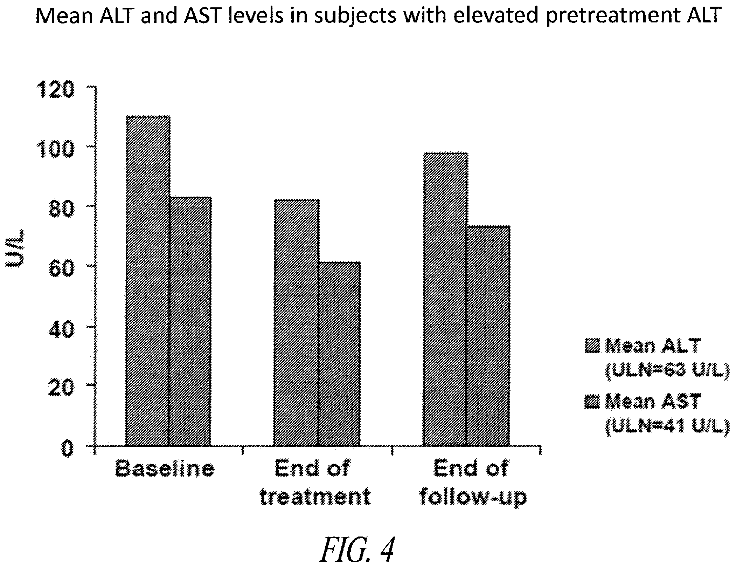

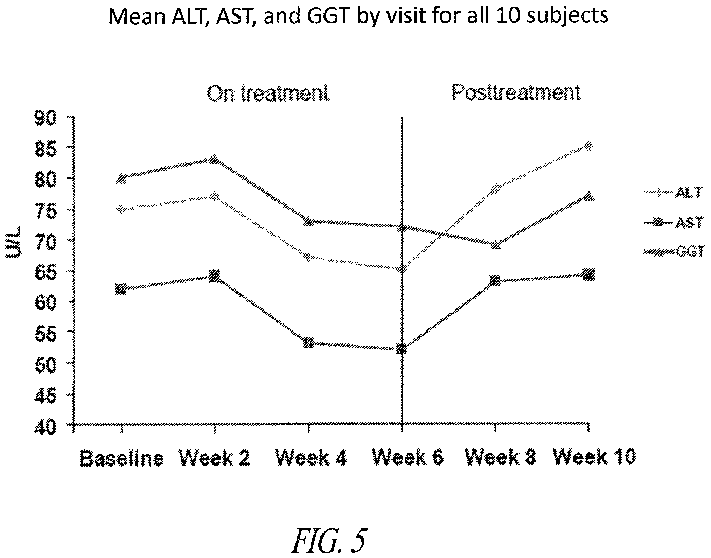

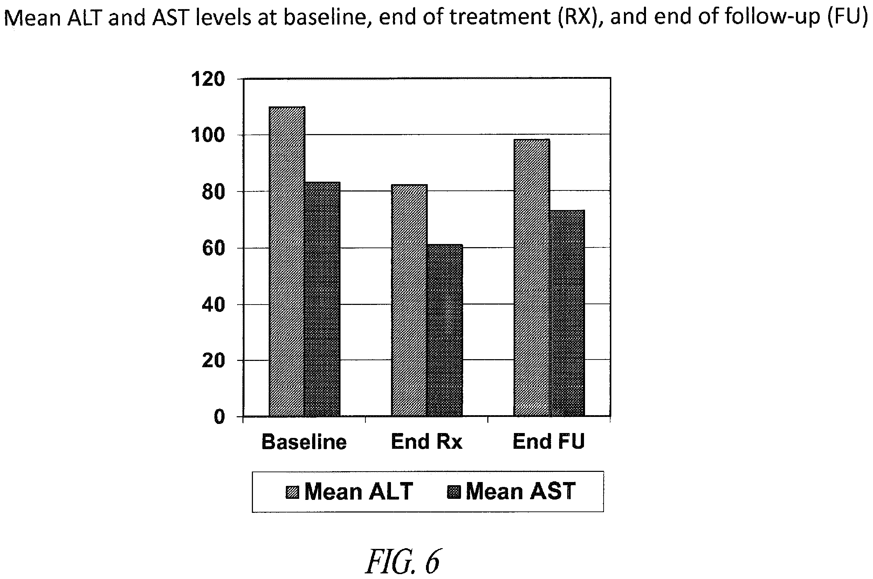

No subject experienced a worsening in liver enzymes on therapy. No medication-associated increases in transaminases were observed. Six of the ten subjects exhibited elevated aminotransferase levels (ALT>ULN (63 upper limit (U/L))) prior to treatment (baseline levels). Five of these six subjects exhibited a reduction in ALT by the end of treatment, with a decline after the second infusion, with all showing a slight increase in ALT toward the baseline level during the follow-up period (Figure 2). AST (41 U/L) levels were similarly improved following treatment of these subjects (Figure 3). These subjects exhibited a mean 22 % reduction in ALT following treatment (Figure 4). The mean values among all 10 subjects for ALT, AST, and gamma-glutamyltransferase (GGT) decreased following treatment and returned toward baseline levels posttreatment (Figure 5). Figure 6 shows mean ALT and AST levels in subjects with elevated pretreatment AST/ALT levels at baseline, end of treatment, and end of follow-up period. p = 0.02 for AST at end of treatment versus baseline.

Accordingly, in this study, administration of anti-LOXL2 antibody was safe and well-tolerated in subjects having hepatic fibrosis associated with viral hepatitis at doses up to 953 mg IV (10 mg/kg) every other week for four weeks. Administration of the anti-LOXL2 antibody significantly reduced transaminases in subjects with elevated pretreatment aminotransferase levels.

### sLOXL2 and Inflammation

Reduction of liver enzymes by anti-LOXL2 treatment suggest a functional role for LOXL2 in liver inflammation in addition to its role in fibrosis. Additionally, overall Knodell necroinflammatory index was found to be associated with LOXL2 on univariate analysis (p=0.001). Among individual components of the Knodell necroinflammatory index, both the Knodell inflammation score and the necrosis score correlated significantly with serum LOXL2 levels (p=0.02 for both on univariate analysis). Table 2 shows results of multivariate regression analysis for subjects in this study.

**Table 2 Multivariate regression analysis**

| | **Regression Coefficient** | **p-value** |
|---|---|---|
| **log₁₀ AST** | 0.830 | 0.0047 |
| **Ishak Fibrosis Score** | 0.061 | 0.0393 |
| **log₁₀ ALT** | - 0.442 | 0.0654 |
| **Genotype¹** | 0.043 | 0.6751 |
| **Gender** | 0.038 | 0.7032 |
| **Race²** | - 0.030 | 0.7873 |
| **Age** | 0.001 | 0.7962 |
| **Knodell NS** | 0.001 | 0.9721 |

| | | |
|---|---|---|
| 1=D vs. other; 2=white vs. other | | |

### Additional subjects

Similar safety results were observed in IPF subjects who received 3 infusions of AB0024 at 3 (n=4), 10 (n=4), or 20 (n-4) mg/kg every 2 weeks or 7 infusions of AB0024 at 125 (n=15) or 200 (n=15) mg every 2 weeks. In another study, healthy subjects received a single dose of AB0024 at 125 mg/kg by subcutaneous (n=18) or IV (n=18) administration. No severe adverse effects were detected. In another study, subjects are administered anti-LOX2 antibody (e.g., AB0024), 700 mg IV every other week for six months. In another study, AB0024 is administered to subjects with liver fibrosis at fixed doses of 200 mg or 700 mg IV, or 75 mg or 125 mg subcutaneously (SC).

### Example 3

### Immunoassay for Detecting LOXL2 in Human Serum or Plasma Samples

### MATERIALS AND METHODS

### Antibodies

A rabbit polyclonal antibody ("rabbit A") was raised against recombinant purified full-length LOXL2 protein. The antibody recognizes multiple epitopes in all domains of LOXL2. Mouse monoclonal antibody, AB0030, binds to the catalytic domain of LOXL2 and recognizes both the full-length LOXL2 protein and the mature LOXL2 protein (which is cleaved between SRCR2 and SRCR3 domains).

### LOXL2 immunoassay on MSD platform

Standard single-spot uncoated electrode plates from MesoScale Discovery (MSD) (cat #L15XA-3) were coated overnight at 4°C with a 30µl volume of a solution of 3µg/ml rabbit anti-human-LOXL2 polyclonal antibody formulated in phosphate-buffered saline (PBS). After coating, the wells of the plates were blocked by addition of a solution of 5% (w/v) Blocker A (MSD cat#R93AA-1) in PBS. After the blocking step, plates were washed 3 times in PBS containing 0.05% Tween-20 non-ionic detergent, using an automated plate washer. Human samples to be tested (serum or plasma) were prepared separately by diluting them 1:4 in PBS (1 part serum, 3 parts PBS). Samples were then added to each well of the plate. Samples were incubated with rotary shaking (300-600rpm) for 2-3 hours at room temperature. After sample binding, the plates were again washed 3 times in PBS containing 0.05% Tween-20 detergent, using an automated plate washer.

A solution of 1 µg/ml AB0030 (primary antibody) in 2% (w/v) Blocker A in PBS was added to each well and the plates incubated with rotary shaking (300-600rpm) for 1 hour at room temperature. After AB0030 binding, the plates were again washed 3 times in PBS containing 0.05% Tween-20 detergent, using an automated plate washer.

The secondary antibody was a goat-anti-mouse-IgG molecule conjugated to SulfoTag dye (MSD cat#R32AC-5). A solution of 1µg/ml secondary antibody in 2% (w/v) Blocker A in PBS was added to each well, and the plates were incubated with rotary shaking (300-600rpm) for 1 hour at room temperature. After secondary antibody binding, the plates were washed 3 times in PBS containing 0.05% Tween-20 detergent, using an automated plate washer.

1x Read Buffer T with Surfactants (MSD cat#R92TC-2) was added to each well, followed by immediate measurement of the plate on the MSD SectorImager 2400 instrument.

Test human samples were given a relative quantitative value of LOXL2 by comparison to the calibrator curve on the same assay plate, comprised of purified recombinant human LOXL2 protein (R&D Systems) added in known concentrations to human serum or plasma pooled from normal healthy donors. Calibrator curve fitting and unknown sample interpolation were carried out using standard techniques.

### LOXL2 immunoassay using standard format

Costar 3922 high-binding multi-well plates were used. Rabbit polyclonal antibody (Ab) (rabbit "A") was diluted to 0.625 µg/ml in CB2 coating buffer (Immunochemical Technologies CB2 (6248)). Diluted polyclonal Ab was added to the wells of the plate at a volume of 50 µl/well, and the plates were kept at 4°C overnight. After coating the wells with the polyclonal antibody, wells were blocked with 200 µl/ well of BB1 block solution (Immunochemical Technologies product # 640) for 1-3 hr at room temperature (RT). Following blocking, the plates were washed 3x using 200 µl per well PBS-T (PBS containing 0.05% Tween 20).

25 µl HiSpec diluent (AbD Serotec BUF049B) was added to each well. An equal volume of test serum was then added to each well; and plates were kept at room temperature for 2 hours. After allowing the serum samples to bind, the plates were washed three times.

The primary antibody (AB0030) was diluted to 5 µg/ml in PBS-T + 0.5% bovine serum albumin (BSA); 50 µl of the diluted primary antibody was added to each well. Plates were kept at room temperature for one hour, then washed three times with PBS-T. The secondary antibody (horse radish peroxidase (HRP)-conjugated goat anti-mouse antibody, Jackson Immunoresearch, 0.8mg/ml) was diluted 1:10,000 in PBS-T + 0.5% BSA. 50 µl of the diluted secondary antibody was added to each well. Plates were kept at room temperature for one hour, then washed three times with PBS-T.

### Example 4

### Calibrator Standards for LOXL2 Immunoassay in Human Serum Matrix

Using the LOXL2 immunoassay described in Example 3 (sandwich mmunoassay developed on the MesoScale Discovery platform), LOXL2 was not detected in serum from healthy individuals. To create a calibrator curve, purified recombinant full-length LOXL2 protein was added into pooled normal human serum, followed by serial dilution in serum.

### RESULTS

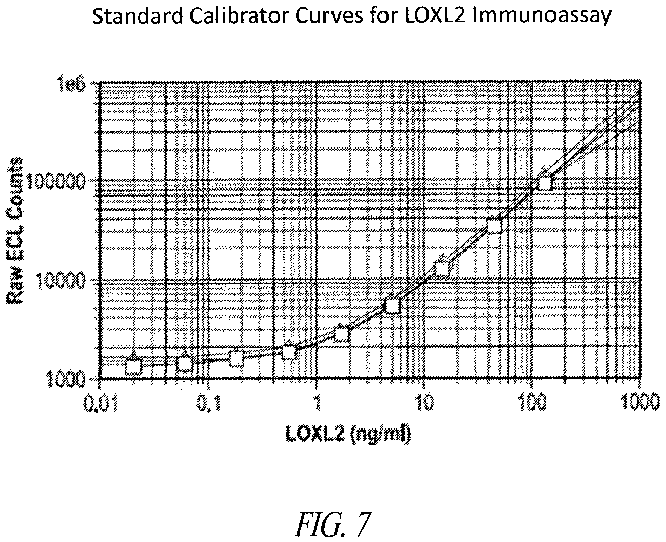

The results are shown in Figure 7. Each data point represents the mean of 3 replicate wells; curves for 4 independent plates are shown.

Table 3 shows the characteristics of calibrator standards in human serum matrix. In Table 3, lower limit of detection (LLOD) is the mean + 2.5*stdev of the blank wells (raw values, extrapolated); Lower limit of quantitation (LLOQ) is the lowest calibrator standard with relative error < 30% and coefficient of variation < 30% for the raw measurements. Intra-assay and inter-assay precision were determined using incurred samples.

**Table 3 LOXL2 immunoassay: characteristics of calibrator standards in human serum matrix**

| **Assay characteristic** | **Result** |
|---|---|
| Accuracy (relative error) | <15% |
| Intra-assay precision | 3.5% |
| Inter-assay precision | 15.5% |
| Recovery after freeze/thaw cycle | 70% one cycle, >70% 2 or more cvcles |
| Lower limit of detection (LLOD) | 150-200 pg/ml |
| Lower limit of quantitation (LLOQ) | 180-550 pg/ml |
| Upper limit of quantitation (ULOQ) | Not determined |

### Example 5

### Serum LOXL2 (sLOXL2) Levels in Patients with Idiopathic Pulmonary Fibrosis (IPF)

### A: sLOXL2 in IPF Patients

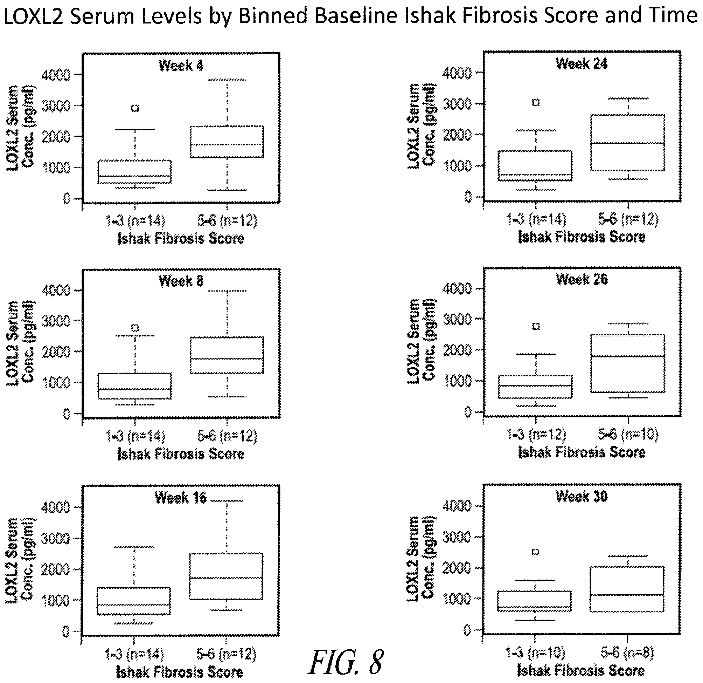

Serum samples from 15 patients with a diagnosis of idiopathic pulmonary fibrosis (IPF) were tested for LOXL2. The results are shown in Figure 8. Individual patient identification numbers are shown. Ten of 15 patients tested positive; the other 5 were below the limit of detection and are reported as "not detected." Age-matched normal subjects were also tested; all were negative ("not detected"; below the limit of detection) for serum LOXL2.

### B: LOXL2 baseline levels in IPF Patients

### 1. ARTEMIS-IPF Patients

Serum samples were collected from subjects participating in the ARTEMIS-IPF trial. a randomized, double-blind, placebo-controlled, event-driven trial. Subjects were randomized in a 2:1 ratio to receive ambrisentan, a selective antagonist of the ET_{A} receptor, or placebo. This study was terminated prematurely; 660 subjects were enrolled.

Baseline variables reflective of IPF severity and functional status were collected. The baseline variables included percent of predicted forced vital capacity (FVC), percent of predicted carbon monoxide diffusion capacity (DL_{CO}), 6-minute walk distance (6MWD), mean pulmonary artery pressure (mPAP), the lowest resting oxygen saturation (SpO2), the composite physiologic index (CPI), the St. George's Respiratory Questionnaire score (SGRQ), and the Transition Dyspnea Index (TDI) score. The mPAP was obtained via a right heart catheterization, which was required of all study subjects at baseline. The CPI was a validated multidimensional model incorporating FVC, the forced expiratory volume in one-second (FEV₁) and DL_{CO} to estimate the extent of fibrosis seen on a computed tomographic scan of the patient's chest. The primary endpoint was time to IPF disease progression, a composite endpoint defined as one of the following: mortality from any cause, respiratory hospitalization, or a categorical decrease in lung function, defined as either a 10% decrease in forced vital capacity (FVC) with a 5% decrease in the diffusion capacity for carbon monoxide (DL_{CO}) or a 15 % decrease in DL_{CO} with a 5% decrease in FVC. Lung function endpoints were confirmed by two pulmonary function tests conducted at least 4 weeks apart.

The baseline levels of LOXL2were quantified in triplicate using an immunoassay developed on the MesoScale Discovery platform using anti-LOXL2 antibodies described in Example 3.

Standard histograms were used to evaluate untransformed and log₁₀X transformed LOXL2 baseline levels. Student's T-test was used to compare distribution of baseline variables. Pearson's correlation coefficient was used to examine the relationship between LOXL2 baseline levels and baseline variables. Stepwise Cox proportional hazard modeling was used to examine the relationship between LOXL2 baseline levels and IPF outcomes. Receiver operating curves were used to estimate the area under the curve.

### RESULTS

Serum samples from 69 subjects in the intent-to-treat cohort were available for the analysis. In comparison to the 423 subjects from ARTEMIS-IPF for whom no serum samples were available, there were no statistically significant differences in baseline measures of IPF severity or functional status (Table 4). However, among the 69 subjects, there were statistically significant differences in baseline measures of IPF severity and functional status when comparing the ambrisentan and the placebo treatment groups (Table 5). Subjects in the ambrisentan group had lower baseline DL_{CO} (p=0.035), lower baseline 6MWD (p=0.004), higher baseline mPAP (p=0.016), higher baseline CPI (p=0.05) and higher baseline SGRQ (p=0.011). The mean baseline LOXL2 level was higher for the ambrisentan subjects (p=0.026).

Analysis of the distribution of LOXL2 baseline levels showed 8 subjects having LOXL2 levels of less than about 88 pg/mL, 34 subjects having LOXL2 levels of about 88 to about 440 pg/mL, and 28 subjects having LOXL2 levels of more than about 440 pg/mL. The median LOXL2 level was about 325 pg/mL with an interquartile range of about 147 pg/mL to about 770 pg/mL, and minimum of about 18 pg/mL and maximum of about 5,400 pg/mL.

Based on Pearson's correlation coefficient, correlation was weak between LOXL2 baseline levels and these baseline measures of IPF severity and functional status. Figure 9 shows scatter plot matrices representing the relationship between LOXL2 baseline levels and FVC, DL_{CO}, 6MWD, CPI, SGRQ, and TDI. Correlations between LOXL2 and baseline severity measures were highlighted within the dark boxes at the top row of panels (a) and (b). The correlation coefficients between LOXL2 and the individual baseline severity measures were as follows: -0.21 (FCV), -0.11 (DL_{CO}), 0.03 (6MWD), 0.10 (mPAP), -0.07 (SpO₂), 0.14 (CPI), 0.06 (SGRQ), and -0.05 (TDI). Whereas Log₁₀X transformation of the LOXL2 baseline levels normalized the distribution, correlation between LOXL2 and baseline measures of IPF severity and functional status remained weak (Figure 9b).

Given the majority of the baseline LOXL2 levels were less than about 800 pg/mL, the LOXL2 baseline levels were dichotomized as ≤ 800 pg/mL ("low") versus > 800 pg/mL ("high") for the remainder of the analysis. Of the 28 subjects having LOXL2 baseline levels of more than about 440 pg/mL, 12 had low LOXL2 baseline levels of about 440-800 pg/mL and were grouped into the low group; and 16 had LOXL2 baseline levels of more than 800pg/mL and were grouped into the high group.

Comparison of disease progression between the "high" and "low" LOXL2 baseline level groups is shown in Figure 10. Because there were only two patients having "high" LOXL2 baseline lines in the placebo group (neither of which had any events), Figure 10 compares only "low" and "high" LOXL2 baseline levels in the ambrisentan group. Results indicated that high LOXL2 baseline level was associated with more disease progression events (Figure 10a) and that high LOXL2 baseline levels were associated with more lung function decline events (Figure 10b), more respiratory hospitalizations (Figure 10c) and more deaths (Figure 10d).

Additionally, as shown in Table 6, Cox proportional hazard modeling indicated that presence of a high LOXL2 baseline level was associated with a 5-fold increase in risk for disease progression (hazard ratio [HR] 4.95, 95% confidence interval [CI] 1.52-16.18, p=0.008), a 7-fold increase in risk for lung function decline (HR 7.36, 95% CI 1.16=46.74, p=0.034), and a 5-fold increase in risk for respiratory hospitalization (HR 4.85, 95% CI 1.09-21.68, p=0.039). All of these statistical models were adjusted for treatment assignment and baseline 6MWD and CPI score. High baseline LOXL2 levels were not significantly associated with a significant increase in risk for death (HR 1.59, 95% CI 0.24-10.53, p=0.633).

Samples were also analyzed for levels of MMP7, ICAM1, IL8, VCAM1, and S100A12. None of these proteins was significantly associated with the treatment outcomes. The results showed that high baseline LOXL2 levels were associated with a 5-7 fold increase in risk for IPF disease progression, but not death.

**Table 4 Comparison of baseline IPF severity and functional status according to availability of serum in ARTEMIS-IPF**

| Baseline Measures of IPF Severity | No Serum N=423 | Serum N=69 | P-value |
|---|---|---|---|
| Mean % FVC (SD) | 69(14) | 70 (12) | 0.649 |
| Mean % DLCO (SD) | 43 (14) | 42(11) | 0.487 |
| Mean 6MWD m (SD) | 416 (120) | 399(116) | 0.256 |
| Mean PAP mmHg (SD) | 20 (7) | 20 (6) | 0.920 |
| Mean lowest SpO2 % (SD) | 88 (6) | 88(6) | 0.825 |
| Mean CPI (SD) | 52(11) | 53(9) | 0.784 |
| Mean SGRQ (SD) | 39 (20) | 38(18) | 0.605 |
| Mean TDI (SD) | 7(2) | 8(2) | 0.588 |

**Table 5 Comparison of baseline IPF severity and functional status according to treatment assignment in ARTEMIS-IPF among subjects with baseline serum available**

| Baseline Measures of IPF Severity | Ambrisentan N=49 | Placebo N=20 | P-value |
|---|---|---|---|
| Mean % FVC (SD) | 68(12) | 73(12) | 0.128 |
| Mean % DLCO (SD) | 40(11) | 47(9) | 0.035 |
| Mean 6MWD m (SD) | 373 (109) | 461(110) | 0.004 |
| Mean PAP mmHg (SD) | 22(6) | 18(5) | 0.016 |
| Mean lowest SpO₂ % (SD) | 87 (6) | 87(5) | 0.166 |
| Mean CPI (SD) | 54 (9) | 49 (8) | 0.050 |
| Mean SGRQ (SD) | 42 (19) | 29(15) | 0.011 |
| Mean TDI (SD) | 7(2) | 8(2) | 0.083 |
| Mean LOXL2 (SD) | 903(1172) | 295(288) | 0.026 |

**Table 6 Levels of baseline LOXL2 in IPF patients and its relationship with study endpoints.**

| Endpoints | # of Events | | Hazard Ratio (95% CI) | P-value |
|---|---|---|---|---|
| | Low LOXL2 | High LOXL2 | for High LOXL2 | |
| Disease Progression | 10 | 8 | 4.95 (1.52-16.18) | 0.008 |
| Lung Function Decline | 5 | 4 | 7.36 (1.16-46.74) | 0.034 |
| Respiratory Hospitalization | 6 | 6 | 4.85 (1.09-21.68) | 0.039 |
| Death | 5 | 4 | 1.59 (0.24-10.53) | 0.633 |

### 2. GAP cohort IPF Patients

Serum LOXL2 levels were assessed in subjects in a second clinical IPF prospective follow-up study, which assessed disease progression in 111 IPF subjects (deemed the GAP cohort) who had no history of other lung illnesses. All GAP cohort subjects were diagnosed with IPF according to ATS/ERS guidelines, confirmed by surgical lung biopsy or radiographic findings of subpleural honeycomb changes, traction bronchiectasis, and minimal alveolar filling in patients over 55 years of age and without a defined etiology. Pulmonary function testing revealed a forced vital capacity of 40-70% predicted. Subjects were able to receive all ongoing care and follow-up at a clinical facility.

At the initial visit, each participant had a blood draw, pulmonary function testing, 6-minute walk test (6MWT), echocardiogram, and CT scan, and several questionnaires designed to measure how the patient was feeling. At follow-up visits in 3-8 month intervals, blood samples were collected and PFTs, questionnaires, and 6MWTs were repeated. The median FVC, FEV1, and DLCO were 65.7 ± 17.5%, 76.8 ± 18.7%, and 47.3 ± 17.9% of the predicted values, respectively.

Baseline serum levels of LOXL2 were quantified as described above for the ARTEMIS-IPF subjects. Standard histograms were used to evaluate LOXL2 baseline serum levels at the natural log format. LLOD of 180 pg/mL and LLOQ of 440 pg/mL were determined experimentally.

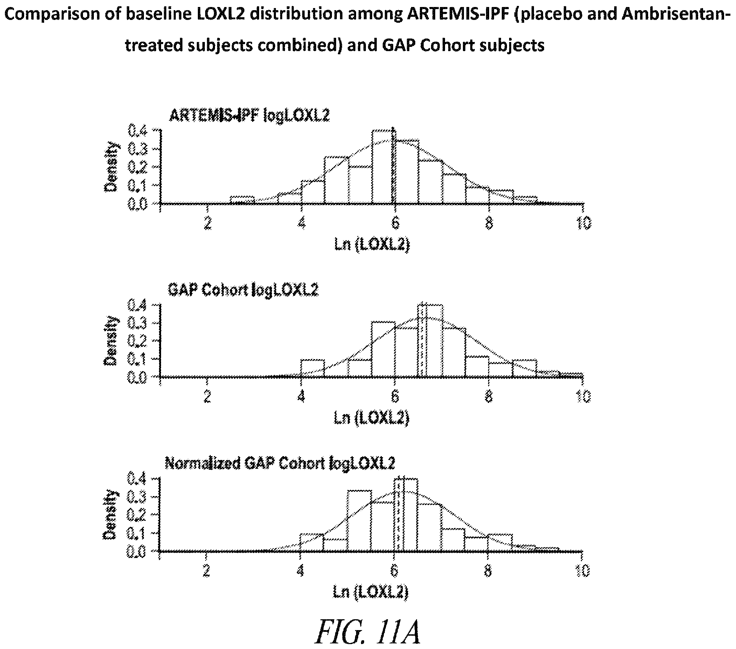

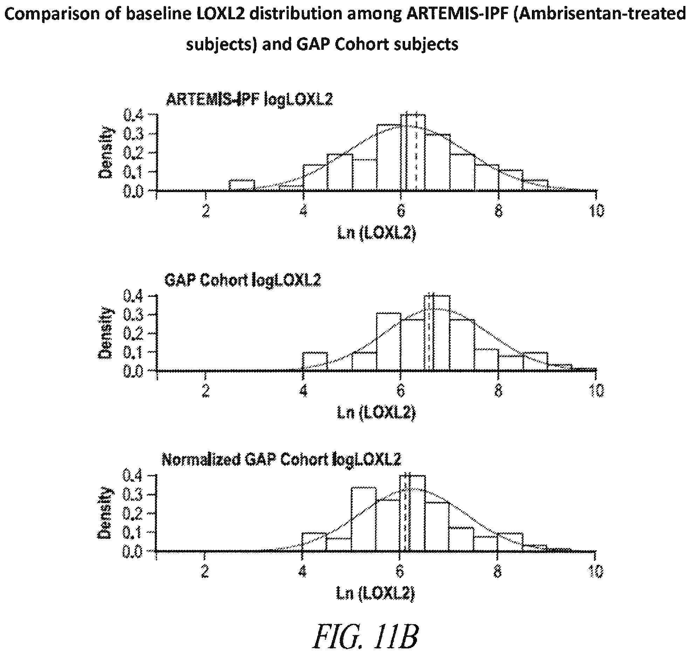

LOXL2 levels for the GAP cohort were normalized to the ARTEMIS-IPF data after natural log transformation using a regression method. The results are shown in Figure 11.

Time to all-cause mortality was assessed, with a lung transplant considered a death event (most lung transplant patients died). A classification and regression trees (CART) method was applied as an unbiased approach to select the optimal threshold or cut-off point for dichotomization of the baseline serum LOXL2 levels. In the GAP cohort, when Log(LOXL2) was the only variable, CART analysis selected 440 pg/mL (6.08 at natural log scale) as the cut-off point.

Table 7 shows baseline and demographic characteristics for subjects in the GAP cohort, and Table 7 shows correlation among various baseline values in this cohort.

**Table 7 GAP Cohort Baseline and demographic characteristics**

| Variable | N | Mean (Std) | Median (Min, Max) |
|---|---|---|---|
| Sex | M: 74 (67%) | | |
| | F: 37 (33%) | | |
| Age(Years) | 111 | 67 (9.3) | 67 (3, 84) |
| FVC %Predicted | 73 | 66 (18) | 64 (34, 113) |
| FEV₁ %Predicted | 73 | 77(19) | 74 (37, 129) |
| DLCO %Predicted | 73 | 48 (18) | 46 (14, 109) |
| CPI | 73 | 52 (13) | 52 (12, 78) |
| 6 Min Walk Distance | 17 | 912 (420) | 890 (100, 1555) |
| LOXL2 | 111 | 1495 (2307) | 717 (90, 15708) |
| LOG(LOXL2) | 111 | 7 (1) | 7 (5, 10) |
| LOG (LOXL2)* | 111 | 6 (1) | 6 (5, 9) |

| | | | |
|---|---|---|---|
| * Normalized LOXL2 through a regression method | | | |

**Table 8 Correlation among baseline variables**

| | Age | FVC % pred. | FEV1% pred. | DLCO % pred. | CPI |
|---|---|---|---|---|---|
| Log LOXL2 | -0.7 | -0.03 | -0.06 | -0.28 | -0.24 |
| Age | | 0.07 | 0.23 | 0.02. | 0.05 |
| FVC % pred. | | | 0.93 | 0.38 | -0.61 |
| FEV1 % pred. | | | | 0.47 | -0.60 |
| DLCO % pred. | | | | | -0.95 |

The correlation between the dichotomized LOXL2 levels and all cause mortality was evaluated using Cox proportional hazard modeling and Kaplan-Meier survival plots at six (6) months, twelve (12) months, eighteen (18) months, and twenty-four (24) months after baseline. The correlation between baseline LOXL2 levels and hospitalization and lung function decline was not evaluated as data was not available.

Analysis of the distribution of baseline LOXL2 levels showed a skewed distribution toward the lower spectrum, similar to that observed for the ARTEMIS-IPF cohort. The median baseline LOXL2 level was 716.5pg/mL (interquartile range 358.3 pg/ml, 1446.6 pg/ml). Correlation was weak between LOXL2 and baseline demographics and baseline clinical indicators of IPF severity (correlation coefficients for age -0.07, FVC -0.03, DLCO -0.28). No additional clinical indicators of disease severity were available for further analysis.

The results showed that a threshold 440 pg/ml baseline serum LOXL2 level was correlated with the risk for all-cause mortality. Presence of a baseline LOXL2 level higher than 440 pg/mL in the serum was associated with more deaths at 12-, 18-, and 24-months after baseline (Figure 12A and B).

Multivariate Cox proportional hazard modeling (covariates included age and sex) suggested that presence of a baseline LOXL2 level higher than 440 pg/mL was associated with a 2.3-fold increase in risk for death at 12-, 18-, and 24-months after baseline (see Table 9 and Table 10).

**Table 9 Event rates and hazard ratios for subjects with low (<440 pg/mL) versus high (>440 pg/mL) baseline LOXL2 levels at 6-, 12-, 18-, and 24-months after baseline in GAP cohort.**

| Time after Baseline | Event Rate | | Hazard ratio* (95% CI) | P-value |
|---|---|---|---|---|
| | Low LOXL2 | High LOXL2 | | |
| 6 months | 5/52 (10%) | 10/59 (17%) | 1.76 (0.60, 5.22) | 0.3051 |
| 12 months | 10/52 (19%) | 23/59 (39%) | 2.27 (1.05, 6.98) | 0.0319 |
| 18 months | 12/52 (23%) | 26/59 (44%) | 2.22 (1.12, 4.43) | 0.0231 |
| 24 months | 14/52 (27%) | 30/59 (51%) | 2.31(1.22, 4.37) | 0.0105 |

| | | | | |
|---|---|---|---|---|
| * Models include age and sex as covariates | | | | |

**Table 10 Event rates and hazard ratios for subjects with low (≤440 pg/ml) versus high (>440 pg/ml) baseline LOXL2 levels at 6-, 12-, 18-, and 24-months after baseline.**

| Time after Baseline | Event Rate | | Hazard ratio (95% CI) | P-value |
|---|---|---|---|---|
| | Low LOXL2 | High LOXL2 | | |
| 6 months | 2/36 (6%) | 3/13 (23%) | 5.08 (0.85, 30.47) | 0.0756 |
| 12 months | 5/36 (14%) | 3/13 (23%) | 1.90 (0.45, 7.99) | 0.3796 |
| 18 months | 5/36 (14%) | 3/13 (23%) | 1.90 (0.45, 7.99) | 0.3796 |
| 24 months | 5/36 (14%) | 4/13 (31%) | 2.11(0.54, 8.24) | 0.2846 |

For a subset of the subjects, additional serum samples were collected prospectively. Over the duration of the study, two (2) samples were collected from 60 subjects, three (3) samples were collected from 42 subjects, four (4) samples were collected from 31 subjects, five (5) samples were collected from 17 subjects, six (6) samples were collected from 12 subjects, seven (7) samples were collected from seven (7) subjects, and eight (8) samples were collected from two (2) subjects. None of the samples were collected in association with an acute exacerbation.

Multivariate Cox proportional hazards modeling (with covariates including age and sex) was used, incorporating LOXL2 levels in each of the samples as a time-dependent continuous variable, to evaluate the relationship between serum LOXL2 levels and all-cause mortality. Serum LOXL2 levels measured over time were associated with the risk for mortality (p=0.003). In the GAP cohort, for each 2.7-fold increase in serum LOXL2 level drawn at any time during the study, the risk for mortality increased by 1.63 fold (95% confidence interval 1.19-2.25).

Table 11 shows results of a multivariate analysis with serum LOXL2 levels at various times after baseline.

**Table 11 Multivariate analysis according to low (<440 pg/mL) versus high (>440 pg/mL) serum LOXL2 levels at 6-, 12-, 18- and 24-months after baseline**

| **Response Variable** | **Model Term** | **Hazard Ratio (95 % CI)** | **p-value** |
|---|---|---|---|
| Time to death 6 month | Log LOXL2 (≤ or > 6.08) | 1.8 (0.6, 5.2) | 0.305 |
| | Sex | *0.5 (0.1 1.8) | 0.299 |
| | Age (continuous) | 1.0 (1.0, 1.1) | 0.931 |
| Time to death 12 months | Log LOXL2 (≤ or > 6.08) | 2.3 (1.1, 7.0) | 0.032 |
| | Sex | *0.4 (0.2, 0.9) | 0.037 |
| | Age (continuous) | 1.0 (1.0, 1.0) | 0.647 |
| Time to death 18 months | Log LOXL2 (≤ or > 6.08) | 2.2 (1.1, 4.4) | 0.023 |
| | Sex | *0.5 (0.2, 1.0) | 0.052 |
| | Age (continuous) | 1.0 (1.0, 1.0) | 0.848 |
| Time to death 24 months | Log LOXL2 (≤ or > 6.08) | 2.3 (1.2, 4.4) | 0.011 |
| | Sex | *0.4 (0.2, 1.0) | 0.026 |
| | Age (continuous) | 1.0 (1.0, 1.0) | 0.808 |

| | | | |
|---|---|---|---|
| *Hazard ratio favors female patients | | | |

The results of the GAP cohort were similar to those of the ARTEMIS-IPF study described above. Both studies showed that a baseline serum LOXL2 level that was higher than the threshold level was associated with an increased risk of negative outcome in IPF patients.

### C: Serum LOXL2 levels in IPF Patients

Serum and clinical data were analyzed from sixty-seven (67) subjects participating in the ARTEMIS-IPF trial (described above) and one hundred four (104) subjects in the GAP cohort (described above). Serum LOXL2 (sLOXL2) levels were quantified by LOXL2 ELISA as described in Example 3. Disease progression (DP), including lung function (LF) decline (10% decrease in FVC and 5% decrease in DL_{CO} or 15 % decrease in DL_{CO} and 5% decrease in FVC), respiratory hospitalizations (RH) and mortality), served as the primary endpoint for the ARTEMIS-IPF subjects. Disease progression (DP) without respiratory hospitalizations (RH) was used as the primary endpoint for the GAP cohort. A classification and regression trees (CART) method was used to select optimal thresholds for dichotomization of sLOXL2 levels in each study.

Compared to the ARTEMIS-IPF subjects assessed in this study, higher sLOXL2 levels were measured in the GAP subjects (medians 324pg/mL (interquartile range [IQR] 147, 770) and 716pg/mL (IQR 358, 1447), respectively). In both cohorts, while sLOXL2 correlated only weakly with baseline FVC and DL_{CO} (r range -0.25 to 0.05), sLOXL2 levels were significantly associated with IPF outcomes. CART-determined thresholds for dichotomization of sLOXL2 levels were 800pg/mL for ARTEMIS-IPF subjects and 700pg/mL for GAP subject.

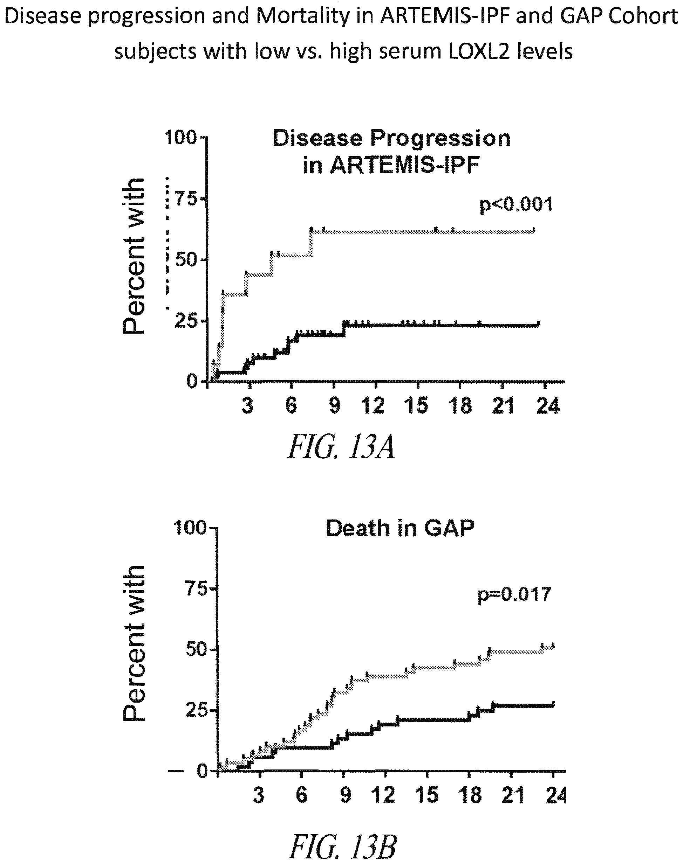

In ARTEMIS-IPF, high sLOXL2 (>800pg/mL) was associated with higher risk for DP (HR 5.4, 95% confidence interval [CI] 1.7-17.7, p=0.005). See Figure 13A. This effect was mainly driven by lung function decline (HR 7.6, 95% CI 1.2-48.3, p=0.031) and RHs (HR 5.4, 95% CI 1.2-24.0, p=0.029), with a trend toward higher risk for death (HR 1.9, 95% CI 0.3-12.4, p=0.517).

Among GAP patients with baseline spirometric data (n=70), high sLOXL2 levels (>700pg/ml) were associated with more DP events at 24-months after enrollment (HR 1.8, 95% CI 1.0-3.1, p=0.045). When all GAP patients were included, high sLOXL2 levels were associated with higher risk for mortality at 24-months after enrollment (HR 2.2, 95% CI 1.1-4.2, p=0.019). See Figure 13B. These results demonstrate that serum LOXL2 levels can be used as a prognostic biomarker for IPF disease outcome.

### Example 6

### Serum LOXL2 Levels in Patients with Chronic Hepatitis B (CHB)

### A. sLOXL2 Levels in CHB Subjects

Serum LOXL2 levels were assessed in subjects with chronic hepatitis B (CHB) and liver fibrosis, both before treatment and after 240 weeks of treatment with 300 mg tenofovir disoproxil fumarate (TDF). Liver biopsies were taken from 348 human subjects with CHB, prior to treatment and after 240 weeks of treatment with TDF. The biopsies were scored by pathologists using the Ishak scale for assessment of fibrosis. In the study, 96.3% of the subjects exhibited improvement in, or no progression of, liver fibrosis. Of the 96 subjects who began the study with biopsy-proven cirrhosis, 74% had regression of cirrhosis after 240 weeks of treatment.

Serum LOXL2 levels were retrospectively assessed by ELISA at baseline and at week 240 for 81 of the 348 subjects, including several subjects exhibited an improvement in fibrosis score. At week 240 following treatment, 42 of these 81 subjects had cirrhosis regression, 16 had persistent cirrhosis, 2 had progressed to cirrhosis over the course of treatment, 18 were non-cirrhotic subjects with no change in fibrosis, and 3 were non-cirrhotic subjects with at least a 2-point reduction in fibrosis as measured by Ishak.

Baseline serum LOXL2 levels were elevated in 91% of the 81 CHB subjects and in 97% of cirrhotic subjects. As shown below, the patients with cirrhosis (Ishak score 5 or 6) had elevated median LOXL2 serum levels at baseline compared to the patients with less severe liver fibrosis. This observation is similar to the LOXL2 serum levels observed in patients with chronic Hepatitis C infection. Moreover, the histology study showed that LOXL2 protein was concentrated at the sites of active fibrogenesis (data not shown). These results suggest that the patients with cirrhosis are still undergoing active fibrogenesis in the liver. Also, over the course of 240 weeks of treatment, 72% of the 60 patients with baseline cirrhosis showed a regression or improvement of their Ishak fibrosis score. Also, these patients had a lower median serum LOXL2 level at week 240 compared to baseline. The results suggest that both overall fibrosis and fibrogenesis were reduced by anti-viral treatment.

Figure 14A shows that serum LOXL2 levels (pg/mL) correlated with fibrosis score and Figures 14B and 14C show that serum baseline LOXL2 levels (pg/mL) correlated with baseline Ishak fibrosis score. At 240 weeks after treatment, mean serum LOXL2 levels had been reduced and no longer correlated with Ishak fibrosis score. See also Table 12.

**Table 12 Mean Serum LOXL2 levels compared to Ishak Stage at baseline and week 240 after initiation of treatment**

| | **N** | **Baseline** | **N** | **Week 240** |
|---|---|---|---|---|
| All subjects (mean LOXL2 (pg/mL)) | 81 | 2678.6 | 81 | 748.9 |
| Ishak Stage 0-3 (mean LOXL2 (pg/mL)) | 18 | 510.2 | 56 | 746.8 |
| Ishak Stage 4-6 (mean LOXL2 (pg/mL)) | 63 | 3298.2 | 25 | 753.5 |

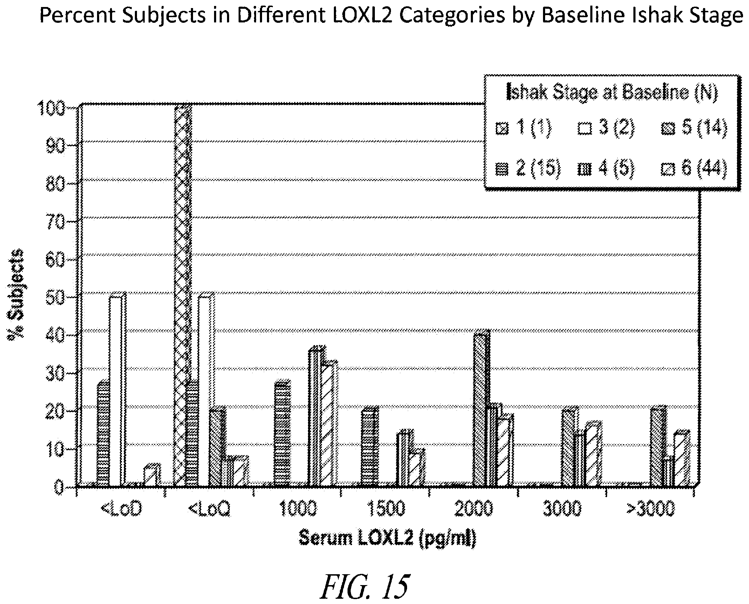

As shown in Figure 15, all subjects having a baseline Ishak stage between 1 and 3 had a serum LOXL2 level below 1500 pg/mL and 49 % of subjects with a baseline Ishak stage between 4 and 6 had serum LOXL2 levels above 1500 pg/mL.

79% of the 81 subjects experienced a reduction in serum LOXL2 levels. The 11% of subjects (each with a baseline level below the limit of quantitation) had no change in LOXL2 levels.

Figure 16 show baseline and week-240 serum LOXL2 levels (pg/mL) for individual subjects in the following groups: subjects with persistent cirrhosis at week 240 (n=16, Figure 16A); subjects with reversal of cirrhosis by week 240 (n=42, Figure 16B); non-cirrhotic subjects that did not experience a change in fibrotic stage (Ishak) by week 240 (n=18, Figure 16C); subjects that experienced a progression to cirrhosis over the course of the study (Figure 16D); and non-cirrhotic subjects with greater than or equal to 2-stage reduction in fibrosis by week 240 (Figure 16E).

Table 13 compares baseline and week 240 serum LOXL2 levels (pg/mL) in subjects with persistent cirrhosis at week 240, subjects with reversed cirrhosis at week 240, and non-cirrhotic subjects that experienced no change in fibrotic change over the course of the study ("Non-Cirrhotic No Δ").

**Table 13 Change in serum LOXL2 levels in different CHB subject groups**

| | **Persistent Cirrhosis** | | **Reversed Cirrhosis** | | **Non-Cirrhotic No Δ** | |
|---|---|---|---|---|---|---|
| | **(n=16)** | | **(n=42)** | | **(n=18)** | |
| | Baseline | Wk 240 | Baseline | Wk 240 | Baseline | Wk 240 |
| Mean | 9124.1 | 603.8 | 1355.0 | 922.6 | 798.4 | 436.8 |
| Median | 1863 | LOQ | 1073 | < LOQ | < LOQ | < LOQ |
| < LoQ | 2 (13%) | 8 (50%) | 4 (10%) | 29 (69%) | 10 (56%) | 14 (78%) |
| < LoD | 1 (6%) | 2 (13%) | 1 (2%) | 13 (31%) | 4 (22%) | 8 (44%) |
| < 1000 | 5 (31%) | 14 (88%) | 20 (48%) | 35 (83%) | 13 (72%) | 15 (83%) |
| > 3000 | 5 (31%) | 0 (0%) | 2 (5%) | 3 (7%) | 1 (6%) | 0 (0%) |
| Decrease | | 14 (88%) | | 37 (88%) | | 9 (50%) |
| Increase | | 0 (0%) | | 5(12%) | | 2 (11%) |

As shown in Table 13, 88% of cirrhotic subjects had a reduction in LOXL2 levels. Additionally, baseline serum LOXL2 levels were determined to be the highest in those subjects who at week 240 had persistent cirrhosis.

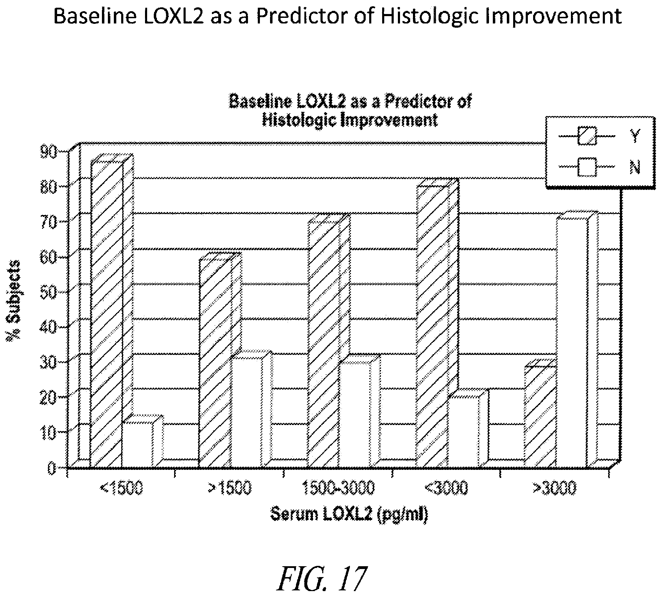

Figure 17 shows the percentage of cirrhotic subjects determined to have a histological improvement at week 240 ("Y") having given baseline serum LOXL2 levels (<1500, >1500, 1500-3000, <3000, and >3000 pg/mL) and the percentage of cirrhotic subjects determined not to have histological improvement at week 240 ("N") having the same given baseline serum LOXL2 levels. As shown, cirrhotic subjects having a baseline serum LOXL2 level less than 1500 pg/mL had an 88% chance of regression. Cirrhotic subjects having a baseline serum LOXL2 level between 1500 pg/mL and 3000 pg/mL had a 70% chance of regression, while cirrhotic subjects having a baseline serum level above 3000 pg/mL had only a 29% chance of regression. Thus, among cirrhotic patients, baseline serum LOXL2 levels below 1500 pg/mL were associated with an 88% likelihood of regression, while baseline serum LOXL2 levels above 3000pg/mL were associated with a 29% likelihood of regression.

Baseline serum LOXL2 levels correlated more with week 240 Ishak fibrosis stage than with Baseline fibrosis stage. This suggests high serum LOXL2 levels reflected active fibrogenesis.

The results of this study demonstrated that serum LOXL2 levels were elevated in patients with CHB and were highest in those with the most fibrosis, demonstrating a general correlation between serum LOXL2 and fibrosis score. Serum LOXL2 levels reflected active disease and active fibrogenesis (for example, given that higher baseline levels were associated with higher fibrosis stages at week 240). Treating the underlying CHB resulted in a decline in LOXL2 in most patients, suggesting downregulation of fibrogenesis. There was a decrease in serum LOXL2 after 5 years even in patients with unchanged fibrosis scores that were clinically doing well. The results demonstrate serum LOXL2 level as a marker of active disease and that high LOXL2 is predictive of lack of regression.

### B. sLOXL2 Levels in CHB Subjects

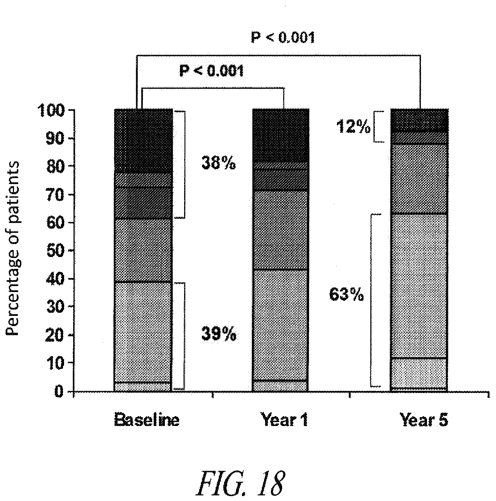

641 CHB-infected subjects were treated with 300 mg tenofovir disoproxil fumarate (TDF) / adefovir for one year and continued on open label TDF for up to five years. From 344 of these subjects, liver biopsies were performed at baseline (before treatment) and at week 48 and week 240 after initiation of treatment. The biopsies were scored by pathologists using the Ishak scale for assessment of fibrosis. A total of 96 subjects (28 %) had cirrhosis (Ishak score ≥ 5) at baseline. 74 % no longer had histologic cirrhosis (Ishak score < 5) after 240 weeks of treatment. See Figure 18.

### 1. sLOXL2 levels in 88 compensated CHB subjects

Serum LOXL2 levels were retrospectively assessed by ELISA for 88 of the 348 subjects, with preference given for advanced fibrotics/cirrhotics. The 88 subjects were 70 % (80) male, 64 % (73) Caucasian, 14 % (16) of Asian descent, 8 % (9) African American/black, were At week 240, 42 of these 88 subjects had cirrhosis regression, 22 had persistent cirrhosis, 3 had progressed to cirrhosis over the course of treatment, 18 were non-cirrhotic subjects with no change in fibrosis (on-study progression), and 3 were non-cirrhotic subjects with at least a 2-point reduction in fibrosis as measured by Ishak.

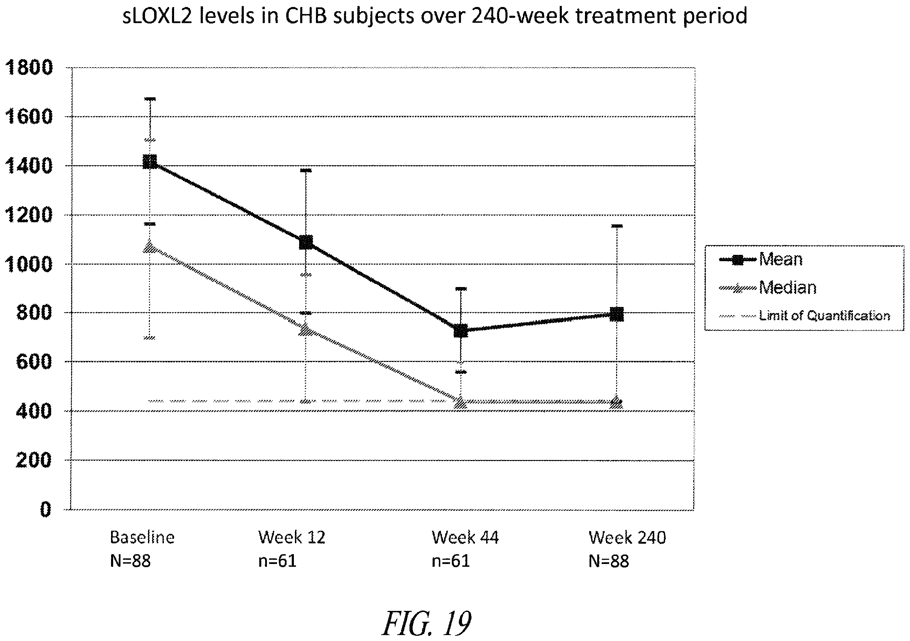

Serum LOXL2 (sLOXL2) was detectable at baseline in 92 % of subjects overall and in 97 % of cirrhotic subjects. Viral suppression resulted in a decrease in sLOXL2 levels. See Figure 19.

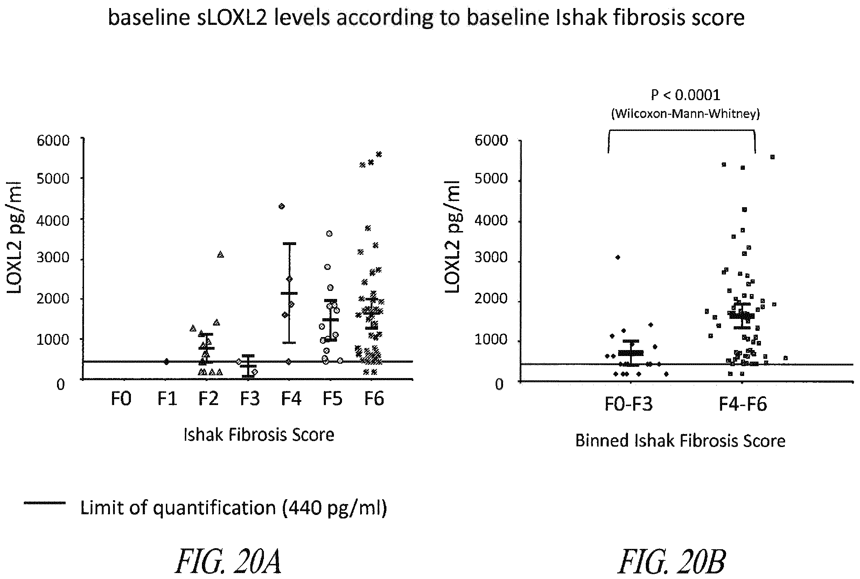

Baseline sLOXL2 levels were higher in subjects with more advanced fibrosis. See Figure 20. Mean LOXL2 levels in subjects with binned Ishak Fibrosis Scores of F0-F3 and F4-F6 were 698pg/mL and 1,629 pg/mL respectively (P < 0.0001Wilcoxon-Mann-Whitney). These results demonstrate that sLOXL2 levels can be used to monitor stage of disease. In this study, change in sLOXL2 was not predictive of change in fibrosis stage over 240 weeks. Baseline sLOXL2 levels were higher in those subjects with persistent cirrhosis (1,999 pg/mL) as compared with those who exhibited regression of cirrhosis (1,334 pg/mL), p=0.13.

### 2. sLOXL2 Levels in 81 decompensated CHB Subjects

A second set of samples was taken from 81 CHB subjects with decompensated liver disease. Table 14 presents demographic information for these 81 subjects in comparison with the 88 subjects discussed above.

**Table 14 Demographic information**

| **Study Population** | **Compensated** | **Decompensated** |
|---|---|---|
| N | 88 | 81 |
| Males (%) | 70 (80) | 69 (85) |
| Mean Age (years) | 44 | 51 |
| Caucasian (%) | 64 (73) | 38 (47) |
| Asian (%) | 14 (16) | 39 (48) |
| African American / Black (%) | 8 (9) | 2 (2) |
| Mean BMI | 26.5 | 27.0 |
| HBeAg Pos (%) | 36 (41) | 29 (36) |
| Cirrhotic at Baseline (%) | 64 (73) | 81 (100) |
| Cirrhotic at Week 240 (%) | 25 (28) | NA |

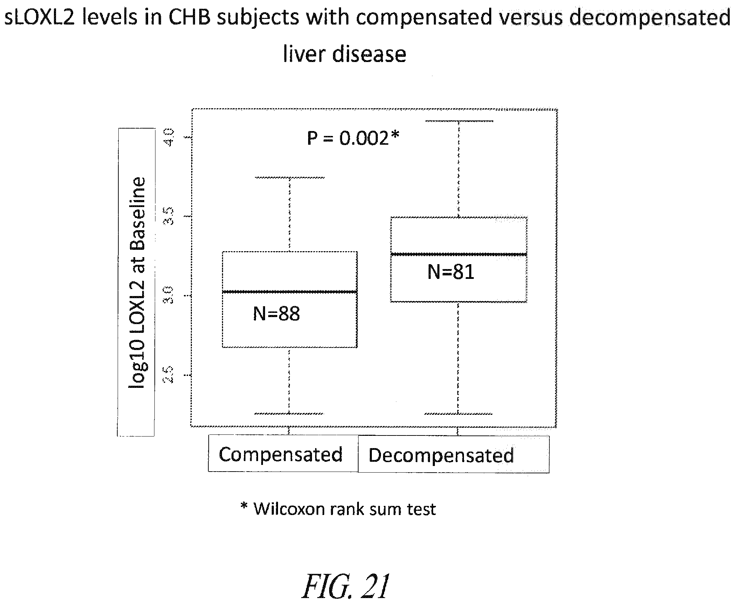

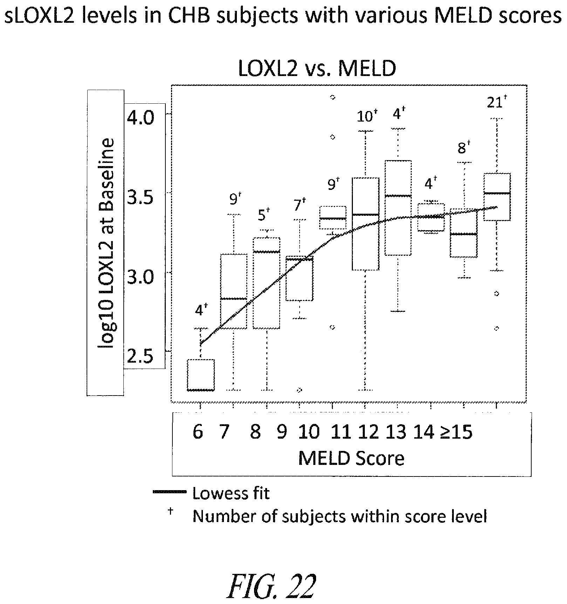

Mean serum levels were higher (2,396 pg/mL) for the 81 decompensated subjects compared to the 88 compensated subjects (1,418 pg/mL), p=0.002 (Wilcoxon rank sum test). Figure 21. Additionally, among the 81 subjects with decompensated disease, mean sLOXL2 levels were higher for those having higher Model for End-stage Liver Disease (MELD) scores. Figure 22. Table 15, below, lists mean sLOXL2 levels for CHB subjects with various degrees of disease severity, including varying degrees of compensated disease and decompensated disease.

**Table 15 Mean sLOXL2 by Disease Severity**

| | Compensated | | | Decompensated Cirrhosis |
|---|---|---|---|---|
| **Disease Severity** | Ishak stage 0-3 | Baseline cirrhosis with regression by wk 240 | Cirhosis at Baseline and at wk 240 | |
| **Mean sLOXL2 (pg/mL)** | 698 | 1,334 | 1,999 | 2,396 |

The results demonstrate that sLOXL2 levels were higher in patients with more severe disease and/or more advanced fibrosis, demonstrating use of sLOXL2 detection as a prognostic and diagnostic marker for CHB.

### C. Precent Collagen Area (PCA)

The PCA was determined from tissue samples from entire study population taken at baseline, week 48, and week 240. The mean PCA was 7.1% at baseline, 5.3% at week 48 and 3.9% at week 240 for the entire population (Figure 34A).

The PCA was determined in subjects with persistent cirrhotic and subjects that showed histologic regression of cirrhosis. The mean PCA was higher in subjects with persistent cirrhosis than in subjects with histologic regression. The regressor groups also had a proportionally greater reduction in PCA over time (Figure 34B).

### Example 7

### LOXL2 Levels in Primary Sclerosing Cholangitis (PSC), Primary Biliary Cirrhosis (PBC), and Animal Models of Cholestatic Liver Disease

LOXL2 expression was assessed in samples from human primary sclerosing cholangitis (PSC) subjects and primary biliary cirrhosis (PBC) subjects and in mouse models of cholestatic liver disease.

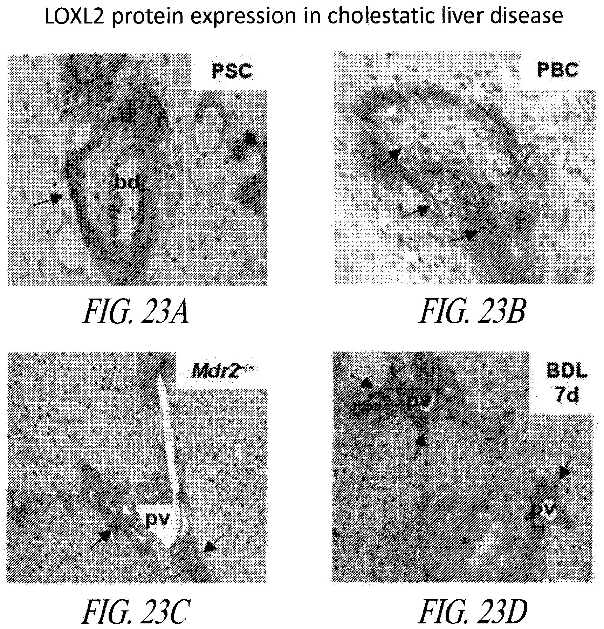

Liver explants from patients with PSC, patients with PBC, and biopsies from non-diseased liver tissue, were evaluated by immunohistochemistry (IHC) and immunofluorescence (IF). LOXL2 protein was detected as abundantly expressed in diseased regions of human PSC (Figure 23A) and PBC (Figure 23B) livers.

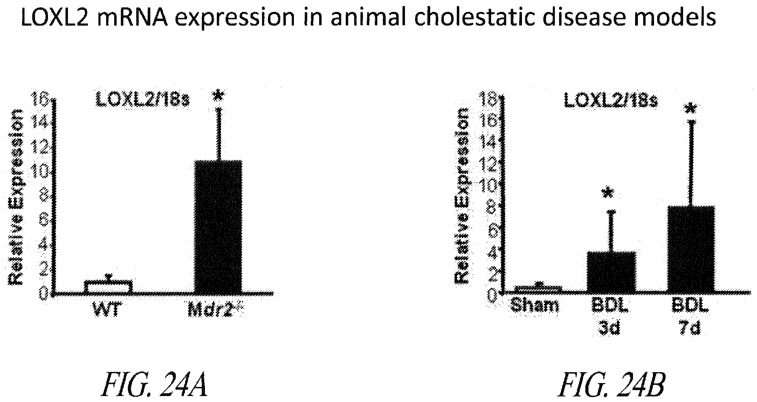

IHC, IF, and qRT-PCR were performed on liver tissue from *Mdr2*^{*-*/*-*} and bile duct-ligated (BDL) mice, at various stages of disease progression, using wild-type (WT) mice and mice subject to sham surgery as controls. LOXL2 protein was induced in both *Mdr2*^{*-*/*-*} mice (Figure 23C) and BDL mice (Figure 23D, 7-days post-surgery) relative to non-diseased controls. Likewise, mRNA was induced in *Mdr2*^{*-*/*-*} mice (Figure 24A) and in BDL mice days 3 and 7 post-surgery(Figure 24B). In both human and animal model tissues , LOXL2 was present in regions of fibrosis, co-localizing with tracts of fibrillar collagen deposition in clear association with onion skin-type fibrosis and fibrotic septa. LOXL2 expression was also observed in non-fibrotic regions of ductular proliferation as well as in endothelial cells of portal vessels.

A mouse model of PSC with increased progressive fibrosis and early-onset portal hypertension was generated by backcrossing the Mdr2 mutation on a fibrosis susceptible background (BALB/c). The Mdr2^{-/-} mice showed increased progression of liver fibrosis with signs of bridging fibrosis, spontaneously developed periductular onion-skin type fibrotic lesions and pronounced ductular reaction, and high levels of collagen deposition in the liver (Figure x A - use 33C). Mdr2^{-/-} BALB/c also showed an early onset of severe portal hypertension compared to control mice (data not shown). Immunohistochemical analyses showed that LOXL2 was absent from healthy liver but was induced in periductular fibrotic areas in Mdr2^{-/-} BALB/c mice (data not shown). The 4-week-old Mdr2^{-/-} BALB/c mice were administered with AB0023 (30mg/kg), M64 (30mg/kg), or BAPN (100 mg/kg) twice a week (n = 10 per group). After 4 weeks of treatment, the mice treated with AB0023 showed reduced hepatic collagen deposition: about 31 % and 34 % reduction compared to the mice treated with M64 (*p* = *0.0032*) and BAPN *(p = 0.0012),* respectively. Also, the mice treated with AB0023 had reduced expression of profibrogenic genes such as procollagen, TGFb1, TGFb2, and MMP-2 (data not shown).

The mouse model of biliary fibrosis was induced in C57B16 mice by 3,5-diethoxycarbonyl-1,4-dihydrocollidine (DDC) feeding for 4 weeks. DDC feeding caused cholangitis with a pronounced ductular reaction and onion-skin type periductal fibrosis. The mice were treated with AB0023 (30 mg/kg), M64 (30 mg/kg), or BAPN (100 mg/kg) by intraperitoneal (i.p.) injection twice a week for 4 weeks. The DDC-fed mice administered with AB0023 showed reduced hepatic collagen content by 39 % (*p* = *0.0151*) compared to vehicle treatment. Also, the immunohistochemical analysis showed that the mice treated with AB0023 exhibited less collagen fibrils in the periductal areas compared to the mice treated with vehicle and M64 treatment (data not shown).

Protein levels of LOXL2 and other candidate disease markers were measured by ELISA in plasma drawn from an independent set of PSC patients and healthy controls. LOXL2 protein was detected in plasma from most PSC patients and was not detected in most healthy donors, with a p value less than 0.005, Fisher's exact test.

The results demonstrate induction of LOXL2 expression in PSC and PBC liver explants and significant increase in plasma LOXL2 levels in PSC patients. The results implicate a functional role for LOXL2 in pathogenesis of cholestatic disease and confirm it as a cholestatic disease diagnostic marker. The results further demonstrate a similar induction and pattern of LOXL2 expression in livers of *Mdr2*^{*-*/*-*} and BDL mice as compared to livers from human subjects with PSC and PBC. In some embodiments, such animal models are used to assess efficacy of treatments (e.g., anti-LOXL2 treatments) for cholestatic liver diseases such as PSC and PBC.

### Example 8

### Detection of LOXL2 in HCV and Other Liver Diseases

### A. Serum LOXL2 measurement for estimation of liver fibrosis in patients with chronic hepatitis C virus (HCV) infection

Analysis of fibrotic liver tissues by immunohistochemistry (IHC) revealed localized LOXL2 expression at the fibrogenic interface composed of fibroblasts, neovasculature, inflammatory cells and hepatocytes, suggesting that LOXL2 is associated with active fibrogenic disease. To further explore the relationship of serum LOXL2 with fibrotic liver disease, a LOXL2-specific ELISA as described in Example 3 was used. Serum samples, along with liver biopsies, were collected from 87 patients with chronic HCV infection. Serum levels of LOXL2 and of the established biomarkers hyaluronic acid (HA) and tissue inhibitor of metalloproteinases-1 (TIMP1) were measured by immunoassay, and the histological stage of liver fibrosis was assessed for each biopsy using the Ishak scoring system. Separately, serum samples from over 30 healthy donors were also collected and assessed for serum LOXL2 levels. The correlation between the serum biomarkers and the fibrosis scores was studied using ANOVA test, as well as the Mann-Whitney U test for samples binned by fibrosis score.

### RESULTS

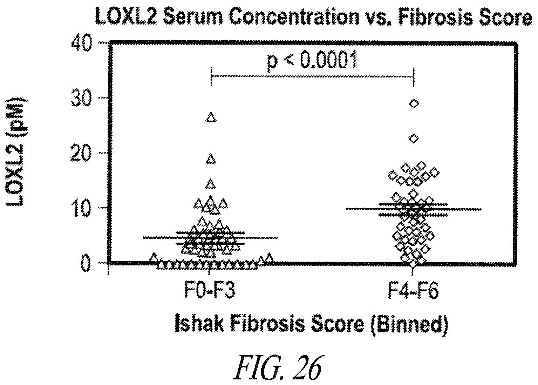

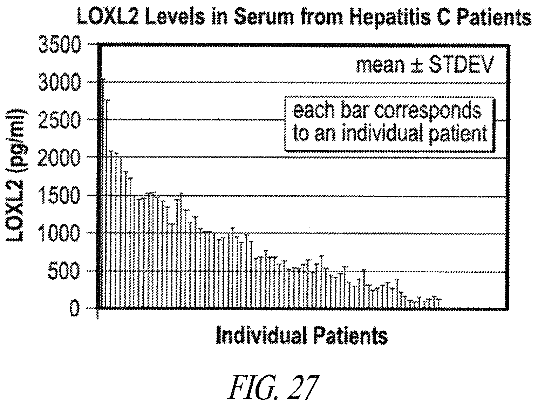

The results are shown in Figures 26 and 27. LOXL2 protein was detected in the serum of 83% of patients with chronic HCV infection, but was not detected in serum from any normal healthy donors. There was a positive correlation between serum levels of HA, TIMP1, and LOXL2 and stage of fibrosis. The serum results were consistent with the IHC analysis, which revealed high levels of LOXL2 protein in areas of active fibrosis, compared to low or undetectable levels in samples from non-infected or healthy individuals.

### B. LOXL2 expression in liver tissue from patients with chronic HCV infection, nonalcoholic steatohepatitis (NASH)1, and alcoholic steatohepatitis (ASH)

Immunohistochemical (IHC) staining demonstrated LOXL2 expression in liver tissues from a patient having chronic HCV infection. Snap-frozen human tissue samples were obtained from Cureline (Burlingame, CA) and Asterand (Detroit, MI) and serial sections were stained with anti-LOXL2.

### RESULTS

Results from sections obtained from a patient with chronic HCV infection are shown in Figure 28, showing LOXL2 protein expression in the liver tissue of this patient. In the left panel of Figure 28 (5x objective magnification), black arrows indicate areas of fibrous expansion into portal regions and tracts. White arrows indicate areas of short fibrous septa surrounding hepatic lobules. The right panel of Figure 28 (40x objective magnification) shows LOXL2 immunoreactivity, observed in the fibrous septa (S) at the interface with hepatocytes (H), within the perisinusoidal space (arrows), and in the myofibroblasts within the liver parenchyma (arrows). The results show that in this study, LOXL2 was expressed in liver tissues of patients with chronic HCV infection, and that the expression is measurable by embodiments of the provided assays. In another IHC study, a strong localization of LOXL2 expression in liver tissue at the active disease interface in NASH, HCV-associated fibrosis, and ASH, but not in healthy liver (data not shown).

### C. Increased serum LOXL2 levels in subjects with liver cirrhosis as compared to those with mild to moderate liver fibrosis

Patient serum samples were collected from twenty-six adults with chronic hepatitis C infection enrolled in the placebo arm of a clinical trial. Subjects were grouped by Ishak fibrosis scores (1-3: mild to moderate fibrosis; 5-6: cirrhosis). Demographic characteristics of the subjects are shown in Table 16.

**Table 16 Demographic characteristics of HCV subjects**

| **Characteristic** | | **Ishak Score 1-3 (n=14)** | | **Ishak Score 5-6 (n=12)** | | **All (n=26)** | |
|---|---|---|---|---|---|---|---|
| Age* | | 53 (50.5, 56.0) | | 55 (47.8, 55.0) | | 53.5 (49.3, 55.8) | |
| Sex Male Female | | 9 (64.3%) 5 (35.7%) | | 9 (75.0%) 3 (25.0%) | | 18 (69.2%) 8(30.8%) | |
| Race White Black | | 11 (78.6%) 3 (21.4%) | | 10 (83.3%) 2 (16.7%) | | 21 (80.8%) 5 (19.2%) | |
| Baseline Ishak Fibrosis Score (n) | | | | | | | |
| F1 | F2 | | F3 | F4 | F5 | | F6 |
| 3 | 6 | | 5 | 0 | 7 | | 5 |

| | | | | | | | |
|---|---|---|---|---|---|---|---|
| *Median and inter-quartile range (25%,75%) reported | | | | | | | |

Serum samples were taken at six time points, relative to the study baseline: weeks 4, 8, 16, 24, 26, and 30. Paired liver biopsies (screening and week 24) were evaluated by a central pathologist in a blinded fashion. *See* Manns M, Palmer R, Flisiak E, et al., J Hepatology. 2011, 54 Supplement 1: S55-S56. Serum LOXL2 was measured using the LOXL2 immunoassay described in Example 3 (sandwich immunoassay developed on the MesoScale Discovery platform).

For statistical analysis, subjects were grouped by Ishak fibrosis scores (1-3: mild to moderate fibrosis; 5-6: cirrhosis). No subject in the study was observed to have a baseline Ishak fibrosis score of 4. Serum samples with detectable LOXL2 below the assay lower limit of quantitation (LLOQ) were set to the LLOQ. Differences in biomarkers levels were descriptively and graphically summarized. 95% confidence intervals (CI) were constructed through 10,000 bootstraps of the median using sampling with replacement with the observed sample sizes per group. P-values were calculated using Wilcoxon rank sum tests when comparing groups within a time point and by a repeated measures linear model with a within-subject random effect when comparing groups across all time points.

### RESULTS

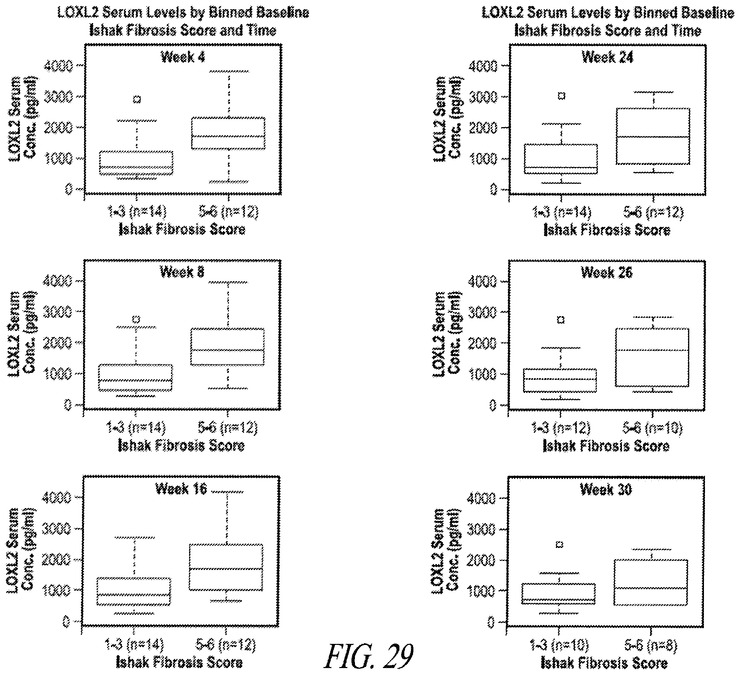

Figure 29 shows LOXL2 serum levels by binned baseline Ishak fibrosis score and time. Each panel shows, for the indicated time point (weeks 4, 8, 16, 24, 26, 30), LOXL2 concentration (pg/mL) for two groups of patients, grouped according to Ishak Fibrosis Score (1-3 and 5-6, respectively). Three outliers (LOXL2 concentration = 5529, 6621, 8845 pg/ml), with LOXL2 concentration out of plot ranges all were from the same subject, having an Ishak fibrosis score of 5.

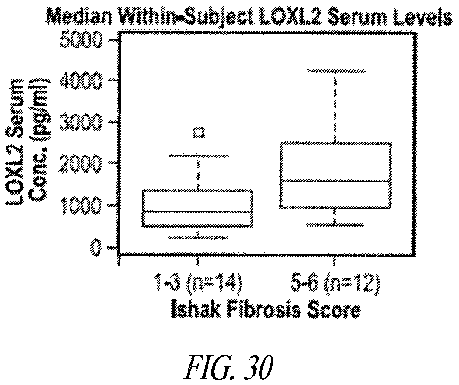

Figure 30 shows the median within-subject LOXL2 serum levels, calculated as median LOXL2 serum concentration over weeks 4-30, for the two groups of patients, grouped according to Ishak Fibrosis Score (1-3 and 5-6, respectively). The average within-subject coefficient of variation was 22 %.

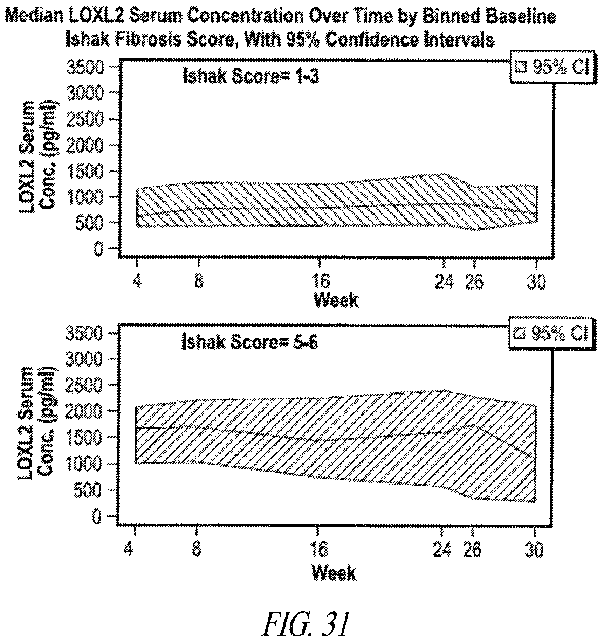

Figure 31 shows median LOXL2 serum concentration (pg/mL) over time (weeks), by binned baseline Ishak fibrosis score, with 95% confidence intervals. Only one subject had a change greater than or equal to 2 in Ishak fibrosis score over the 25-28 weeks between study biopsies.

Table 17 shows the median LOXL2 concentration (pg/mL) for each time-point, with p-values showing statistical significance of the increase in subjects with liver cirrhosis compared to those with mild to moderate liver fibrosis.

**Table 17 Statistical significance of LOXL2 serum levels according to binned fibrosis score**

| **Time point** | **Median LOXL2 conc. (pg/ml)** | | **P-value** |
|---|---|---|---|
| | **Ishak F1-F3** | **Ishak F5-F6** | |
| Week 4 | 641 | 1684 | 0.0149 |
| Week 8 | 786 | 1700 | 0.0091 |
| Week 16 | 814 | 1457 | 0.0407 |
| Week 24 | 881 | 1616 | 0.0596 |
| Week 26 | 865 | 1763 | 0.0716 |
| Week 30 | 711 | 1118 | 0.5890 |
| Overall | 810 | 1591 | **0.0275** |

These results confirm the ability of an embodiment of provided immunoassays to measure serum concentrations of LOXL2 protein. The results also demonstrate that in this study, serum LOXL2 protein levels were significantly increased in subjects with liver cirrhosis as compared with those with mild to moderate liver fibrosis, and that the increase is measurable in serum using embodiments of the provided assays.

### D. Serum LOXL2 levels correlated with serum hyaluronic acid TIMP1 levels in subjects with chronic HCV infection

The immunoassay and statistical analysis was carried out as in part C, above. Additionally, hyaluronic acid (HA) and TIMP1 were measured using commercial immunoassay kits. The association between the biomarkers (LOXL2 and HA or TIMP1) was assessed using Spearman rank correlation.

### RESULTS

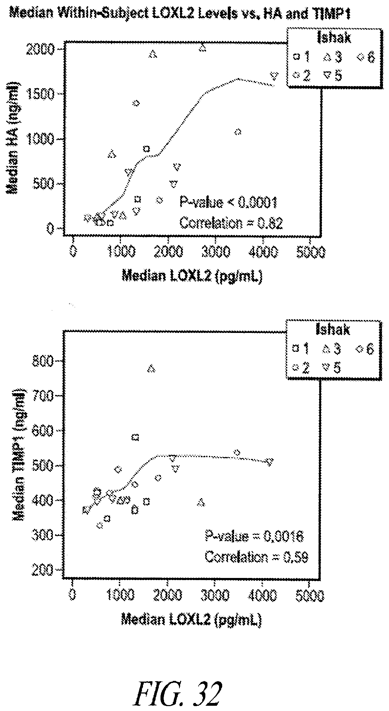

Figure 32 shows median within-subject levels of LOXL2 vs. levels of Hyaluronic acid (HA) (top panel) and tissue inhibitor of metalloproteinases-1 (TIMP1) (bottom panel), for subjects having the indicated Ishak scores (1-6). Median within-subject expression was calculated as median expression over weeks 4 through 30. The curve was constructed using locally weighted scatter plot smoothing.

These results demonstrate that in this study, serum LOXL2 levels were correlated with serum HA and TIMP1 levels, as measured using an embodiment of the provided immunoassay.

### Example 9

### Serum LOXL2 in Oncology Patients

Eight cancer patients being treated with anti-LOXL2 (AB0024) antibody for the cancer were studied. Patient identification ("Pt ID"); cancer diagnosis; dose level of anti-LOXL2 antibody; time to progression; and LOXL2 expression, as examined by immunohistochemistry in a sample (∼5 µm section) of fixed tissue isolated from the original primary tumor or related sample, are provided in Table 18, below.

**Table 18**

| **Pt ID** | **Diagnosis** | **Dose level (mpk)** | **Time to progression** | **LOXL2 expression** |
|---|---|---|---|---|
| 001 | Renal cell | 1 | 44 days | Vascular |
| 002 | Colorectal | 1 | Stable (∼7 months) | Positive desmoplastic |
| 003 | Endometrial mixed mullerian | 1 | 57 days | Minimal; not desmoplastic |
| 004 | Breast | 3 | 38 days | Minimal; patchy |
| 005 | Colorectal | 3 | 56 days | Positive desmoplastic |
| 006 | Melanoma | 3 | 42 days | positive |
| 007 | Colon SC | 10 | 57 days | |
| 008 | Prostate | 10 | 30 days | Positive desmoplastic |
| 009 | Ovarian/breast | 10 | 51 days | Weak, not desmoplastic |

Blood samples were obtained at Day 1 on which anti-LOXL2 treatment began (sample taken before anti-LOXL2 treatment); and on days 29 and 57 following the beginning of anti-LOXL2 treatment.

### RESULTS

LOXL2 was detected in plasma of 8 of 8 patients, and in serum samples of 5 of 8 patients, at all time points available. AB0024 administration did not clear or mask the LOXL2 signal.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto

The invention provides, in particular, the following:
1. A method, comprising: administering an agent that binds to and/or inhibits LOXL2 to a subject having a liver disease or condition, thereby treating or ameliorating the disease or condition.
2. The method of item 1, wherein the disease or condition is associated with fibrosis.
3. The method of item 1 or 2, wherein the liver disease or condition is selected from the group consisting of: NASH (nonalcoholic steatohepatitis), PSC (primary sclerosing cholangitis), cirrhosis, portal hypertension, PBC (primary biliary cirrhosis), autoimmune hepatitis, alcoholic cirrhosis, alpha 1 antitrypsin deficiency disease, hereditary hemochromatosis, Wilson's disease, hepatitis B virus (HBV), hepatitis C virus (HCV), and HIV associated steatohepatitis.
4. The method of any of items 1-3, wherein the agent is an antibody that specifically binds to LOXL2.
5. The method of item 4, wherein the antibody competes for binding to LOXL2 with an antibody having a heavy chain variable region sequence of SEQ ID NO: 8 and/or a light chain variable region sequence of SEQ ID NO: 9.
6. The method of item 4 or 5, wherein the antibody comprises a heavy chain variable region having an amino acid sequence with at least 75 % identity to a sequence set forth in SEQ ID NO: 6, 8, 10, 11, 12 and/or a light chain variable region having an amino acid sequence with at least 75 % identity to a sequence of SEQ ID NO: 7, 9, 13, or 14.
7. The method of any of items 4-6, wherein the antibody comprises a heavy chain CDR of the heavy chain variable region sequence set forth in SEQ ID NO: 8 and/or a light chain CDR of the light chain variable region sequence set forth in SEQ ID NO: 9.
8. The method of any of items 1-7, wherein the agent is administered at a dose of at or about or at least at or about 10 mg/kg or 20 mg/kg or between about 10-20 mg/kg.
9. The method of any of items 1-7, wherein the agent is administered at a dose of at least at or about or at or about 200 mg or 700 mg or between about 200-700 mg.
10. The method of any of items 1-7, wherein the agent is administered at a dose of at least at or about or at or about 75 mg or 125 mg or between at or about 75-125 mg.
11. The method of any of items 1-10, wherein the agent is administered intravenously or subcutaneously.
12. The method of any of items 1-11, wherein the method increases or prolongs survival of the subject, reduces or prevent an increase in bridging fibrosis, reduces or prevents an increase in alpha smooth muscle actin (αSMA) levels, reduces or prevents an increase in stellate cell activation, and/or reduces or prevents increase in alanine aminotransferase (ALT) or aspartate aminotransferase (AST), or gamma-glutamyltransferase (GGT).
13. The method of item 12, wherein the method reduces ALT, AST, GGT, or ALT/AST ratio to less than the upper limit or normal (ULN), or to less than 2x, 5x, or 10x the upper limit of normal (ULN).
14. A method for detecting, predicting, or monitoring a disease or condition, the method comprising:
   a) contacting a liquid sample obtained from an individual with an antibody specific for lysyl oxidase-like 2 (LOXL2); and
   b) detecting binding of the antibody with LOXL2 present in the liquid sample, thereby detecting a level of LOXL2 in the liquid sample,
   wherein the detected level of LOXL2 indicates the presence or absence of the disease or condition in the individual or the likelihood of a response to a treatment for the disease or condition by the individual.
15. The method of item 14, wherein the disease or condition is pulmonary fibrosis, liver fibrosis, kidney fibrosis, cardiac fibrosis, or myelofibrosis, cirrhosis, chronic viral hepatitis, hepatitis C virus (HCV), hepatitis B virus (HBV), or decompensated liver disease.
16. The method of item 14 or 15, wherein the disease or condition is idiopathic pulmonary fibrosis (IPF), NASH (nonalcoholic steatohepatitis), PSC (primary sclerosing cholangitis), cirrhosis, portal hypertension, PBC (primary biliary cirrhosis), autoimmune hepatitis, alcoholic cirrhosis, alpha 1 antitrypsin deficiency disease, hereditary hemochromatosis, Wilson's disease, hepatitis B virus (HBV), hepatitis C virus (HCV), and HIV associated steatohepatitis.
17. The method of any of items 14-16, wherein the detected level of LOXL2 is greater than about 700 pg/mL.
18. The method of item 14, wherein the detected level of LOXL2 is greater than about 800 pg/mL.
19. The method of any of items 14-18, further comprising determining that the detected level of LOXL2 is greater than a threshold level of LOXL2, thereby determining a likelihood of outcome, endpoint, or event of the disease or condition in the individual.
20. The method of item 14, wherein the antibody comprises a heavy chain variable region having an amino acid sequence with at least 75 % identity to a sequence set forth in SEQ ID NO: 6, 8, 10, 11, 12 and/or a light chain variable region having an amino acid sequence with at least 75 % identity to a sequence of SEQ ID NO: 7, 9, 13, or 14.

## Claims

1. A method for detecting, predicting, or monitoring a disease or condition, the method comprising:
a) contacting a liquid sample obtained from an individual with an antibody specific for lysyl oxidase-like 2 (LOXL2); and
b) detecting binding of the antibody with LOXL2 present in the liquid sample, thereby detecting a level of LOXL2 in the liquid sample,
wherein the detected level of LOXL2 indicates the presence or absence of the disease or condition in the individual or the likelihood of a response to a treatment for the disease or condition by the individual, and
wherein the antibody comprises
(i) a heavy chain having the amino acid sequence set forth in SEQ ID NO: 24 or 26 and a light chain having the amino acid sequence set forth in SEQ ID NO: 20 or 22,
(ii) a heavy chain having the amino acid sequence set forth in SEQ ID NO: 24 and a light chain having the amino acid sequence set forth in SEQ ID NO: 20,
(iii) a heavy chain having the amino acid sequence set forth in SEQ ID NO: 26 and a light chain having the amino acid sequence set forth in SEQ ID NO: 22, or
(iv) the amino acid sequence set forth in SEQ ID NO: 28 or 29.

2. The method of claim 1, wherein the disease or condition is fibrosis or cancer.

3. The method of claim 1 or 2, wherein the disease or condition is pulmonary fibrosis, liver fibrosis, kidney fibrosis, cardiac fibrosis, myelofibrosis, cirrhosis, chronic viral hepatitis, hepatitis C virus (HCV) infection, hepatitis B virus (HBV) infection, or decompensated liver disease.

4. The method of any one of claims 1 to 3, wherein the disease or condition is idiopathic pulmonary fibrosis (IPF), NASH (nonalcoholic steatohepatitis), PSC (primary sclerosing cholangitis), portal hypertension, PBC (primary biliary cirrhosis), autoimmune hepatitis, alcoholic cirrhosis, alpha 1 antitrypsin deficiency disease, hereditary hemochromatosis, Wilson's disease, and HIV associated steatohepatitis.

5. A method for detecting, predicting, or monitoring a disease or condition, the method comprising:
a) contacting a liquid sample obtained from an individual with an antibody specific for lysyl oxidase-like 2 (LOXL2); and
b) detecting binding of the antibody with LOXL2 present in the liquid sample, thereby detecting a level of LOXL2 in the liquid sample,
wherein the detected level of LOXL2 indicates the presence or absence of the disease or condition in the individual or the likelihood of a response to a treatment for the disease or condition by the individual, and
wherein the disease or condition is decompensated liver disease, such as liver disease associated with ascites, esophageal varices, encephalopathy, and/or jaundice, silicosis, asbestosis, keloids, scleroderma, portal hypertension, alcoholic cirrhosis, alpha 1 antitrypsin deficiency disease, hereditary hemochromatosis, or HIV associated steatohepatitis.

6. The method of claim 5, wherein the antibody comprises a heavy chain variable region having an amino acid sequence with at least 75% identity to a sequence set forth in SEQ ID NO: 6, 8, 10, 11, 12 and/or a light chain variable region having an amino acid sequence with at least 75% identity to a sequence set forth in SEQ ID NO: 7, 9, 13, 14.

7. The method of claim 5, wherein the antibody comprises
a heavy chain having an amino acid sequence with at least 75% identity to a sequence set forth in SEQ ID NO: 24 or 26 and/or a light chain having an amino acid sequence with at least 75% identity to a sequence set forth in SEQ ID NO: 20 or 22, or
a heavy chain having the amino acid sequence set forth in SEQ ID NO: 24 or 26 and a light chain having the amino acid sequence set forth in SEQ ID NO: 20 or 22, or
a heavy chain having the amino acid sequence set forth in SEQ ID NO: 24 and a light chain having the amino acid sequence set forth in SEQ ID NO: 20, or
a heavy chain having the amino acid sequence set forth in SEQ ID NO: 26 and a light chain having the amino acid sequence set forth in SEQ ID NO: 22.

8. The method of claim 5, wherein the antibody comprises
an amino acid sequence with at least 75% identity to a sequence set forth in SEQ ID NO: 28 or 29, or
the amino acid sequence set forth in SEQ ID NO: 28 or 29.

9. The method of any one of the preceding claims, wherein the detected level of LOXL2 is greater than about 700 pg/mL or wherein the detected level of LOXL2 is greater than about 800 pg/mL.

10. The method of any one of the preceding claims, further comprising determining that the detected level of LOXL2 is greater than a threshold level of LOXL2, thereby determining a likelihood of outcome, endpoint, or event of the disease or condition in the individual.

11. An agent that binds to and/or inhibits LOXL2 for use in a method of treating or ameliorating a liver disease or condition, said method comprising administering the agent to a subject having a liver disease or condition, optionally wherein the agent is administered intravenously or subcutaneously.

12. The agent for use of claim 11, wherein the liver disease or condition is associated with fibrosis or wherein the liver disease or condition is selected from the group consisting of: NASH (nonalcoholic steatohepatitis), PSC (primary sclerosing cholangitis), cirrhosis, portal hypertension, PBC (primary biliary cirrhosis), autoimmune hepatitis, alcoholic cirrhosis, alpha 1 antitrypsin deficiency disease, hereditary hemochromatosis, Wilson's disease, hepatitis B virus (HBV) infection, hepatitis C virus (HCV) infection, and HIV associated steatohepatitis.

13. The agent for use of claim 11 or 12, wherein the agent is an antibody that specifically binds to LOXL2.

14. The agent for use of claim 13, wherein the antibody comprises
a heavy chain variable region having an amino acid sequence with at least 75% identity to a sequence set forth in SEQ ID NO: 6, 8, 10, 11, 12 and/or a light chain variable region having an amino acid sequence with at least 75% identity to a sequence set forth in SEQ ID NO: 7, 9, 13, 14, or
a heavy chain having an amino acid sequence with at least 75% identity to a sequence set forth in SEQ ID NO: 24 or 26 and/or a light chain having an amino acid sequence with at least 75% identity to a sequence set forth in SEQ ID NO: 20 or 22, or
a heavy chain having the amino acid sequence set forth in SEQ ID NO: 24 or 26 and a light chain having the amino acid sequence set forth in SEQ ID NO: 20 or 22, or
a heavy chain having the amino acid sequence set forth in SEQ ID NO: 24 and a light chain having the amino acid sequence set forth in SEQ ID NO: 20, or
a heavy chain having the amino acid sequence set forth in SEQ ID NO: 26 and a light chain having the amino acid sequence set forth in SEQ ID NO: 22, or
wherein the antibody comprises (i) an amino acid sequence with at least 75% identity to a sequence set forth in SEQ ID NO: 28 or 29, or (ii) the amino acid sequence set forth in SEQ ID NO: 28 or 29.

15. The agent for use of any one of claims 11 to 14,
wherein the agent is administered at a dose of at or about or at least at or about 10 mg/kg or 20 mg/kg or between about 10-20 mg/kg, or
wherein the agent is administered at a dose of at least at or about or at or about 200 mg or 700 mg or between about 200-700 mg, or
wherein the agent is administered at a dose of at least at or about or at or about 75 mg or 125 mg or between at or about 75-125 mg.


SEQUENCE LISTING

<110>  Gilead Sciences, Inc.

<120>  THERAPEUTIC AND DIAGNOSTIC METHODS RELATED TO LYSYL OXIDASE-LIKE
       2 (LOXL2)

<130>  411-143 PCTEPT1

<150>  US 61/720,350
<151>  2012-10-30

<150>  US 61/724,858
<151>  2012-11-09

<160>  30

<170>  PatentIn version 3.5

<210>  1
<211>  774
<212>  PRT
<213>  Homo sapiens


<220>
<221>  MISC_FEATURE
<222>  (1)..(774)
<223>  LOXL2

<400>  1

Met Glu Arg Pro Leu Cys Ser His Leu Cys Ser Cys Leu Ala Met Leu
1               5                   10                  15


Ala Leu Leu Ser Pro Leu Ser Leu Ala Gln Tyr Asp Ser Trp Pro His
            20                  25                  30


Tyr Pro Glu Tyr Phe Gln Gln Pro Ala Pro Glu Tyr His Gln Pro Gln
        35                  40                  45


Ala Pro Ala Asn Val Ala Lys Ile Gln Leu Arg Leu Ala Gly Gln Lys
    50                  55                  60


Arg Lys His Ser Glu Gly Arg Val Glu Val Tyr Tyr Asp Gly Gln Trp
65                  70                  75                  80


Gly Thr Val Cys Asp Asp Asp Phe Ser Ile His Ala Ala His Val Val
                85                  90                  95


Cys Arg Glu Leu Gly Tyr Val Glu Ala Lys Ser Trp Thr Ala Ser Ser
            100                 105                 110


Ser Tyr Gly Lys Gly Glu Gly Pro Ile Trp Leu Asp Asn Leu His Cys
        115                 120                 125

Thr Gly Asn Glu Ala Thr Leu Ala Ala Cys Thr Ser Asn Gly Trp Gly
    130                 135                 140

Val Thr Asp Cys Lys His Thr Glu Asp Val Gly Val Val Cys Ser Asp
145                 150                 155                 160

Lys Arg Ile Pro Gly Phe Lys Phe Asp Asn Ser Leu Ile Asn Gln Ile
                165                 170                 175

Glu Asn Leu Asn Ile Gln Val Glu Asp Ile Arg Ile Arg Ala Ile Leu
            180                 185                 190

Ser Thr Tyr Arg Lys Arg Thr Pro Val Met Glu Gly Tyr Val Glu Val
        195                 200                 205

Lys Glu Gly Lys Thr Trp Lys Gln Ile Cys Asp Lys His Trp Thr Ala
    210                 215                 220

Lys Asn Ser Arg Val Val Cys Gly Met Phe Gly Phe Pro Gly Glu Arg
225                 230                 235                 240

Thr Tyr Asn Thr Lys Val Tyr Lys Met Phe Ala Ser Arg Arg Lys Gln
                245                 250                 255

Arg Tyr Trp Pro Phe Ser Met Asp Cys Thr Gly Thr Glu Ala His Ile
            260                 265                 270

Ser Ser Cys Lys Leu Gly Pro Gln Val Ser Leu Asp Pro Met Lys Asn
        275                 280                 285

Val Thr Cys Glu Asn Gly Leu Pro Ala Val Val Ser Cys Val Pro Gly
    290                 295                 300

Gln Val Phe Ser Pro Asp Gly Pro Ser Arg Phe Arg Lys Ala Tyr Lys
305                 310                 315                 320

Pro Glu Gln Pro Leu Val Arg Leu Arg Gly Gly Ala Tyr Ile Gly Glu
                325                 330                 335

Gly Arg Val Glu Val Leu Lys Asn Gly Glu Trp Gly Thr Val Cys Asp
            340                 345                 350

Asp Lys Trp Asp Leu Val Ser Ala Ser Val Val Cys Arg Glu Leu Gly
        355                 360                 365

Phe Gly Ser Ala Lys Glu Ala Val Thr Gly Ser Arg Leu Gly Gln Gly
    370                 375                 380

Ile Gly Pro Ile His Leu Asn Glu Ile Gln Cys Thr Gly Asn Glu Lys
385                 390                 395                 400

Ser Ile Ile Asp Cys Lys Phe Asn Ala Glu Ser Gln Gly Cys Asn His
                405                 410                 415

Glu Glu Asp Ala Gly Val Arg Cys Asn Thr Pro Ala Met Gly Leu Gln
            420                 425                 430

Lys Lys Leu Arg Leu Asn Gly Gly Arg Asn Pro Tyr Glu Gly Arg Val
        435                 440                 445

Glu Val Leu Val Glu Arg Asn Gly Ser Leu Val Trp Gly Met Val Cys
    450                 455                 460

Gly Gln Asn Trp Gly Ile Val Glu Ala Met Val Val Cys Arg Gln Leu
465                 470                 475                 480

Gly Leu Gly Phe Ala Ser Asn Ala Phe Gln Glu Thr Trp Tyr Trp His
                485                 490                 495

Gly Asp Val Asn Ser Asn Lys Val Val Met Ser Gly Val Lys Cys Ser
            500                 505                 510

Gly Thr Glu Leu Ser Leu Ala His Cys Arg His Asp Gly Glu Asp Val
        515                 520                 525

Ala Cys Pro Gln Gly Gly Val Gln Tyr Gly Ala Gly Val Ala Cys Ser
    530                 535                 540

Glu Thr Ala Pro Asp Leu Val Leu Asn Ala Glu Met Val Gln Gln Thr
545                 550                 555                 560

Thr Tyr Leu Glu Asp Arg Pro Met Phe Met Leu Gln Cys Ala Met Glu
                565                 570                 575

Glu Asn Cys Leu Ser Ala Ser Ala Ala Gln Thr Asp Pro Thr Thr Gly
            580                 585                 590

Tyr Arg Arg Leu Leu Arg Phe Ser Ser Gln Ile His Asn Asn Gly Gln
        595                 600                 605

Ser Asp Phe Arg Pro Lys Asn Gly Arg His Ala Trp Ile Trp His Asp
    610                 615                 620

Cys His Arg His Tyr His Ser Met Glu Val Phe Thr His Tyr Asp Leu

625                 630                 635                 640

Leu Asn Leu Asn Gly Thr Lys Val Ala Glu Gly His Lys Ala Ser Phe
                645                 650                 655

Cys Leu Glu Asp Thr Glu Cys Glu Gly Asp Ile Gln Lys Asn Tyr Glu
            660                 665                 670

Cys Ala Asn Phe Gly Asp Gln Gly Ile Thr Met Gly Cys Trp Asp Met
        675                 680                 685

Tyr Arg His Asp Ile Asp Cys Gln Trp Val Asp Ile Thr Asp Val Pro
    690                 695                 700

Pro Gly Asp Tyr Leu Phe Gln Val Val Ile Asn Pro Asn Phe Glu Val
705                 710                 715                 720

Ala Glu Ser Asp Tyr Ser Asn Asn Ile Met Lys Cys Arg Ser Arg Tyr
                725                 730                 735

Asp Gly His Arg Ile Trp Met Tyr Asn Cys His Ile Gly Gly Ser Phe
            740                 745                 750

Ser Glu Glu Thr Glu Lys Lys Phe Glu His Phe Ser Gly Leu Leu Asn
        755                 760                 765

Asn Gln Leu Ser Pro Gln
    770

<210>  2
<211>  227
<212>  PRT
<213>  Homo sapiens

<220>
<221>  DOMAIN
<222>  (1)..(227)
<223>  catalytic domain of LOXL2 protein

<400>  2

Pro Asp Leu Val Leu Asn Ala Glu Met Val Gln Gln Thr Thr Tyr Leu
1               5                   10                  15

Glu Asp Arg Pro Met Phe Met Leu Gln Cys Ala Met Glu Glu Asn Cys
            20                  25                  30

Leu Ser Ala Ser Ala Ala Gln Thr Asp Pro Thr Thr Gly Tyr Arg Arg
        35                  40                  45

Leu Leu Arg Phe Ser Ser Gln Ile His Asn Asn Gly Gln Ser Asp Phe
    50                  55                  60

Arg Pro Lys Asn Gly Arg His Ala Trp Ile Trp His Asp Cys His Arg
65                  70                  75                  80

His Tyr His Ser Met Glu Val Phe Thr His Tyr Asp Leu Leu Asn Leu
                85                  90                  95

Asn Gly Thr Lys Val Ala Glu Gly His Lys Ala Ser Phe Cys Leu Glu
            100                 105                 110

Asp Thr Glu Cys Glu Gly Asp Ile Gln Lys Asn Tyr Glu Cys Ala Asn
        115                 120                 125

Phe Gly Asp Gln Gly Ile Thr Met Gly Cys Trp Asp Met Tyr Arg His
    130                 135                 140

Asp Ile Asp Cys Gln Trp Val Asp Ile Thr Asp Val Pro Pro Gly Asp
145                 150                 155                 160

Tyr Leu Phe Gln Val Val Ile Asn Pro Asn Phe Glu Val Ala Glu Ser
                165                 170                 175

Asp Tyr Ser Asn Asn Ile Met Lys Cys Arg Ser Arg Tyr Asp Gly His
            180                 185                 190

Arg Ile Trp Met Tyr Asn Cys His Ile Gly Gly Ser Phe Ser Glu Glu
        195                 200                 205

Thr Glu Lys Lys Phe Glu His Phe Ser Gly Leu Leu Asn Asn Gln Leu
    210                 215                 220

Ser Pro Gln
225

<210>  3
<211>  227
<212>  PRT
<213>  Mus musculus

<220>
<221>  DOMAIN
<222>  (1)..(227)
<223>  catalytic domain of LOXL2 protein

<400>  3

Pro Asp Leu Val Leu Asn Ala Glu Ile Val Gln Gln Thr Ala Tyr Leu

1               5                   10                  15

Glu Asp Arg Pro Met Ser Leu Leu Gln Cys Ala Met Glu Glu Asn Cys
            20                  25                  30

Leu Ser Ala Ser Ala Val His Thr Asp Pro Thr Arg Gly His Arg Arg
        35                  40                  45

Leu Leu Arg Phe Ser Ser Gln Ile His Asn Asn Gly Gln Ser Asp Phe
    50                  55                  60

Arg Pro Lys Asn Gly Arg His Ala Trp Ile Trp His Asp Cys His Arg
65                  70                  75                  80

His Tyr His Ser Met Glu Val Phe Thr Tyr Tyr Asp Leu Leu Ser Leu
                85                  90                  95

Asn Gly Thr Lys Val Ala Glu Gly His Lys Ala Ser Phe Cys Leu Glu
            100                 105                 110

Asp Thr Glu Cys Glu Gly Asp Ile Gln Lys Ser Tyr Glu Cys Ala Asn
        115                 120                 125

Phe Gly Glu Gln Gly Ile Thr Met Gly Cys Trp Asp Met Tyr Arg His
    130                 135                 140

Asp Ile Asp Cys Gln Trp Ile Asp Ile Thr Asp Val Pro Pro Gly Asp
145                 150                 155                 160

Tyr Leu Phe Gln Val Val Ile Asn Pro Asn Tyr Glu Val Pro Glu Ser
                165                 170                 175

Asp Phe Ser Asn Asn Ile Met Lys Cys Arg Ser Arg Tyr Asp Gly Tyr
            180                 185                 190

Arg Ile Trp Met Tyr Asn Cys His Val Gly Gly Ala Phe Ser Glu Glu
        195                 200                 205

Thr Glu Gln Lys Phe Glu His Phe Ser Gly Leu Leu Asn Asn Gln Leu
    210                 215                 220

Ser Val Gln
225

<210>  4
<211>  227
<212>  PRT
<213>  Rattus norvegicus

<220>
<221>  DOMAIN
<222>  (1)..(227)
<223>  catalytic domain of LOXL2 protein

<400>  4

Pro Asp Leu Val Leu Asn Ala Glu Ile Val Gln Gln Thr Ala Tyr Leu
1               5                   10                  15

Glu Asp Arg Pro Met Ala Leu Leu Gln Cys Ala Met Glu Glu Asn Cys
            20                  25                  30

Leu Ser Ala Ser Ala Val His Thr Asp Pro Thr Arg Gly His Arg Arg
        35                  40                  45

Leu Leu Arg Phe Ser Ser Gln Ile His Asn Asn Gly Gln Ser Asp Phe
    50                  55                  60

Arg Pro Lys Asn Gly Arg His Ala Trp Ile Trp His Asp Cys His Arg
65                  70                  75                  80

His Tyr His Ser Met Glu Val Phe Thr Tyr Tyr Asp Leu Leu Ser Leu
                85                  90                  95

Asn Gly Thr Lys Val Ala Glu Gly His Lys Ala Ser Phe Cys Leu Glu
            100                 105                 110

Asp Thr Glu Cys Glu Gly Asp Ile Gln Lys Ser Tyr Glu Cys Ala Asn
        115                 120                 125

Phe Gly Glu Gln Gly Ile Thr Met Gly Cys Trp Asp Met Tyr Arg His
    130                 135                 140

Asp Ile Asp Cys Gln Trp Ile Asp Ile Thr Asp Val Pro Pro Gly Asp
145                 150                 155                 160

Tyr Leu Phe Gln Val Val Ile Asn Pro Asn Tyr Glu Val Pro Glu Ser
                165                 170                 175

Asp Phe Ser Asn Asn Ile Met Lys Cys Arg Ser Arg Tyr Asp Gly Tyr
            180                 185                 190

Arg Ile Trp Met Tyr Asn Cys His Val Gly Gly Ala Phe Ser Glu Glu
        195                 200                 205

Thr Glu Gln Lys Phe Glu His Phe Ser Gly Leu Leu Asn Asn Gln Leu
    210                 215                 220

Ser Val Gln
225

<210> 5
<211> 227
<212> PRT
<213> Macaca fascicularis

<220>
<221> DOMAIN
<222> (1)..(227)
<223> catalytic domain of LOXL2 protein

<400> 5

Pro Asp Leu Val Leu Asn Ala Glu Met Val Gln Gln Thr Thr Tyr Leu
1               5                   10                  15

Glu Asp Arg Pro Met Phe Met Leu Gln Cys Ala Met Glu Glu Asn Cys
            20                  25                  30

Leu Ser Ala Ser Ala Ala Gln Thr Asn Pro Thr Thr Gly Tyr Arg Arg
        35                  40                  45

Leu Leu Arg Phe Ser Ser Gln Ile His Asn Asn Gly Gln Ser Asp Phe
    50                  55                  60

Arg Pro Lys Asn Gly Arg His Ala Trp Ile Trp His Asp Cys His Arg
65                  70                  75                  80

His Tyr His Ser Met Glu Val Phe Thr His Tyr Asp Leu Leu Asn Leu
                85                  90                  95

Asn Gly Thr Lys Val Ala Glu Gly His Lys Ala Ser Phe Cys Leu Glu
            100                 105                 110

Asp Thr Glu Cys Glu Gly Asp Ile Gln Lys Asn Tyr Glu Cys Ala Asn
        115                 120                 125

Phe Gly Asp Gln Gly Ile Thr Met Gly Cys Trp Asp Met Tyr Arg His
    130                 135                 140

Asp Ile Asp Cys Gln Trp Ile Asp Ile Thr Asp Val Pro Pro Gly Asp
145                 150                 155                 160

Tyr Leu Phe Gln Val Val Ile Asn Pro Asn Phe Glu Val Ala Glu Ser
                165                 170                 175

Asp Tyr Ser Asn Asn Ile Met Lys Cys Arg Ser Arg Tyr Asp Gly His
            180                 185                 190

Arg Ile Trp Met Tyr Asn Cys His Ile Gly Gly Ser Phe Ser Glu Glu
        195                 200                 205

Thr Glu Lys Lys Phe Glu His Phe Ser Gly Leu Leu Asn Asn Gln Leu
    210                 215                 220

Ser Pro Gln
225

<210>  6
<211>  135
<212>  PRT
<213>  Artificial Sequence

<220>
<223>  synthetic construct

<220>
<221>  CHAIN
<222>  (1)..(135)
<223>  heavy chain variable region

<400>  6

Met Glu Trp Ser Arg Val Phe Ile Phe Leu Leu Ser Val Thr Ala Gly
1               5                   10                  15

Val His Ser Gln Val Gln Leu Gln Gln Ser Gly Ala Glu Leu Val Arg
            20                  25                  30

Pro Gly Thr Ser Val Lys Val Ser Cys Lys Ala Ser Gly Tyr Ala Phe
        35                  40                  45

Thr Tyr Tyr Leu Ile Glu Trp Val Lys Gln Arg Pro Gly Gln Gly Leu
    50                  55                  60

Glu Trp Ile Gly Val Ile Asn Pro Gly Ser Gly Gly Thr Asn Tyr Asn
65                  70                  75                  80

Glu Lys Phe Lys Gly Lys Ala Thr Leu Thr Ala Asp Lys Ser Ser Ser
                85                  90                  95

Thr Ala Tyr Met Gln Leu Ser Ser Leu Thr Ser Asp Asp Ser Ala Val
            100                 105                 110

Tyr Phe Cys Ala Arg Asn Trp Met Asn Phe Asp Tyr Trp Gly Gln Gly
        115                 120                 125

Thr Thr Leu Thr Val Ser Ser
    130                 135

<210>  7
<211>  132
<212>  PRT
<213>  Artificial Sequence

<220>
<223>  synthetic construct

<220>
<221>  CHAIN
<222>  (1)..(132)
<223>  light chain variable region

<400>  7

Met Arg Cys Leu Ala Glu Phe Leu Gly Leu Leu Val Leu Trp Ile Pro
1               5                   10                  15

Gly Ala Ile Gly Asp Ile Val Met Thr Gln Ala Ala Pro Ser Val Ser
            20                  25                  30

Val Thr Pro Gly Glu Ser Val Ser Ile Ser Cys Arg Ser Ser Lys Ser
        35                  40                  45

Leu Leu His Ser Asn Gly Asn Thr Tyr Leu Tyr Trp Phe Leu Gln Arg
    50                  55                  60

Pro Gly Gln Ser Pro Gln Phe Leu Ile Tyr Arg Met Ser Asn Leu Ala
65                  70                  75                  80

Ser Gly Val Pro Asp Arg Phe Ser Gly Ser Gly Ser Gly Thr Ala Phe
                85                  90                  95

Thr Leu Arg Ile Ser Arg Val Glu Ala Glu Asp Val Gly Val Tyr Tyr
            100                 105                 110

Cys Met Gln His Leu Glu Tyr Pro Tyr Thr Phe Gly Gly Gly Thr Lys
        115                 120                 125

Leu Glu Ile Lys
    130

<210>  8
<211>  116
<212>  PRT
<213>  Artificial Sequence

<220>

<223>  synthetic construct

<220>
<221>  CHAIN
<222>  (1)..(116)
<223>  heavy chain variable region

<400>  8

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ala Phe Thr Tyr Tyr
            20                  25                  30

Leu Ile Glu Trp Val Arg Gln Ala Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Arg Ala Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Asn Trp Met Asn Phe Asp Tyr Trp Gly Gln Gly Thr Thr Val
            100                 105                 110

Thr Val Ser Ser
        115

<210>  9
<211>  112
<212>  PRT
<213>  Artificial Sequence

<220>
<223>  synthetic construct

<220>
<221>  DOMAIN
<222>  (1)..(112)
<223>  light chain variable region

<400>  9

Asp Ile Val Met Thr Gln Thr Pro Leu Ser Leu Ser Val Thr Pro Gly
1               5                   10                  15

Gln Pro Ala Ser Ile Ser Cys Arg Ser Ser Lys Ser Leu Leu His Ser

20                  25                  30

Asn Gly Asn Thr Tyr Leu Tyr Trp Phe Leu Gln Lys Pro Gly Gln Ser
        35                  40                  45

Pro Gln Phe Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Val Glu Ile Lys
            100                 105                 110

<210>  10
<211>  116
<212>  PRT
<213>  Artificial Sequence

<220>
<223>  synthetic construct


<220>
<221>  DOMAIN
<222>  (1)..(116)
<223>  heavy chain variable region

<400>  10

Gln Val Gln Leu Val Gln Ser Gly Ala Glu Leu Lys Lys Pro Gly Ala
1               5                   10                  15

Ser Val Lys Val Ser Cys Lys Ala Ser Gly Tyr Ala Phe Thr Tyr Tyr
            20                  25                  30

Leu Ile Glu Trp Val Lys Gln Ala Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Arg Ala Thr Leu Thr Ala Asp Lys Ser Thr Ser Thr Ala Tyr
65                  70                  75                  80

Met Glu Leu Ser Ser Leu Arg Ser Glu Asp Ser Ala Val Tyr Phe Cys
                85                  90                  95

Ala Arg Asn Trp Met Asn Phe Asp Tyr Trp Gly Gln Gly Thr Thr Val
            100                 105                 110

Thr Val Ser Ser
        115

<210>  11
<211>  116
<212>  PRT
<213>  Artificial Sequence

<220>
<223>  synthetic construct

<220>
<221>  DOMAIN
<222>  (1)..(116)
<223>  heavy chain variable region

<400>  11

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ala Phe Thr Tyr Tyr
            20                  25                  30

Leu Ile Glu Trp Val Arg Gln Ala Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Arg Ala Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Asn Trp Met Asn Phe Asp Tyr Trp Gly Gln Gly Thr Thr Val
            100                 105                 110

Thr Val Ser Ser
        115

<210>  12
<211>  116
<212>  PRT
<213>  Artificial Sequence

<220>
<223>  synthetic construct

<220>
<221>  DOMAIN
<222>  (1)..(116)
<223>  heavy chain variable region

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ala Phe Thr Tyr Tyr
            20                  25                  30

Leu Ile Glu Trp Val Arg Gln Ala Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Trp Met Asn Phe Asp Tyr Trp Gly Gln Gly Thr Thr Val
            100                 105                 110

Thr Val Ser Ser
        115


<210>  13
<211>  112
<212>  PRT
<213>  Artificial Sequence

<220>
<223>  synthetic construct


<220>
<221>  DOMAIN
<222>  (1)..(112)
<223>  light chain variable region

<400>  13

Asp Ile Val Met Thr Gln Thr Pro Leu Ser Leu Ser Val Thr Pro Gly
1               5                   10                  15

Gln Pro Ala Ser Ile Ser Cys Arg Ser Ser Lys Ser Leu Leu His Ser
            20                  25                  30

Asn Gly Asn Thr Tyr Leu Tyr Trp Phe Leu Gln Lys Pro Gly Gln Ser
        35                  40                  45

Pro Gln Phe Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Val Glu Ile Lys
            100                 105                 110

<210> 14
<211> 112
<212> PRT
<213> Artificial Sequence

<220>
<223> synthetic construct

<220>
<221> DOMAIN
<222> (1)..(112)
<223> light chain variable region

<400> 14

Asp Ile Val Met Thr Gln Thr Pro Leu Ser Leu Ser Val Thr Pro Gly
1               5                   10                  15

Gln Pro Ala Ser Ile Ser Cys Arg Ser Ser Lys Ser Leu Leu His Ser
            20                  25                  30

Asn Gly Asn Thr Tyr Leu Tyr Trp Tyr Leu Gln Lys Pro Gly Gln Ser
        35                  40                  45

Pro Gln Phe Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Val Glu Ile Lys

            100                 105                 110

<210> 15
<211> 15
<212> PRT
<213> Homo sapiens

<400> 15

Val Trp Gly Met Val Cys Gly Gln Asn Trp Gly Ile Val Glu Ala
1               5                   10                  15

<210> 16

<400> 16
000

<210> 17
<211> 15
<212> PRT
<213> Homo sapiens

<400> 17

Val Glu Ala Met Val Val Cys Arg Gln Leu Gly Leu Gly Phe Ala
1               5                   10                  15

<210> 18
<211> 15
<212> PRT
<213> Homo sapiens

<400> 18

Gly Phe Ala Ser Asn Ala Phe Gln Glu Thr Trp Tyr Trp His Gly
1               5                   10                  15

<210> 19
<211> 210
<212> PRT
<213> Homo sapiens

<400> 19

Val Arg Leu Arg Gly Gly Ala Tyr Ile Gly Glu Gly Arg Val Glu Val
1               5                   10                  15

Leu Lys Asn Gly Glu Trp Gly Thr Val Cys Asp Asp Lys Trp Asp Leu
            20                  25                  30

Val Ser Ala Ser Val Val Cys Arg Glu Leu Gly Phe Gly Ser Ala Lys
        35                  40                  45

Glu Ala Val Thr Gly Ser Arg Leu Gly Gln Gly Ile Gly Pro Ile His
    50                  55                  60

Leu Asn Glu Ile Gln Cys Thr Gly Asn Glu Lys Ser Ile Ile Asp Cys
65                  70                  75                  80

Lys Phe Asn Ala Glu Ser Gln Gly Cys Asn His Glu Glu Asp Ala Gly
                85                  90                  95

Val Arg Cys Asn Leu Arg Leu Asn Gly Gly Arg Asn Pro Tyr Glu Gly
            100                 105                 110

Arg Val Glu Val Leu Val Glu Arg Asn Gly Ser Leu Val Trp Gly Met
        115                 120                 125

Val Cys Gly Gln Asn Trp Gly Ile Val Glu Ala Met Val Val Cys Arg
    130                 135                 140

Gln Leu Gly Leu Gly Phe Ala Ser Asn Ala Phe Gln Glu Thr Trp Tyr
145                 150                 155                 160

Trp His Gly Asp Val Asn Ser Asn Lys Val Val Met Ser Gly Val Lys
                165                 170                 175

Cys Ser Gly Thr Glu Leu Ser Leu Ala His Cys Arg His Asp Gly Glu
            180                 185                 190

Asp Val Ala Cys Pro Gln Gly Gly Val Gln Tyr Gly Ala Gly Val Ala
        195                 200                 205

Cys Ser
    210

<210>  20
<211>  239
<212>  PRT
<213>  Artificial Sequence

<220>
<223>  Light chain humanized anti-LOXL2 antibody

<400>  20

Met Arg Val Pro Ala Gln Leu Leu Gly Leu Leu Leu Leu Trp Leu Pro
1               5                   10                  15

Gly Ala Arg Cys Asp Ile Val Met Thr Gln Thr Pro Leu Ser Leu Ser
            20                  25                  30

Val Thr Pro Gly Gln Pro Ala Ser Ile Ser Cys Arg Ser Ser Lys Ser
        35                  40                  45

Leu Leu His Ser Asn Gly Asn Thr Tyr Leu Tyr Trp Phe Leu Gln Lys
    50                  55                  60

Pro Gly Gln Ser Pro Gln Phe Leu Ile Tyr Arg Met Ser Asn Leu Ala
65                  70                  75                  80

Ser Gly Val Pro Asp Arg Phe Ser Gly Ser Gly Ser Gly Thr Asp Phe
                85                  90                  95

Thr Leu Lys Ile Ser Arg Val Glu Ala Glu Asp Val Gly Val Tyr Tyr
            100                 105                 110

Cys Met Gln His Leu Glu Tyr Pro Tyr Thr Phe Gly Gly Gly Thr Lys
        115                 120                 125

Val Glu Ile Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys
225                 230                 235

<210>  21
<211>  720
<212>  DNA
<213>  Artificial Sequence

<220>
<223>  nucleic acid encoding light chain of a humanized anti-LOXL2
       antibody:

<400>  21
atgagggtcc ccgctcagct cctggggctc ctgctgctct ggctcccagg cgcgcgatgt        60

gatattgtga tgacccagac tccactctct ctgtccgtca cccctggaca gccggcctcc       120

atctcctgca ggtctagtaa gagtctcctg catagtaatg gcaacactta cttgtattgg       180

ttcctgcaga agccagggca gtctccacag ttcctgatct atcggatgtc caaccttgcc 240

tcaggagtgc cagataggtt cagtggcagc gggtcaggga cagacttcac actgaaaatc 300

agccgggtgg aggctgagga tgttggggtt tattactgca tgcaacatct agaatatcct 360

tacaccttcg gcggagggac caaggtggag atcaaacgaa ctgtggctgc accatctgtc 420

ttcatcttcc cgccatctga tgagcagttg aaatctggaa ctgcctctgt tgtgtgcctg 480

ctgaataact tctatcccag agaggccaaa gtacagtgga aggtggataa cgccctccaa 540

tcgggtaact cccaggagag tgtcacagag caggacagca aggacagcac ctacagcctc 600

agcagcaccc tgacgctgag caaagcagac tacgagaaac acaaagtcta cgcctgcgaa 660

gtcacccatc agggcctgag ctcgcccgtc acaaagagct tcaacagggg agagtgttag 720

<210> 22
<211> 219
<212> PRT
<213> Artificial Sequence

<220>
<223> synthetic construct

<220>
<221> CHAIN
<222> (2)..(219)
<223> light chain polypeptide

<400> 22

Asp Ile Val Met Thr Gln Thr Pro Leu Ser Leu Ser Val Thr Pro Gly
1               5                   10                  15

Gln Pro Ala Ser Ile Ser Cys Arg Ser Ser Lys Ser Leu Leu His Ser
            20                  25                  30

Asn Gly Asn Thr Tyr Leu Tyr Trp Phe Leu Gln Lys Pro Gly Gln Ser
        35                  40                  45

Pro Gln Phe Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  23
<211>  660
<212>  DNA
<213>  Artificial Sequence

<220>
<223>  synthetic construct encoding SEQ ID NO:22

<400>  23
gatattgtga tgacccagac tccactctct ctgtccgtca cccctggaca gccggcctcc      60

atctcctgca ggtctagtaa gagtctcctg catagtaatg gcaacactta cttgtattgg     120

ttcctgcaga agccagggca gtctccacag ttcctgatct atcggatgtc caaccttgcc     180

tcaggagtgc cagataggtt cagtggcagc gggtcaggga cagacttcac actgaaaatc     240

agccgggtgg aggctgagga tgttggggtt tattactgca tgcaacatct agaatatcct     300

tacaccttcg gcggagggac caaggtggag atcaaacgaa ctgtggctgc accatctgtc     360

ttcatcttcc cgccatctga tgagcagttg aaatctggaa ctgcctctgt tgtgtgcctg     420

ctgaataact tctatcccag agaggccaaa gtacagtgga aggtggataa cgccctccaa     480

tcgggtaact cccaggagag tgtcacagag caggacagca aggacagcac ctacagcctc     540

agcagcaccc tgacgctgag caaagcagac tacgagaaac acaaagtcta cgcctgcgaa     600

gtcacccatc agggcctgag ctcgcccgtc acaaagagct tcaacagggg agagtgttag     660

<210>  24
<211>  462

<212>  PRT
<213>  Artificial Sequence

<220>
<223>  Heavy chain humanized anti-LOXL2 antibody

<400>  24

Met Gly Trp Ser Leu Ile Leu Leu Phe Leu Val Ala Val Ala Thr Arg
1               5                   10                  15

Val His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Ala Phe
        35                  40                  45

Thr Tyr Tyr Leu Ile Glu Trp Val Arg Gln Ala Pro Gly Gln Gly Leu
    50                  55                  60

Glu Trp Ile Gly Val Ile Asn Pro Gly Ser Gly Gly Thr Asn Tyr Asn
65                  70                  75                  80

Glu Lys Phe Lys Gly Arg Ala Thr Ile Thr Ala Asp Lys Ser Thr Ser
                85                  90                  95

Thr Ala Tyr Met Glu Leu Ser Ser Leu Arg Ser Glu Asp Thr Ala Val
            100                 105                 110

Tyr Phe Cys Ala Arg Asn Trp Met Asn Phe Asp Tyr Trp Gly Gln Gly
        115                 120                 125

Thr Thr Val Thr Val Ser Ser Ala Ser Thr Lys Gly Pro Ser Val Phe
    130                 135                 140

Pro Leu Ala Pro Cys Ser Arg Ser Thr Ser Glu Ser Thr Ala Ala Leu
145                 150                 155                 160

Gly Cys Leu Val Lys Asp Tyr Phe Pro Glu Pro Val Thr Val Ser Trp
                165                 170                 175

Asn Ser Gly Ala Leu Thr Ser Gly Val His Thr Phe Pro Ala Val Leu
            180                 185                 190

Gln Ser Ser Gly Leu Tyr Ser Leu Ser Ser Val Val Thr Val Pro Ser
        195                 200                 205

Ser Ser Leu Gly Thr Lys Thr Tyr Thr Cys Asn Val Asp His Lys Pro
    210                 215                 220

Ser Asn Thr Lys Val Asp Lys Arg Val Glu Ser Lys Tyr Gly Pro Pro
225                 230                 235                 240

Cys Pro Pro Cys Pro Ala Pro Glu Phe Leu Gly Gly Pro Ser Val Phe
                245                 250                 255

Leu Phe Pro Pro Lys Pro Lys Asp Thr Leu Met Ile Ser Arg Thr Pro
            260                 265                 270

Glu Val Thr Cys Val Val Val Asp Val Ser Gln Glu Asp Pro Glu Val
        275                 280                 285

Gln Phe Asn Trp Tyr Val Asp Gly Val Glu Val His Asn Ala Lys Thr
    290                 295                 300

Lys Pro Arg Glu Glu Gln Phe Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Gly Leu Pro Ser Ser Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Gln Glu Glu Met Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Lys Thr Thr Pro Pro Val Leu Asp Ser Asp
                405                 410                 415

Gly Ser Phe Phe Leu Tyr Ser Arg Leu Thr Val Asp Lys Ser Arg Trp
            420                 425                 430

Gln Glu Gly Asn Val Phe Ser Cys Ser Val Met His Glu Ala Leu His
        435                 440                 445

Asn His Tyr Thr Gln Lys Ser Leu Ser Leu Ser Leu Gly Lys
    450                 455                 460

<210>  25
<211>  1389

<212>  DNA
<213>  Artificial Sequence

<220>
<223>  nucleic acid encoding a heavy chain humanized anti-LOXL2 antibody

<400>  25
atgggttgga gcctcatctt gctcttcctt gtcgctgttg ctacgcgtgt ccactcccag      60

gtgcagctgg tgcagtctgg ggctgaggtg aagaagcctg gggcctcagt gaaggtctcc     120

tgcaaggctt ctggatacgc cttcacttat tacttgatag agtgggtgag acaggcccct     180

ggacaagggc ttgagtggat cggagtgatt aatcctggaa gtggtggtac taactacaat     240

gagaagttca agggcagagc cacgatcacc gcggacaaat ccacgagcac agcctacatg     300

gagctgagca gcctgagatc tgaggacacc gccgtgtatt tctgtgcgag aaactggatg     360

aactttgact actggggcca agggaccacg gtcaccgtct cctcagcttc caccaagggc     420

ccatccgtct tccccctggc gccctgctcc aggagcacct ccgagagcac agccgccctg     480

ggctgcctgg tcaaggacta cttccccgaa ccggtgacgg tgtcgtggaa ctcaggcgcc     540

ctgaccagcg gcgtgcacac cttcccggct gtcctacagt cctcaggact ctactccctc     600

agcagcgtgg tgaccgtgcc ctccagcagc ttgggcacga agacctacac ctgcaatgta     660

gatcacaagc ccagcaacac caaggtggac aagagagttg agtccaaata tggtccccca     720

tgcccaccat gcccagcacc tgagttcctg gggggaccat cagtcttcct gttcccccca     780

aaacccaagg acactctcat gatctcccgg acccctgagg tcacgtgcgt ggtggtggac     840

gtgagccagg aagaccccga ggtccagttc aactggtacg tggatggcgt ggaggtgcat     900

aatgccaaga caaagccgcg ggaggagcag ttcaacagca cgtaccgtgt ggtcagcgtc     960

ctcaccgtcc tgcaccagga ctggctgaac ggcaaggagt acaagtgcaa ggtctccaac    1020

aaaggcctcc cgtcctccat cgagaaaacc atctccaaag ccaaagggca gccccgagag    1080

ccacaggtgt acaccctgcc cccatcccag gaggagatga ccaagaacca ggtcagcctg    1140

acctgcctgg tcaaaggctt ctaccccagc gacatcgccg tggagtggga gagcaatggg    1200

cagccggaga acaactacaa gaccacgcct cccgtgctgg actccgacgg ctccttcttc    1260

ctctacagca ggctaaccgt ggacaagagc aggtggcagg aggggaatgt cttctcatgc    1320

tccgtgatgc atgaggctct gcacaaccac tacacacaga agagcctctc cctgtctctg    1380

ggtaaatga                                                            1389

<210>  26
<211>  443
<212>  PRT
<213>  Artificial Sequence

<220>
<223>  synthetic construct

<220>
<221>  DOMAIN
<222>  (1)..(443)
<223>  heavy chain polypeptide

<400>  26

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ala Phe Thr Tyr Tyr
            20                  25                  30

Leu Ile Glu Trp Val Arg Gln Ala Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Arg Ala Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Asn Trp Met Asn Phe Asp Tyr Trp Gly Gln Gly Thr Thr Val
            100                 105                 110

Thr Val Ser Ser Ala Ser Thr Lys Gly Pro Ser Val Phe Pro Leu Ala
        115                 120                 125

Pro Cys Ser Arg Ser Thr Ser Glu Ser Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr
        195                 200                 205

Lys Val Asp Lys Arg Val Glu Ser Lys Tyr Gly Pro Pro Cys Pro Pro
    210                 215                 220

Cys Pro Ala Pro Glu Phe Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser Gln Glu Asp Pro Glu Val Gln Phe Asn
            260                 265                 270

Trp Tyr Val Asp Gly Val Glu Val His Asn Ala Lys Thr Lys Pro Arg
        275                 280                 285

Glu Glu Gln Phe Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Gly Leu Pro Ser Ser Ile Glu Lys Thr Ile Ser Lys Ala Lys
                325                 330                 335

Gly Gln Pro Arg Glu Pro Gln Val Tyr Thr Leu Pro Pro Ser Gln Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Arg Leu Thr Val Asp Lys Ser Arg Trp Gln Glu Gly
                405                 410                 415

Asn Val Phe Ser Cys Ser Val Met His Glu Ala Leu His Asn His Tyr
            420                 425                 430

Thr Gln Lys Ser Leu Ser Leu Ser Leu Gly Lys
        435                 440

<210>  27
<211>  1332
<212>  DNA
<213>  Artificial Sequence

<220>
<223>  synthetic construct encoding SEQ ID NO:26

<400>  27
caggtgcagc tggtgcagtc tggggctgag gtgaagaagc ctggggcctc agtgaaggtc      60

tcctgcaagg cttctggata cgccttcact tattacttga tagagtgggt gagacaggcc     120

cctggacaag ggcttgagtg gatcggagtg attaatcctg gaagtggtgg tactaactac     180

aatgagaagt tcaagggcag agccacgatc accgcggaca aatccacgag cacagcctac     240

atggagctga gcagcctgag atctgaggac accgccgtgt atttctgtgc gagaaactgg     300

atgaactttg actactgggg gcaagggacc acggtcaccg tctcctcagc ttccaccaag     360

ggcccatccg tcttccccct ggcgccctgc tccaggagca cctccgagag cacagccgcc     420

ctgggctgcc tggtcaagga ctacttcccc gaaccggtga cggtgtcgtg gaactcaggc     480

gccctgacca gcggcgtgca caccttcccg gctgtcctac agtcctcagg actctactcc     540

ctcagcagcg tggtgaccgt gccctccagc agcttgggca cgaagaccta cacctgcaat     600

gtagatcaca agcccagcaa caccaaggtg gacaagagag ttgagtccaa atatggtccc     660

ccatgcccac catgcccagc acctgagttc ctggggggac catcagtctt cctgttcccc     720

ccaaaaccca aggacactct catgatctcc cggacccctg aggtcacgtg cgtggtggtg     780

gacgtgagcc aggaagaccc cgaggtccag ttcaactggt acgtggatgg cgtggaggtg     840

cataatgcca agacaaagcc gcgggaggag cagttcaaca gcacgtaccg tgtggtcagc     900

gtcctcaccg tcctgcacca ggactggctg aacggcaagg agtacaagtg caaggtctcc     960

aacaaaggcc tcccgtcctc catcgagaaa accatctcca aagccaaagg gcagccccga    1020

gagccacagg tgtacaccct gcccccatcc caggaggaga tgaccaagaa ccaggtcagc    1080

ctgacctgcc tggtcaaagg cttctacccc agcgacatcg ccgtggagtg ggagagcaat    1140

gggcagccgg agaacaacta caagaccacg cctcccgtgc tggactccga cggctccttc    1200

ttcctctaca gcaggctaac cgtggacaag agcaggtggc aggaggggaa tgtcttctca    1260

tgctccgtga tgcatgaggc tctgcacaac cactacacac agaagagcct ctccctgtct    1320

ctgggtaaat ga                                                        1332

<210>  28
<211>  662
<212>  PRT
<213>  Artificial Sequence

<220>
<223>  light/heavy chain polypetide construct

<400>  28

Asp Ile Val Met Thr Gln Thr Pro Leu Ser Leu Ser Val Thr Pro Gly
1               5                   10                  15

Gln Pro Ala Ser Ile Ser Cys Arg Ser Ser Lys Ser Leu Leu His Ser
            20                  25                  30

Asn Gly Asn Thr Tyr Leu Tyr Trp Phe Leu Gln Lys Pro Gly Gln Ser
        35                  40                  45

Pro Gln Phe Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys Gln Val Gln Leu Val
    210                 215                 220

Gln Ser Gly Ala Glu Val Lys Lys Pro Gly Ala Ser Val Lys Val Ser
225                 230                 235                 240

Cys Lys Ala Ser Gly Tyr Ala Phe Thr Tyr Tyr Leu Ile Glu Trp Val
                245                 250                 255

Arg Gln Ala Pro Gly Gln Gly Leu Glu Trp Ile Gly Val Ile Asn Pro

            260                 265                 270

Gly Ser Gly Gly Thr Asn Tyr Asn Glu Lys Phe Lys Gly Arg Ala Thr
        275                 280                 285

Ile Thr Ala Asp Lys Ser Thr Ser Thr Ala Tyr Met Glu Leu Ser Ser
    290                 295                 300

Leu Arg Ser Glu Asp Thr Ala Val Tyr Phe Cys Ala Arg Asn Trp Met
305                 310                 315                 320

Asn Phe Asp Tyr Trp Gly Gln Gly Thr Thr Val Thr Val Ser Ser Ala
                325                 330                 335

Ser Thr Lys Gly Pro Ser Val Phe Pro Leu Ala Pro Cys Ser Arg Ser
            340                 345                 350

Thr Ser Glu Ser Thr Ala Ala Leu Gly Cys Leu Val Lys Asp Tyr Phe
        355                 360                 365

Pro Glu Pro Val Thr Val Ser Trp Asn Ser Gly Ala Leu Thr Ser Gly
    370                 375                 380

Val His Thr Phe Pro Ala Val Leu Gln Ser Ser Gly Leu Tyr Ser Leu
385                 390                 395                 400

Ser Ser Val Val Thr Val Pro Ser Ser Ser Leu Gly Thr Lys Thr Tyr
                405                 410                 415

Thr Cys Asn Val Asp His Lys Pro Ser Asn Thr Lys Val Asp Lys Arg
            420                 425                 430

Val Glu Ser Lys Tyr Gly Pro Pro Cys Pro Pro Cys Pro Ala Pro Glu
        435                 440                 445

Phe Leu Gly Gly Pro Ser Val Phe Leu Phe Pro Pro Lys Pro Lys Asp
    450                 455                 460

Thr Leu Met Ile Ser Arg Thr Pro Glu Val Thr Cys Val Val Val Asp
465                 470                 475                 480

Val Ser Gln Glu Asp Pro Glu Val Gln Phe Asn Trp Tyr Val Asp Gly
                485                 490                 495

Val Glu Val His Asn Ala Lys Thr Lys Pro Arg Glu Glu Gln Phe Asn
            500                 505                 510

Ser Thr Tyr Arg Val Val Ser Val Leu Thr Val Leu His Gln Asp Trp
        515                 520                 525

Leu Asn Gly Lys Glu Tyr Lys Cys Lys Val Ser Asn Lys Gly Leu Pro
    530                 535                 540

Ser Ser Ile Glu Lys Thr Ile Ser Lys Ala Lys Gly Gln Pro Arg Glu
545                 550                 555                 560

Pro Gln Val Tyr Thr Leu Pro Pro Ser Gln Glu Glu Met Thr Lys Asn
                565                 570                 575

Gln Val Ser Leu Thr Cys Leu Val Lys Gly Phe Tyr Pro Ser Asp Ile
            580                 585                 590

Ala Val Glu Trp Glu Ser Asn Gly Gln Pro Glu Asn Asn Tyr Lys Thr
        595                 600                 605

Thr Pro Pro Val Leu Asp Ser Asp Gly Ser Phe Phe Leu Tyr Ser Arg
    610                 615                 620

Leu Thr Val Asp Lys Ser Arg Trp Gln Glu Gly Asn Val Phe Ser Cys
625                 630                 635                 640

Ser Val Met His Glu Ala Leu His Asn His Tyr Thr Gln Lys Ser Leu
                645                 650                 655

Ser Leu Ser Leu Gly Lys
            660

<210>  29
<211>  1324
<212>  PRT
<213>  Artificial Sequence

<220>
<223>  light/heavy chain polypeptide construct

<400>  29

<210> 30
<211> 327
<212> PRT
<213> Homo sapiens

<220>
<221> CHAIN
<222> (1)..(327)
<223> IgG4 heavy chain constant region polypeptide

<400> 30

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325